# EUROPEAN PATENT SPECIFICATION

(11) **EP 2 761 289 B1**
(45) Date of publication and mention of the grant of the patent: **12.02.2020**
(21) Application number: 12836076.5
(22) Date of filing: 28.09.2012
(51) Int. Cl.: G01N 33/68

(54) **CARDIOVASCULAR RISK EVENT PREDICTION AND USES THEREOF**
VORHERSAGE EINES KARDIOVASKULÄREN RISIKOEREIGNISSES UND VERWENDUNGEN DAVON
PRÉDICTION D'ÉVÈNEMENT DE RISQUE CARDIO-VASCULAIRE ET UTILISATIONS DE CELLE-CI

(30) Priority: 30.09.2011 US 201161541828 P
(43) Date of publication of application: 06.08.2014
(73) Proprietor: Somalogic, Inc., Boulder, CO 80301 (US)
(72) Inventor: GILL, Rosalynn Dianne, Boulder Colorado 80302 (US); WILLIAMS, Stephen Alaric, Boulder Colorado 80304 (US); STEWART, Alex A.E., San Francisco California 94132 (US); MEHLER, Robert, Boulder Colorado 80303 (US); FOREMAN, Trudi, Boulder Colorado 80302 (US); SINGER, Britta, Boulder Colorado 80305 (US)
(74) Representative: Mewburn Ellis LLP
(86) International application number: PCT/US2012/058060
(87) International publication number: WO 2013/049674

(56) References cited:
- WO-A1-2011/059721
- US-A1- 2011 003 707
- US-A1- 2011 144 914

## Description

### FIELD OF THE INVENTION

The present application relates generally to the detection of biomarkers and a method of evaluating the risk of a future cardiovascular event in an individual and, more specifically, to one or more biomarkers, methods, devices, reagents, systems, and kits used to assess an individual for the prediction of risk of developing a Cardiovascular (CV) Event over a 5 year period. Such Events include but are not limited to myocardial infarction, stroke, congestive heart failure or death.

### BACKGROUND

The following description provides a summary of information relevant to the present application and is not an admission that any of the information provided or publications referenced herein is prior art to the present application.

Cardiovascular disease is the leading cause of death in the USA. There are a number of existing and important predictors of risk of primary events (D'Agostino, R et al., "General Cardiovascular Risk Profile for Use in Primary Care: The Framingham Heart Study" Circulation 117:743-53 (2008); and Ridker, P. et al., "Development and Validation of Improved Algorithms fo rthe Assessment of Global Cardiovascular Risk in Women" JAMA 297(6):611-619 (2007)) and secondary events (Shlipak, M. et al. "Biomarkers to Predict Recurrent Cardiovascular Disease: The Heart & Soul Study" Am. J. Med. 121:50-57 (2008)) which are widely used in clinical practice and therapeutic trials. Unfortunately, the receiver-operating characteristic curves, hazard ratios, and concordance show that the performance of existing risk factors and biomarkers is modest (AUCs of -0.75 mean that these factors are only halfway between a coin-flip and perfection). In addition to a need for improved diagnostic performance, there is a need for a risk product which is both near-term and personally responsive within individuals to beneficial (and destructive) interventions and lifestyle changes. The commonly utilized Framingham equation has three main problems. Firstly, it is too long term: it gives 10-year risk calculations but humans discount future risks and are reluctant to make behavior and lifestyle modifications based on them. Secondly, it is not very responsive to interventions: it's most heavily weighted factor is chronological age, which cannot decline. Thirdly, within the high risk population envisioned here, the Framingham factors fail to discriminate well between high and low risk: the hazard ratio between high and low quartiles is only 2.

Risk factors for cardiovascular disease are widely used to drive the intensity and the nature of medical treatment, and their use has undoubtedly contributed to the reduction in cardiovascular morbidity and mortality that has been observed over the past two decades. These factors have routinely been combined into algorithms but unfortunately they do not capture all of the risk (the most common initial presentation for heart disease is still death). In fact they probably only capture half the risk. An area under the ROC curve of -0.76 is typical for such risk factors, and again, is only about halfway between a coin-flip at 0.5 and perfection at 1.0.

The addition of novel biomarkers to clinical risk scores has been disappointing. For example, in the Framingham study (Wang et al., "Multiple Biomarkers for the Prediction of First Major Cardiovascular Events and Death" N. Eng. J. Med. 355:2631-2637 (2006)) in 3209 people, the addition of 10 biomarkers (CRP, BNP, NT-proBNP, aldosterone, renin, fribrinogen, D-dimer, plasminogen-activator inhibitor type 1, homocysteine and the urinary albumin to creatinine ratio), did not significantly improve the AUC when added to existing risk factors: the AUC for events 0-5 years was 0.76 with age, sex and conventional risk factors and 0.77 with the best combination of biomarkers added to the mix.

Early identification of patients with higher risk of a cardiovascular event within a 1-10 year window is important because more aggressive treatment of individuals with elevated risk may improve outcome. Thus, optimal management requires agressive intervention to reduce the risk of a cardiovascular event in those patients who are considered to have a higher risk, while patients with a lower risk of a cardiovascular event can be spared expensive and potentially invasive treatments, which are likely to have no beneficial effect to the patient.

Biomarker selection for the prediction of risk of having specific disease state or condition within a defined time period involves first the identification of markers that have a measurable and statistically significant difference in populations in which the event has or has not occurred during the time period for a specific medical application. Biomarkers can include secreted or shed molecules that parallel disease or condition development or progression and readily diffuse into the blood stream from cardiovascular tissue or from surrounding tissues and circulating cells in response to a cardiovascular event. The biomarker or set of biomarkers identified are generally clinically validated or shown to be a reliable indicator for the original intended use for which it was selected. Biomarkers can include small molecules, peptides, proteins, and nucleic acids. Some of the key issues that affect the identification of biomarkers include over-fitting of the available data and bias in the data.

A variety of methods have been utilized in an attempt to identify biomarkers and diagnose or predict the risk of having disease or a condition. For protein-based markers, these include two-dimensional electrophoresis, mass spectrometry, and immunoassay methods. For nucleic acid markers, these include mRNA expression profiles, microRNA profiles, FISH, serial analysis of gene expression (SAGE), large scale gene expression arrays, gene sequencing and genotyping (SNP or small variant analysis).

The utility of two-dimensional electrophoresis is limited by low detection sensitivity; issues with protein solubility, charge, and hydrophobicity; gel reproducibility; and the possibility of a single spot representing multiple proteins. For mass spectrometry, depending on the format used, limitations revolve around the sample processing and separation, sensitivity to low abundance proteins, signal to noise considerations, and inability to immediately identify the detected protein. Limitations in immunoassay approaches to biomarker discovery are centered on the inability of antibody-based multiplex assays to measure a large number of analytes. One might simply print an array of high-quality antibodies and, without sandwiches, measure the analytes bound to those antibodies. (This would be the formal equivalent of using a whole genome of nucleic acid sequences to measure by hybridization all DNA or RNA sequences in an organism or a cell. The hybridization experiment works because hybridization can be a stringent test for identity. Even very good antibodies are not stringent enough in selecting their binding partners to work in the context of blood or even cell extracts because the protein ensemble in those matrices have extremely different abundances.) Thus, one must use a different approach with immunoassay-based approaches to biomarker discovery - one would need to use multiplexed ELISA assays (that is, sandwiches) to get sufficient stringency to measure many analytes simultaneously to decide which analytes are indeed biomarkers. Sandwich immunoassays do not scale to high content, and thus biomarker discovery using stringent sandwich immunoassays is not possible using standard array formats. Lastly, antibody reagents are subject to substantial lot variability and reagent instability. The instant platform for protein biomarker discovery overcomes this problem.

Many of these methods rely on or require some type of sample fractionation prior to the analysis. Thus the sample preparation required to run a sufficiently powered study designed to identify and discover statistically relevant biomarkers in a series of well-defined sample populations is extremely difficult, costly, and time consuming. During fractionation, a wide range of variability can be introduced into the various samples. For example, a potential marker could be unstable to the process, the concentration of the marker could be changed, inappropriate aggregation or disaggregation could occur, and inadvertent sample contamination could occur and thus obscure the subtle changes anticipated in early disease.

It is widely accepted that biomarker discovery and detection methods using these technologies have serious limitations for the identification of diagnostic or predictive biomarkers. These limitations include an inability to detect low-abundance biomarkers, an inability to consistently cover the entire dynamic range of the proteome, irreproducibility in sample processing and fractionation, and overall irreproducibility and lack of robustness of the method. Further, these studies have introduced biases into the data and not adequately addressed the complexity of the sample populations, including appropriate controls, in terms of the distribution and randomization required to identify and validate biomarkers within a target disease population.

Although efforts aimed at the discovery of new and effective biomarkers have gone on for several decades, the efforts have been largely unsuccessful. Biomarkers for various diseases typically have been identified in academic laboratories, usually through an accidental discovery while doing basic research on some disease process. Based on the discovery and with small amounts of clinical data, papers were published that suggested the identification of a new biomarker. Most of these proposed biomarkers, however, have not been confirmed as real or useful biomarkers, primarily because the small number of clinical samples tested provide only weak statistical proof that an effective biomarker has in fact been found. That is, the initial identification was not rigorous with respect to the basic elements of statistics. In each of the years 1994 through 2003, a search of the scientific literature shows that thousands of references directed to biomarkers were published. During that same time frame, however, the FDA approved for diagnostic use, at most, three new protein biomarkers a year, and in several years no new protein biomarkers were approved.

Based on the history of failed biomarker discovery efforts, theories have been proposed that further promote the general understanding that biomarkers for diagnosis, prognosis or prediction of risk of developing diseases and conditions are rare and difficult to find. Biomarker research based on 2D gels or mass spectrometry supports these notions. Very few useful biomarkers have been identified through these approaches. However, it is usually overlooked that 2D gel and mass spectrometry measure proteins that are present in blood at approximately 1 nM concentrations and higher, and that this ensemble of proteins may well be the least likely to change with disease or the development of a particular condition. Other than the instant biomarker discovery platform, proteomic biomarker discovery platforms that are able to accurately measure protein expression levels at much lower concentrations do not exist.

Much is known about biochemical pathways for complex human biology. Many biochemical pathways culminate in or are started by secreted proteins that work locally within the pathology; for example, growth factors are secreted to stimulate the replication of other cells in the pathology, and other factors are secreted to ward off the immune system, and so on. While many of these secreted proteins work in a paracrine fashion, some operate distally in the body. One skilled in the art with a basic understanding of biochemical pathways would understand that many pathology-specific proteins ought to exist in blood at concentrations below (even far below) the detection limits of 2D gels and mass spectrometry. What must precede the identification of this relatively abundant number of disease biomarkers is a proteomic platform that can analyze proteins at concentrations below those detectable by 2D gels or mass spectrometry.

As is discussed above, cardiovascular events may be prevented by aggressive treatment if the propensity for such events can be accurately determined. Existing multi-marker tests either require the collection of multiple samples from an individual or require that a sample be partitioned between multiple assays. Optimally, an improved test would require only a single blood, urine or other sample, and a single assay. Accordingly, a need exists for biomarkers, methods, devices, reagents, systems, and kits that enable the prediction of Cardiovascular Events within a 5 year period.

WO2011/059721 described protein and lipid biomarkers providing consistent improvement to the prediction of type 2 diabetes.

US2011/0003707 described a multi-marker immunoassay, including cardiac pathology and vascular inflammation biomarkers for early detection of congestive heart failure (CHF).

### SUMMARY OF THE INVENTION

The invention is as defined in the appended claims.

The present application includes biomarkers, methods, reagents, devices, systems, and kits for the prediction of risk of having a Cardiovascular (CV) Event within a 5 year period. The biomarkers of the present application were identified using a multiplex SOMAmer-based assay which is described in detail in Examples 1 and 2. By using the SOMAmer-based biomarker identification method described herein, this application describes a surprisingly large number of CV event biomarkers that are useful for the prediction of CV events. The sample population used to discover biomarkers associated with the risk of a CV event was from the Heart & Soul Study, a prospective cohort study examining coronary artery disease progression in a population with pre-existing CV disease, including prior myocardial infarction, evidence of greater than 50% stenosis in 1 or more coronary vessels, exercise-induced ischemia by treadmill or nuclear testing or prior coronory revascularization. The participants were recruited from the San Francisco Bay Area. The CV event type and time for the study population are shown in Table 4. In identifying these CV event biomarkers, over 1000 proteins from over 900 individual samples were measured, some of which were at concentrations in the low femtomolar range. This is about four orders of magnitude lower than biomarker discovery experiments done with 2D gels and/or mass spectrometry.

While certain of the described CV event biomarkers are useful alone for prediction of risk of having a CV event, methods are described herein for the grouping of multiple subsets of the CV event biomarkers that are useful as a panel of biomarkers. Once an individual biomarker or subset of biomarkers has been identified, the prediction of risk of a CV event in an individual can be accomplished using any assay platform or format that is capable of measuring differences in the levels of the selected biomarker or biomarkers in a biological sample.

However, it was only by using the SOMAmer-based biomarker identification method described herein, wherein over 1000 separate potential biomarker values were individually screened from a large number of individuals having previously been diagnosed either as having or not having a CV event within a 5 year time frame, that it was possible to identify the CV event biomarkers disclosed herein. This discovery approach is in stark contrast to biomarker discovery from tissue samples, conditioned media or lysed cells as it queries a more patient-relevant system that requires no translation to human pathology. Furthermore, this form of blood-based measurement is far more applicable clinically.

Thus, in one aspect of the instant application, one or more biomarkers are provided for use either alone or in various combinations to predict the risk of the occurrence of a CV event within a 5 year time frame. Exemplary embodiments include the biomarkers provided in Table 1, Col. 7, "PUBLIC_ NAME", which as noted above, were identified using a multiplex SOMAmer-based assay, as described generally in Example 1 and more specifically in Example 2. The markers provided in Table 1 are useful in the prediction of risk of having a CV event within a 5 year time period. The biomarkers from Table 2 and Table 3, respectively, demonstrate the reduction of the 155 biomarkers from Table 1 to a smaller number which performs the same task with less technical complexity and cost; however, other combinations with similar efficacy may be compiled from Table 1.

While certain of the described CV event risk biomarkers are useful alone for the prediction of risk of a CV event within 5 years, methods are also described herein for the grouping of multiple subsets of the CV event risk biomarkers that are each useful as a panel of two or more biomarkers. Thus, various embodiments of the instant application provide combinations comprising N biomarkers, wherein N is at least two biomarkers. In other embodiments, N is selected to be any number from 2-155 biomarkers.

In yet other embodiments, N is selected to be any number from 2-7, 2-10, 2-15, 2-20, 2-25, 2-30, 2-35, 2-40, 2-45, 2-50, 2-55, or in successive increments of 5 for the upper limit of the range, up to and including 2-155. In other embodiments, N is selected to be any number from 3-7, 3-10, 3-15, 3-20, 3-25, 3-30, 3-35, 3-40, 3-45, 3-50, 3-55, or in successive increments of 5 for the upper limit of the range, up to and including 3-155. In other embodiments, N is selected to be any number from 4-7, 4-10, 4-15, 4-20, 4-25, 4-30, 4-35, 4-40, 4-45, 4-50, 4-55, or in successive increments of 5 for the upper limit of the range, up to and including 4-155. In other embodiments, N is selected to be any number from 5-7, 5-10, 5-15, 5-20, 5-25, 5-30, 5-35, 5-40, 5-45, 5-50, 5-55, or in successive increments of 5 for the upper limit of the range, up to and including 5-155. In other embodiments, N is selected to be any number from 6-10, 6-15, 6-20, 6-25, 6-30, 6-35, 6-40, 6-45, 6-50, 6-55, or in successive increments of 5 for the upper limit of the range, up to and including 6-155. In other embodiments, N is selected to be any number from 7-10, 7-15, 7-20, 7-25, 7-30, 7-35, 7-40, 7-45, 7-50, 7-55, or in successive increments of 5 for the upper limit of the range, up to and including 7-155. In other embodiments, N is selected to be any number from 8-10, 8-15, 8-20, 8-25, 8-30, 8-35, 8-40, 8-45, 8-50, 8-55, or in successive increments of 5 for the upper limit of the range, up to and including 8-155. In other embodiments, N is selected to be any number from 9-15, 9-20, 9-25, 9-30, 9-35, 9-40, 9-45, 9-50, 9-55, or in successive increments of 5 for the upper limit of the range, up to and including 9-155. In other embodiments, N is selected to be any number from 10-15, 10-20, 10-25, 10-30, 10-35, 10-40, 10-45, 10-50, 10-55, or in successive increments of 5 for the upper limit of the range, up to and including 10-155. It will be appreciated that N can be selected to encompass similar, but higher order, ranges.

As discussed above, cardiovascular events may be avoided by aggressive treatment if the propensity for such events can be accurately determined. Prior art multi-marker tests either require the collection of multiple samples from an individual, or require that a sample be partitioned between multiple assays. It would be preferred to provide a prognostic assay that would require only a single biological sample, measured in a single assay, rather than multiple samples for different analyte types (lipids, proteins, metabolites) or panels of analytes. The central benefit to a single sample test is simplicity at the point of use, since a test with multiple sample collections is more complex to administer and this forms a barrier to adoption. An additional advantage derives from running that single sample in a single assay for multiple proteins. A single assay should mitigate unwanted variation due to calibrating multiple assay results together. The test which forms the basis of this application is such a "single sample, single assay" test. This combination of single sample and single assay is a novel feature of this cardiovascular event risk test which addresses the logistic complexity of collecting multiple samples and the problems and biohazards involved in splitting samples into multiple aliquots for multiple independent analytical procedures.

Cardiovascular disease is known to involve multiple biological processes and tissues. Well known examples of biological systems and processes associated with cardiovascular disease are inflammation, thrombosis, disease-associated angiogenesis, platelet activation, macrophage activation, liver acute response, extracellular matrix remodeling, and renal function. These processes can be observed as a function of gender, menopausal status, and age, and according to status of coagulation and vascular function. Since these systems communicate partially through protein based signaling systems, and multiple proteins may be measured in a single blood sample, the invention provides a single sample, single assay multiple protein based test focused on proteins from the specific biological systems and processes involved in cardiovascular disease.

As is discussed herein, one of the central functions of measuring risk for a cardiovascular event is to enable the assessment of progress in response to treatment and behavioral changes such as diet and exercise. Current risk prediction methods such as the Framingham equation, include clearly correlated clinical covariate information, the largest such factor is the age of the subject. This makes the Framingham equation less useful for monitoring the change in an individual's risk, although it may be accurate for a population. A novel feature of this CV event risk test is that it does not require age as a part of the prognostic model. The subject invention is based on the premise that, within the biology of aging, there are causal factors which are variable and thus better used to assess risk. The invention is premised on the belief that age itself is not a causal factor in the disease, and that age is acting as a surrogate or proxy for the underlying biology. While age is indeed prognostic of CV events, it cannot be used to assess individual improvement, and presumably the effect of age is mediated through biological function. This effect can be better determined through measurement of the relevant biology. In this invention, the proteins that are targeted are involved in the biology of the disease. Thus, the invention captures the biological information that is reflected in the correlation between age and risk of a CV event. In fact, adding a factor for age to our model for risk based on proteins does not improve performance in predicting events.

The strategy to identify proteins from multiple processes involved in cardiovascular disease necessitated choosing parameters that provided a wide range/diversity of CV disease patients presenting with a variety of events or symptoms. Events due to cardiovascular disease are heterogeneous, involving two main classes of event: thrombotic and CHF related events. Some presenting events may lack specific diagnostic information (e.g., death at home). In view of these characteristics of CV disease, the inventive test was developed by measuring proteins involved from the biological processes associated with CV disease, on blood samples from a broad range of events. This strategy resulted in the inclusion of information from multiple processes involved in the disease (e.g., angiogenesis, platelet activation, macrophage activation, liver acute response, other lymphocyte inflammation, extracellular matrix remodeling, and renal function). In order to develop a multiple protein based prognostic single sample test for CV disease, the chosen study population was a high risk group of subjects from the "Heart & Soul" study. By choosing this set of subjects with a high rate of CV events, it was possible to determine risk associated with protein measurements more accurately than would have been possible in the general population (within which events are rarer). The development of the subject test on this high risk group, permitted identification of protein biomarker combinations that could be generalized due to common biology. As a result, the subject inventive test and biomarkers are likely to be effective beyond event prediction in a larger population than those individuals matching the entry criteria of the "Heart & Soul" study.

As is mentioned above, CV disease involves the blood coagulation system, inflammatory white blood cells and platelet activation. The signals from the activation of these systems in the body can be obscured due to common errors in sample preparation which lead to platelets and white blood cells being only partially spun down from plasma samples. If these cells are not completely spun down, they may be lysed by freeze-thaw when the samples are shipped and assayed. During the course of identification of the subject biomarkers, it became apparent that in at least some cases, conventionally prepared samples contain whole cells and platelets after freeze-thaw. During the subsequent proteomic assay, any whole cells would lyse and interfere with the detection of proteins characteristic of the disease processes of *in vivo* activation of platelets and monocytes. Thus, in one embodiment of the subject invention, an additional step of re-spinning the samples after thawing is conducted prior to the assay. This additional spin step can remove platelets and monocytes which would otherwise prevent the identification of biomarkers related to platelet and monocyte activation. The additional step to remove the insoluble and cellular components of the samples (by spinning or filtering), represents an advance for a cardiovascular event risk test which is believed to not be described in the prior art.

While there are specific proteins from the literature which are known to be prognostic for CV events such as apolipoprotein B, apolipoprotein A-1, BNP and CRP, and which have known associations to CV disease, it has not been clear that a particular set of protein measurements can be combined to perform optimally in terms of prediction performance due to common biological information being represented by multiple protein measurements. A specific example of how combinations of proteins observed to vary during CV disease may not provide an optimal prediction performance, involves the many small serum proteins excreted in urine which are related to renal function as measured by Glomerular Filtration Rate (GFR). Poor renal function, as indicated by low GFR, is related to cardiovascular risk. Thus, the many small proteins associated with low GFR, appear to be related to CV disease, but not independently. During the development of the subject test, corrections were made to protein measurements for estimated GFR in order to determine which proteins provided additional prognostic value beyond GFR.

The measurement of GFR is clearly useful in predicting the risk of a CV event. However, the clinical measurement of GFR involves urine collection over 24 hours, which does not meet the subject standard of a "single sample, single assay" test. Other estimates of GFR are less onerous; however, to meet the goal of a "single sample" prognostic test, the strategy underlying the subject invention sought the use of the protein measurements themselves to provide GFR information for the risk analysis. For example, in the Table 3 ten marker model, the protein ESAM strongly predicts CV event risk due to its correlation with GFR. After correcting the measurements of the protein ESAM to remove the correlation with estimated GFR, ESAM is no longer predictive of risk. This use of a protein such as ESAM to convey the biological signal related to GFR in a "single sample, single assay" represents a novel advance for the prognosis of a CV event.

The identification of the Table 3 biomarkers involved selection for proteins that could work together in the prognosis of a CV event. Studies, such as Wang, T. et al., (2006) N. Eng. J. Med. 355:2631-9, have shown that combinations of biomarkers often fail to provide additional performance over simple risk calculations using common clinical information such as age and lipid levels. In order to avoid an *ad hoc* combination of biomarkers, the subject invention provides a statistical analysis procedure in which proteins were screened for both for their individual prognostic power, and also, crucially, the capability of the proteins to work together synergistically to improve the prognostic value of the combination. Multiple independent biological processes are represented in the Table 3 ten marker protein model provided herein.

In one embodiment, the invention comprises a method for evaluating the risk of a future cardiovascular (CV) Event within a 5 year time period in a population. This method comprises the detection, in a biological sample from an individual of the population, biomarker values that each correspond to one of at least N biomarkers selected from Table 1, wherein the risk for an individual of a CV event is evaluated, based on the biomarker values, and wherein N = 2 - 155. In another embodiment, the biomarkers are selected from Table 2 and N=2-46. In another embodiment, the biomarkers are selected from Table 3 and N=2-10.

In one embodiment, the selection of a population is such that the population is characterized as having no prior history of cardiovascular disease. In the alternative, the population may be selected such that it is characterized as having a prior history of cardiovascular disease.

The prior history can comprise prior myocardial infarction, angiographic evidence of greater than 50% stenosis in 1 or more coronary vessles, exercise-induced ischemia by treadmill or nuclear testing or prior coronary revascularization.

Further, the population may be selected such that it is characterized by genetic risk factors comprising mutations, single nucleotide polymorphisms and insertion/deletions. Such genetic risk factors can be used to complement the evaluation of risk.

The evaluation of risk of a CV event can be measured on a dynamic scale that is responsive to change over time in response to interventions comprising therapeutics, nutritional programs, supplementation, lifestyle modification, smoking cessation programs and disease management protocols.

The foregoing methods relating to evaluation of CV event risk over a 5 year period can be used to allocate the individuals into increased or decreased disease management programs, based on their biomarker values. The method can also be used to stratify the individuals into different risk bands relating to life insurance coverage depending on said biomarker value. Also, it can be used for evaluation of CV event risk in order to stratify the individuals into different risk bands relating to health insurance coverage depending on said biomarker value. Additionally, it can be used to assess potential candidates for partnership depending on biomarker values.

Further, the foregoing methods for CV event risk prediction can be used to: predict medical resource consumption of the population based on the biomarker values; use the biomarker value of the individual as an entry criterion for clinical trials of CV therapeutics; prediction of efficacy of clinical trial results based on said biomarker value; use the biomarker value for cardiovascular safety surveillance of a CV therapeutic or any therapeutic agent; use the biomarker value as a surrogate endpoint of efficacy of CV therapeutics; and/or monitor compliance with any intervention, dietary or therapeutic protocol based on said biomarker value. In regard to the CV safety surveillance of a CV therapeutic or any therapeutic agent, such surveillance is important in large, costly, required phase 3 CV safety studies for CV therapeutic and non-cardiovascular drugs for nearly every chronic use.

The subject method for evaluating a CV event risk can also be used to select or refer the individual for other diagnostic procedures based on said biomarker value. Additionally, the subject method can be used to select a CV therapeutic based on biomarker value.

In the subject method for evaluation of CV event risk, the biomarker values can be detected by performing an *in vitro* assay. The *in vitro* assay can involve at least one capture reagent corresponding to each of the biomarkers, and further can include at least one capture reagent from the group consisting of SOMAmers, antibodies, and a nucleic acid probe. In a preferred embodiment, the capture reagent is a SOMAmer.

In another embodiment, the *in vitro* assay can be selected from the group consisting of an immunoassay, a SOMAmer-based assay, a histological or cytological assay, and an mRNA expression level assay.

In the subject methods, the biological sample can be whole blood, plasma, serum, urine or the like. In a preferred embodiment, the biological sample is serum, plasma or urine.

Additionally, it is provided that in the subject methods the individual can be a mammal, and in particular, a human.

In alternative embodiments of the subject methods, N = 3 - 10; N = 3 - 15; N = 2 - 10; N = 4 - 10; or N = 5 - 10.

In another embodiment, the disclosure provides for a computer-implemented method for evaluating the risk of a cardiovascular (CV) Event. This method can include retrieving on a computer biomarker information for an individual, wherein the biomarker information comprises biomarker values that each correspond to one of at least N biomarkers selected from Table 1; performing with the computer a classification of each of the biomarker values; and indicating a result of the evaluation of risk for a CV event for said individual based upon a plurality of classifications, and wherein N = 2 - 155. In alternate embodiments of this method, the biomarkers can be selected from Table 2 (with N=2-46) or Table 3 (with N=2-10).

The result of the evaluation of risk of a CV event for the individual can be displayed a computer display.

In another embodiment, the disclosure comprises a computer program product for evaluating the risk of a CV event. The computer program product can include a computer readable medium embodying program code executable by a processor of a computing device or system, where the program code comprises: code that retrieves data attributed to a biological sample from an individual, wherein the data comprises biomarker values that each correspond to one of at least N biomarkers selected from Table 1, where the biomarkers were detected in the biological sample; and code that executes a classification method that indicates a result of the evaluation of risk for a CV event of the individual as a function of said biomarker values. When the biomarkers are selected from Table 1, col. 7, N = 2 - 155. In other embodiments, the biomarkers can be selected from Table 2 (with N=2-46) or from Table 3 (with N=2-10).

The classification method can use a continuous score or measure or risk metric. The classification method can also use two or more classes.

The subject disclosure further comprises a method for screening an individual for evaluation of risk of a CV event. This method comprises the detection, in a biological sample from an individual, biomarker values that each correspond to one of at least N biomarkers selected from Table 1, wherein the individual is evaluated for risk for a CV event based on said biomarker values, and wherein N = 2 - 155. In other embodiments the biomarkers can be selected from Table 2 (with N=2-46) or from Table 3 (with N=2-10), respectively.

In the subject methods, the detection of the biomarker values can be done in an *in vitro* assay. Such *in vitro* assay can include at least one capture reagent corresponding to each of the biomarkers, and can further comprise the selection of the at least one capture reagent from the group consisting of SOMAmers, antibodies, and a nucleic acid probe. Preferably, the at least one capture reagent is a SOMAmer. The *in vitro* assay can be selected from an immunoassay, a SOMAmer-based assay, a histological or cytological assay, and an mRNA expression level assay.

The biological sample can be selected from whole blood, plasma, serum, urine and the like. Preferably, the biological sample is serum, plasma or urine. In the subject method, the individual can be a mammal, and is preferably a human.

In one of the embodiments of the subject disclosure, it is provided that the individual is evaluated for risk of a CV event, based on said biomarker values and at least one item of additional biomedical information corresponding to said individual. At least one item of additional biomedical information can include, but is not limited to, any of the following: (a) information corresponding to the presence of cardiovascular risk factors including one or more of a prior myocardial infarction, angiographic evidence of greater than 50% stenosis in one or more coronary vessels, exercise-induced ischemia by treadmill or nuclear testing or prior coronary revascularization; (b) information corresponding to physical descriptors of said individual; (c) information corresponding to a change in weight of said individual; (d) information corresponding to the ethnicity of said individual; (e) information corresponding to the gender of said individual; (f) information corresponding to said individual's smoking history; (g) information corresponding to said individual's alcohol use history; (h) information corresponding to said individual's occupational history; (i) information corresponding to said individual's family history of cardiovascular disease or other circulatory system conditions; (j) information corresponding to the presence or absence in said individual of at least one genetic marker correlating with a higher risk of cardiovascular disease in said individual or a family member of said individual; (k) information corresponding to clinical symptoms of the individual; (1) information corresponding to other laboratory tests; (m) information corresponding to gene expression values of the individual; (n) information corresponding to said individual's consumption of known cardiovascular risk factors such as diet high in saturated fats, high salt; (o) information corresponding to the individual's imaging studies including electrocardiogram, echocardiography, carotid ultrasound for intima-media thickness, flow mediated dilation, pulse wave velocity, ankle-brachial index, stress echocardiography, myocardial perfusion imaging, coronary calcium by CT, high resolution CT angiography, MRI imaging, and other imaging modalities; and (p) information about medications .

The disclosure further comprises a panel of biomarkers for evaluating the risk of a future CV event within a five year time period, wherein the panel comprises N biomarkers selected from the group consisting of the biomarkers of Table 1, wherein N = 2 - 155. In alternative embodiments, the biomarkers are selected from Table 2, wherein N = 2 - 46 or from Table 3, wherein N = 2 - 10.

In another aspect, the disclosure comprises a method for screening an individual in a population by evaluating or prognosing the risk of a future CV event within a 5 year period, by detecting, in a biological sample from the individual, a biomarker value for angiopoietin 2, and determining the risk of a future CV event on the basis of the angiopoietin 2 biomarker value. The biomarker value can be expressed as a measurement score or a classification into one of a plurality of classifications.

In a variation of the screening method using angiopoietin 2, the subject disclosure also comprises adding to the method, before the determining step, the step comprising providing information regarding the individual's use of a statin. Thus, the determining of the risk of a future CV event is on the basis of the angiopoietin 2 biomarker value and the statin information.

The angiopoietin 2 is surprisingly useful in prognosing a secondary cardiovascular event for individuals on statins. Statins have been reported in the prior art to not only reduce the risk of a secondary cardiovascular event, but also cause an increase in angiopoietin 2. This rise in angiopoietin 2 would have been expected to negate it's use as a biomarker. Unexpectedly, angiopoietin 2 has demonstrated that it is a good marker for prediction of secondary cardiovascular events in high risk individuals.

The method of detecting angiopoietin 2 biomarker value can comprise the further step of: detecting in the biological sample a biomarker value for one or more of MMP7, CHRDL1, MATN2, PSA-ACT biomarkers or a combination thereof. The method can additionally involve the detection, in the biological sample, of biomarker values that each correspond to N biomarkers selected from the biomarkers of Table 3, wherein N = 2-10.

In another aspect, the disclosure provides a panel of biomarkers for screening an individual in a population by evaluating or prognosing the risk of a future CV event within a 5 year period. The panel includes at least the angiopoietin 2 biomarker. This panel can further include one or more of the MMP7, CHRDL1, MATN2, PSA-ACT biomarkers or any combination thereof. In addition, the panel can include one or more biomarkers selected from Table 3, wherein N = 2-10.

In another embodiment, the disclosure provides a method for screening an individual by evaluating the risk of a future CV event within a 5 year period, wherein the evaluating comprises a differential prognosis of a thrombotic event or congestive heart failure (CHF) event. This method comprises: detecting in a biological sample from the individual of the population, biomarker values that each correspond to GPVI biomarker for prognosis of the thrombotic event, and MATN2 biomarker for the prognosis of the CHF event. The method can involve the further step of detecting in the biological sample biomarker values for N biomarkers selected from the group of biomarkers set forth in Table 3, wherein N = 3-10. The thrombotic event can include any of a myocardial infarction (MI), transient ischemic attack (TIA), stroke, acute coronary syndrome and a need for coronary re-vascularization.

Further provided is a panel of biomarkers for screening an individual in a population by evaluating or prognosing the risk of a future CV event within a 5 year period, wherein the panel comprises a GPVI biomarker and a MATN2 biomarker. The panel can additionally include at least one of N biomarkers selected from the group consisting of the biomarkers set forth in Table 3.

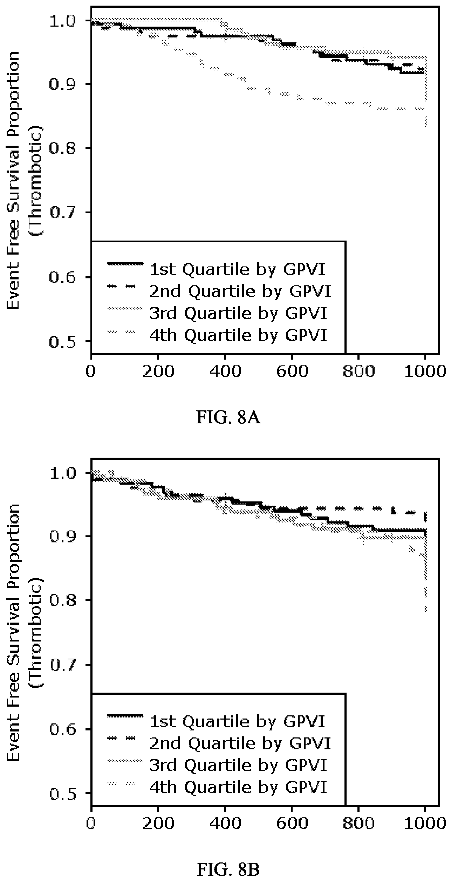

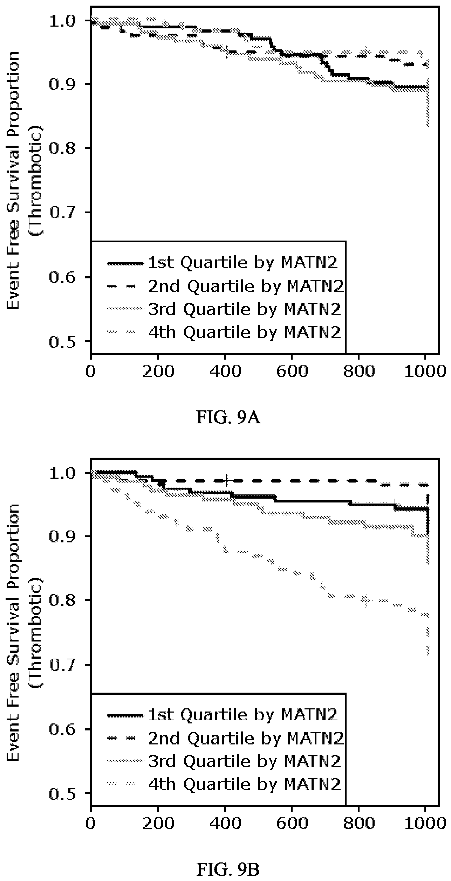

Multiple classes of treatment for CV disease are available, reflecting the variety of biological systems involved. For example, anti-thrombotic, platelet inhibitor, lipid metabolism, fluid and electrolyte balance medications, and beta blockers, have been used in the treatment of CV disease. In order to guide treatment, it is useful to identify not only the overall risk, but also distinguish the class of event indicated by the biology. The foregoing method using MATN2 and GPVI allows for distinguishing the probable event classes of thrombotic events and CHF events. The GPVI is more specific to the development of the thrombotic event, and the MATN2 is more specific to CHF events. The specifity of GPVI for thrombotic events is demonstrated in Figures 8A and 8B. The specificity of MATN2 for prognosis of CHF is illustrated in Figures 9A and 9B. These differences can be interpreted in terms of the related biological processes. This multiple protein based test can therefore provide the patient with information to distinguish the risk of developing CHF versus the risk of thrombotic events. This is a significant and important feature of the invention that is believed not to be described in the prior art.

In addition to providing prognosis of CV event risk based on protein measurements alone, the subject method also provides the advantage of a more complete picture derived from taking into account simple information such as gender, medication, other markers such as LDL cholesterol, HDL cholesterol, total cholesterol, and other conditions such as diabetes. Such models can be built upon the existing Table 3 ten protein model introduced here.

Further provided herein is a kit for screening an individual in a population by evaluating or prognosing the risk of a future CV event within a 5 year period. The kit includes the following components: at least one of the biomarkers set forth in Table 1; at least one corresponding capture reagent, wherein each of the corresponding capture reagents is specific to the selected biomarkers; and a signal generating material, said material being specific to the selected corresponding biomarkers and/or corresponding capture reagents, wherein each signal is activated upon binding of each capture reagent to the corresponding biomarker.

In another aspect, the kit of can comprise one or more biomarkers selected from the group consisting of: angiopoietin 2 biomarker; angiopoietin 2 biomarker and any biomarker selected from the group consisting of MMP7, CHRDL1, MATN2, PSA-ACT and any combination thereof; a biomarker selected from the group consisting of GPVI biomarker, MATN2 biomarker and any combination thereof; N biomarkers selected from the group of biomarkers set forth in Table 3, wherein N = 2-10; and any combination thereof.

The capture reagents of the kits can be any one or more of SOMAmers, antibodies, and nucleic acid probes or a combination thereof. The kit can also include instructions or one or more software or computer program products for classifying the individual from whom the biological sample was obtained, as either having or not having increased risk of a CV event.

In another embodiment, the subject disclosure comprises a classifier comprising the biomarkers of Table 1, col. 7, Table 2 or Table 3.

### BRIEF DESCRIPTION OF THE DRAWINGS

Figure 1A is a flowchart for an exemplary method for the prediction of a CV event in a biological sample. Figure 1B is a flowchart for an exemplary method for the prediction of a CV event in a biological sample using a naive Bayes classification method.
Figure 2A shows a Principal component analysis for a subgroup of cases with Events and controls with no CV events within 6 months. The cases with events are partially separated from the controls along the vertical axis.
Figure 2B shows a DSGA analysis for a subgroup of cases with Events and controls with no CV events within 6 months. The cases with events are partially separated from the controls along the horizontal axis.
Figure 3 provides a risk score analysis for the study population. This score was calculated by building a simple Cox proportional hazard model using the logarithm of the measurements of the ten proteins in Table 3. The population was split into quintiles based on this score. The Kaplan Meier plots in Figure 3 demonstrate how these quintiles differ in the proportion of the individual experiencing a cardiovascular event or death for various event types.
Figure 3A shows the Kaplan Meier plots of all deaths and cardiovascular events of the study population, with the population split into quintiles based on the Table 3 protein scores.
Figure 3B shows the Kaplan Meier plots for the cases of CV events: unclassified deaths, those deaths without a known proximal cause such as MI or CHF (congestive heart failure), with the population split into quintiles based on the Table 3 protein scores.
Figure 3C shows the Kaplan Meier plots for the cases of CV events: incident CHF with the population split into quintiles based on the Table 3 protein scores.
Figure 3D shows the Kaplan Meier plots for the cases of CV events: CHF recurrence for chronic CHF patients (those with a previous diagnosis of CHF) with the population split into quintiles based on the Table 3 protein scores.
Figure 3E shows the Kaplan Meier plots for the cases of CV events: thrombotic event (MI + stroke) with the population split into quintiles based on the Table 3 protein scores.
Figure 3F shows the Kaplan Meier plots for the cases of CV events: all CHF, with the population split into quintiles based on the Table 3 protein scores.
Figure 4 illustrates an exemplary computer system for use with various computer-implemented methods described herein.
Figure 5 is a flowchart for a method of indicating evaluating risk of a CV event in accordance with one embodiment.
Figure 6 is a flowchart for a method of evaluating risk of a CV event in accordance with one embodiment.

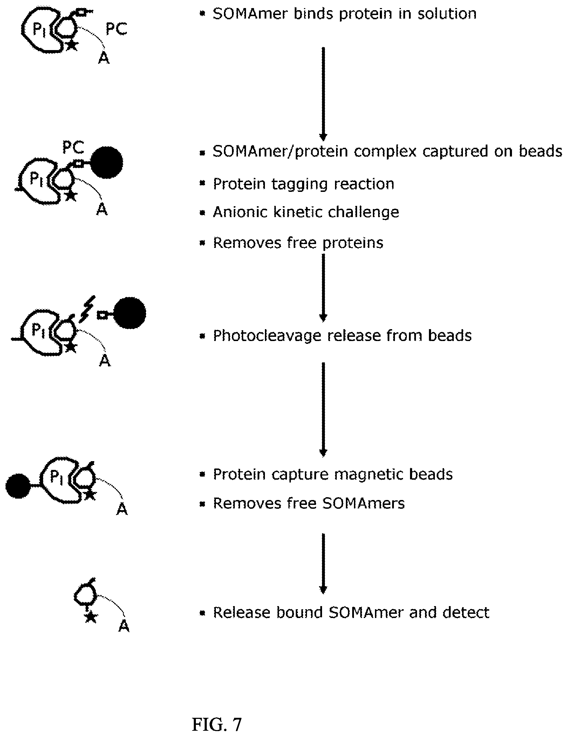

Figure 7 illustrates an exemplary aptamer assay that can be used to detect one or more CV event biomarkers in a biological sample.
Figure 8 shows the Kaplan Meier plots based on GPVI, one of the ten proteins in Table 3, demonstrating that this protein distinguishes between thrombotic and CHF events. The population is split into quartiles of GPVI. Figure 8A shows the highest quartile of GPVI is prognostic for thrombotic cardiovascular events. Figure 8B shows that the quartiles of GPVI have little or no discriminative ability to forecast CHF events.
Figure 9 shows the Kaplan Meier plots based on MATN2, one of the ten proteins in Table 3, demonstrating that this protein distinguishes between thrombotic and CHF events. The population is split into quartiles of MATN2. Figure 9A shows that the quartiles of MATN2 are not prognostic for thrombotic cardiovascular events. Figure 9B shows that individuals from the highest quartile of MATN2 have a higher rate of CHF events.
Figure 10 shows the Kaplan Meier plots of all 538 subjects taking statin medication showing that those individuals in the 4th quartile of the population distribution for angiopoietin-2 suffer cardiovascular events at an increased rate compared to those not in the 4th Quartile for angiopoietin-2. Thus despite the effects of treatment with statins, angiopoietin-2 is a useful biomarker of the risk of CV events.
Figure 11 shows the Kaplan Meier plots of all 538 subjects taking statin medication showing that CHRDL1 is associated with the event free survival of cardiovascular events in individuals treated with statin. Thus despite the effects of treatment with statins, CHRDL1 is a useful biomarker of the risk of CV events.

### DETAILED DESCRIPTION

Reference will now be made in detail to representative embodiments of the disclosure. While the disclosure will be described in conjunction with the enumerated embodiments, it will be understood that the invention is not intended to be limited to those embodiments. On the contrary, the invention is intended to cover all alternatives, modifications, and equivalents that may be included within the scope of the present invention as defined by the claims.

One skilled in the art will recognize many methods and materials similar or equivalent to those described herein, which could be used in and are within the scope of the practice of the present invention. The present invention is in no way limited to the methods and materials described.

Unless defined otherwise, technical and scientific terms used herein have the same meaning as commonly understood by one of ordinary skill in the art to which this invention belongs. Although any methods, devices, and materials similar or equivalent to those described herein can be used in the practice or testing of the invention, the preferred methods, devices and materials are now described.

All publications, published patent documents, and patent applications cited in this application are indicative of the level of skill in the art(s) to which the application pertains.

As used in this application, including the appended claims, the singular forms "a," "an," and "the" include plural references, unless the content clearly dictates otherwise, and are used interchangeably with "at least one" and "one or more." Thus, reference to "a SOMAmer" includes mixtures of SOMAmers, reference to "a probe" includes mixtures of probes, and the like.

As used herein, the term "about" represents an insignificant modification or variation of the numerical value such that the basic function of the item to which the numerical value relates is unchanged.

As used herein, the terms "comprises," "comprising," "includes," "including," "contains," "containing," and any variations thereof, are intended to cover a non-exclusive inclusion, such that a process, method, product-by-process, or composition of matter that comprises, includes, or contains an element or list of elements does not include only those elements but may include other elements not expressly listed or inherent to such process, method, product-by-process, or composition of matter.

The present application includes biomarkers, methods, devices, reagents, systems, and kits for the prediction of risk of CV events within a defined period of time, such as 5 years.

"Cardiovascular Event" means a failure or malfunction of any part of the circulatory system. In one embodiment, "Cardiovascular Event" means stroke, transient ischemic attack (TIA), myocardial infarction (MI), sudden death attributable to malfunction of the circulatory system, and/or heart failure. In another embodiment, "Cardiovascular Event" means any of the foregoing malfunctions and/or unstable angina, need for stent or angioplasty, or the like.

Cardiovascular Events include "Congestive Heart Failure" or "CHF" and "thrombotic events." Thrombotic Events include MIs, transient ischemic attacks (TIA), stroke, acute coronary syndrome and need for coronary re-vascularization.

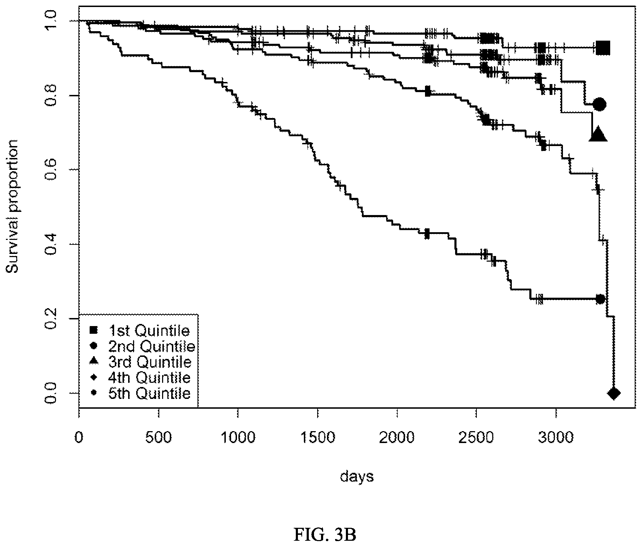

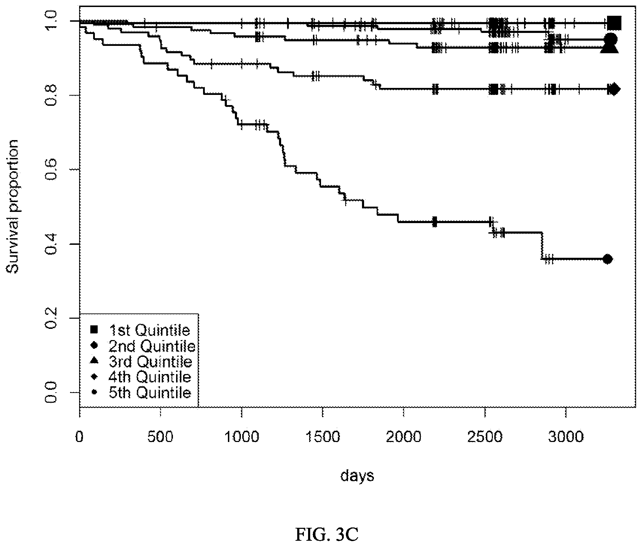

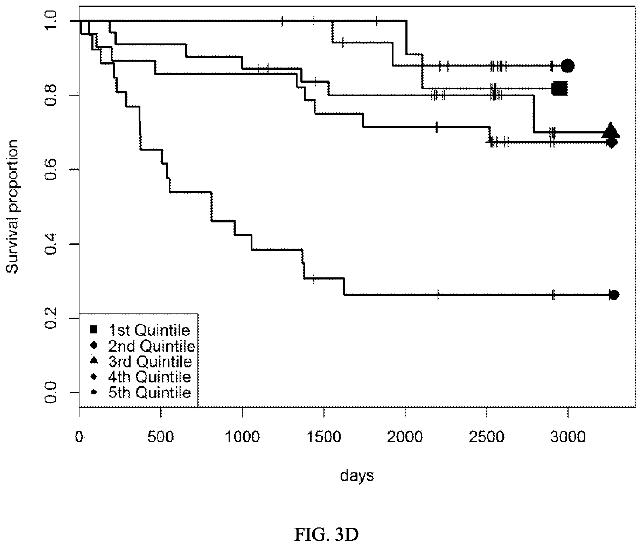

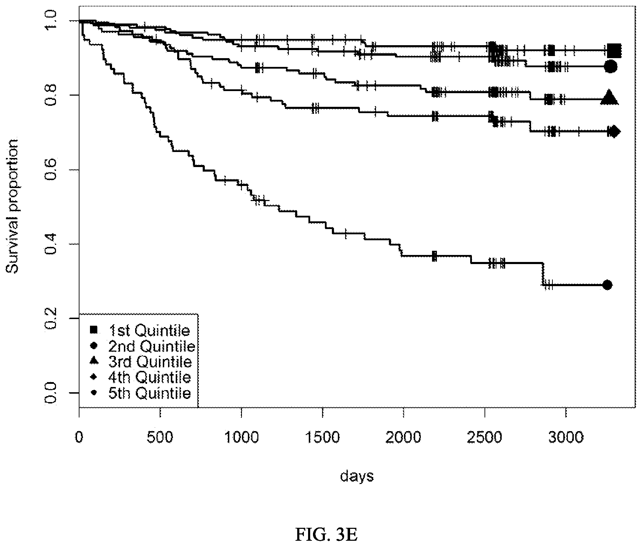

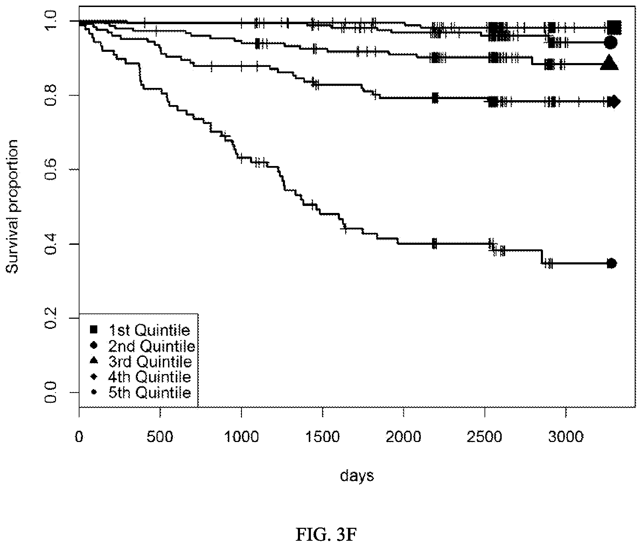

In one aspect, one or more biomarkers are provided for use either alone or in various combinations to evaluate the risk of a future CV event within a 5 year time period with CV events defined as myocardial infarction, stroke, death and congestive heart failure. Thrombotic events (Figure 3e) consist of myocardial infarction and stroke combined. As described in detail below, exemplary embodiments include the biomarkers provided in Table 1, Col. 7, which were identified using a multiplex SOMAmer-based assay that is described generally in Example 1 and more specifically in Example 2.

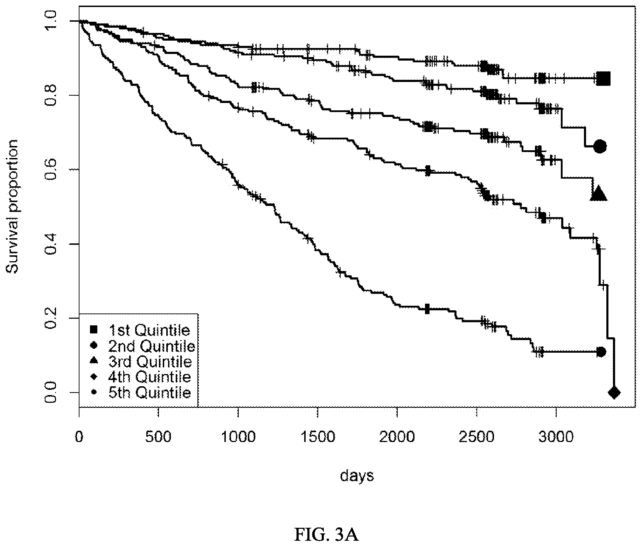

Table 1, Col. 7, sets forth the findings obtained from analyzing hundreds of individual blood samples from patients who have had a CV event within a 6 month - 10 year time frame (Event Positive) after initial blood draw (time point 1), and hundreds of equivalent individual blood samples from individuals who did not have a CV event within that time frame (Event Negative). The potential biomarkers were measured in individual samples rather than pooling the Event Positive and Event Negative blood samples; this allowed a better understanding of the individual and group variations in the phenotypes associated with the presence and absence of a CV event. Since over 1000 protein measurements were made on each sample, and several hundred samples from each of the Event Positive and the Event Negative populations were individually measured, the biomarkers reported in Table 1, Col. 7 resulted from an analysis of an uncommonly large set of data. The measurements were analyzed using the methods described in the section, "Classification of Biomarkers and Calculation of Risk Scores" herein. Table 1, Col. 7 lists the 155 biomarkers found to be useful in stratifying the population of individuals according to their propensity to exhibit a future CV event in the period of 0-5 years after blood sample was drawn. The Kaplan-Meier curves in Figures 3A-3F show a strong dependence of event risk upon quintile of a score determined by a small subset of such biomarkers, as listed in Table 3.

While certain of the described CV event biomarkers are useful alone for evaluating the risk of a CV event, methods are also described herein for the grouping of multiple subsets of the CV event biomarkers, where each grouping or subset selection is useful as a panel of three or more biomarkers, interchangeably referred to herein as a "biomarker panel" and a panel. Thus, various embodiments of the instant application provide combinations comprising N biomarkers, wherein N is at least two biomarkers. In other embodiments, N is selected from 2-155 biomarkers.

In yet other embodiments, N is selected to be any number from 2-7, 2-10, 2-15, 2-20, 2-25, 2-30, 2-35, 2-40, 2-45, 2-50, 2-55, or in successive increments of 5 for the upper limit of the range, up to and including 2-155. In other embodiments, N is selected to be any number from 3-7, 3-10, 3-15, 3-20, 3-25, 3-30, 3-35, 3-40, 3-45, 3-50, 3-55, or in successive increments of 5 for the upper limit of the range, up to and including 3-155. In other embodiments, N is selected to be any number from 4-7, 4-10, 4-15, 4-20, 4-25, 4-30, 4-35, 4-40, 4-45, 4-50, 4-55, or in successive increments of 5 for the upper limit of the range, up to and including 4-155. In other embodiments, N is selected to be any number from 5-7, 5-10, 5-15, 5-20, 5-25, 5-30, 5-35, 5-40, 5-45, 5-50, 5-55, or in successive increments of 5 for the upper limit of the range, up to and including 5-155. In other embodiments, N is selected to be any number from 6-10, 6-15, 6-20, 6-25, 6-30, 6-35, 6-40, 6-45, 6-50, 6-55, or in successive increments of 5 for the upper limit of the range, up to and including 6-155. In other embodiments, N is selected to be any number from 7-10, 7-15, 7-20, 7-25, 7-30, 7-35, 7-40, 7-45, 7-50, 7-55, or in successive increments of 5 for the upper limit of the range, up to and including 7-155. In other embodiments, N is selected to be any number from 8-10, 8-15, 8-20, 8-25, 8-30, 8-35, 8-40, 8-45, 8-50, 8-55, or in successive increments of 5 for the upper limit of the range, up to and including 8-155. In other embodiments, N is selected to be any number from 9-15, 9-20, 9-25, 9-30, 9-35, 9-40, 9-45, 9-50, 9-55, or in successive increments of 5 for the upper limit of the range, up to and including 9-155. In other embodiments, N is selected to be any number from 10-15, 10-20, 10-25, 10-30, 10-35, 10-40, 10-45, 10-50, 10-55, or in successive increments of 5 for the upper limit of the range, up to and including 10-155. It will be appreciated that N can be selected to encompass similar, but higher order, ranges.

In one embodiment, the number of biomarkers useful for a biomarker subset or panel is based on the sensitivity and specificity value for the particular combination of biomarker values. The terms "sensitivity" and "specificity" are used herein with respect to the ability to correctly classify an individual, based on one or more biomarker values detected in their biological sample, as having an increased risk of having a CV Event within 5 years or not having increased risk of having a CV event within the same time period. "Sensitivity" indicates the performance of the biomarker(s) with respect to correctly classifying individuals that have increased risk of a CV event. "Specificity" indicates the performance of the biomarker(s) with respect to correctly classifying individuals who do not have increased risk of a CV event. For example, 85% specificity and 90% sensitivity for a panel of markers used to test a set of Event Negative samples and Event Positive samples indicates that 85% of the control samples were correctly classified as Event Negative samples by the panel, and 90% of the Event Positive samples were correctly classified as Event Positive samples by the panel.

In an alternate method, scores may be reported on a continuous range, with a threshold of high, intermediate or low risk of a CV event, with thresholds determined based on clinical findings.

The CV event risk biomarkers identified herein represent a exceedingly large number of choices for subsets or panels of biomarkers that can be used to predict the risk of a CV event. Selection of the desired number of such biomarkers depends on the specific combination of biomarkers chosen. It is important to remember that panels of biomarkers for predicting CV event risk may also include biomarkers not found in Table 1, Col. 7, and that the inclusion of additional biomarkers not found in Table 1, Col. 7 may reduce the number of biomarkers in the particular subset or panel that is selected from Table 1, Col. 7. The number of biomarkers from Table 1, Col. 7 used in a subset or panel may also be reduced if additional biomedical information is used in conjunction with the biomarker values to establish acceptable threshold values for a given assay.

Another factor that can affect the number of biomarkers to be used in a subset or panel of biomarkers is the procedures used to obtain biological samples from individuals who are being assessed for risk of a CV event. In a carefully controlled sample procurement environment, the number of biomarkers necessary to meet desired sensitivity and specificity and/or threshold values will be lower than in a situation where there can be more variation in sample collection, handling and storage.

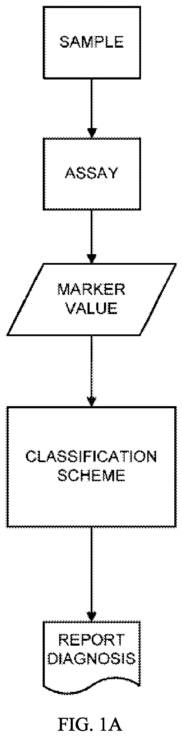

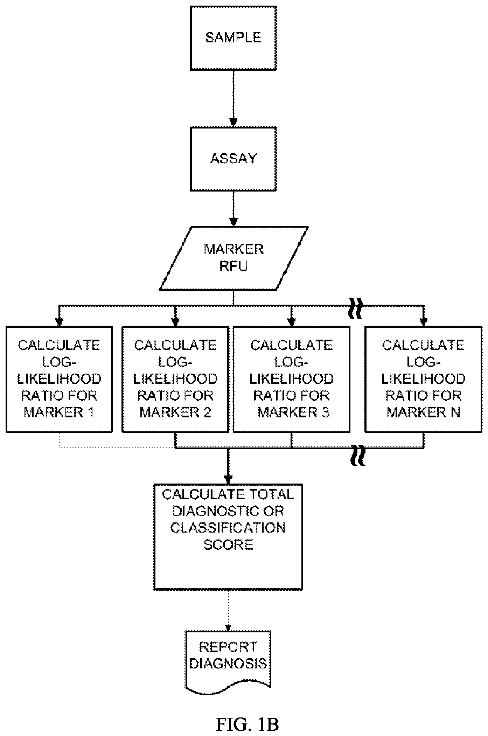

One aspect of the instant application can be described generally with reference to Figures 1A and 1B. A biological sample is obtained from an individual or individuals of interest. The biological sample is then assayed to detect the presence of one or more (N) biomarkers of interest and to determine a biomarker value for each of said N biomarkers (referred to in Figure 1B as marker RFU). Once a biomarker has been detected and a biomarker value assigned each marker is scored or classified as described in detail herein. The marker scores are then combined to provide a total diagnostic score, which indicates the likelihood that the individual from whom the sample was obtained has high, medium or low risk of a CV event, particularly when reported on a continuous range.

"Biological sample", "sample", and "test sample" are used interchangeably herein to refer to any material, biological fluid, tissue, or cell obtained or otherwise derived from an individual. This includes blood (including whole blood, leukocytes, peripheral blood mononuclear cells, buffy coat, plasma, and serum), dried blood spots (e.g., obtained from infants), sputum, tears, mucus, nasal washes, nasal aspirate, breath, urine, semen, saliva, peritoneal washings, ascites, cystic fluid, meningeal fluid, amniotic fluid, glandular fluid, pancreatic fluid, lymph fluid, pleural fluid, nipple aspirate, bronchial aspirate, bronchial brushing, synovial fluid, joint aspirate, organ secretions, cells, a cellular extract, and cerebrospinal fluid. This also includes experimentally separated fractions of all of the preceding. For example, a blood sample can be fractionated into serum, plasma or into fractions containing particular types of blood cells, such as red blood cells or white blood cells (leukocytes). If desired, a sample can be a combination of samples from an individual, such as a combination of a tissue and fluid sample. The term "biological sample" also includes materials containing homogenized solid material, such as from a stool sample, a tissue sample, or a tissue biopsy, for example. The term "biological sample" also includes materials derived from a tissue culture or a cell culture. Any suitable methods for obtaining a biological sample can be employed; exemplary methods include, e.g., phlebotomy, swab (e.g., buccal swab), and a fine needle aspirate biopsy procedure. Exemplary tissues susceptible to fine needle aspiration include lymph node, lung, lung washes, BAL (bronchoalveolar lavage),thyroid, breast, pancreas and liver. Samples can also be collected, e.g., by micro dissection (e.g., laser capture micro dissection (LCM) or laser micro dissection (LMD)), bladder wash, smear (e.g., a PAP smear), or ductal lavage. A "biological sample" obtained or derived from an individual includes any such sample that has been processed in any suitable manner after being obtained from the individual.

Further, it should be realized that a biological sample can be derived by taking biological samples from a number of individuals and pooling them or pooling an aliquot of each individual's biological sample. The pooled sample can be treated as a sample from a single individual and if an increased or decreased risk of a CV event is established in the pooled sample, then each individual biological sample can be re-tested to determine which individual/s have an increased or decreased risk of a CV event.

As mentioned above, the biological sample can be urine. Urine samples provide certain advantages over blood or serum samples. Collecting blood or plasma samples through venipuncture is more complex than is desirable, can deliver variable volumes, can be worrisome for the patient, and involves some (small) risk of infection. Also, phlebotomy requires skilled personnel. The simplicity of collecting urine samples can lead to more widespread application of the subject methods.

In order to determine the suitability of using urine as a sample, such samples from healthy subjects were assessed for quality and quantity of each protein, and this information was combined with the quality of the Tables 1-3 biomarkers in CV risk prognosis. Because urine is an ultra-filtrate of plasma, the quantity of a specific protein excreted in urine is proportional to the protein concentration in blood. If quality biomarkers of any of Tables 1-3 are available in sufficient quantity in the urine, then they are suitable for use in a method for screening an individual for evaluation of risk of a CV event. Biomarkers predictive of a CV event that have been found in urine include ESAM, MMP7, and GP6, which show a strong signal. These biomarkers are smaller and are freely filtered by the kidney into the urine. In addition, PSA-ACT and Plasminogen have been found to demonstrate variability between individuals in urine. This indicates that the quantification of these biomarkers in urine can also be useful in the method of screening an individual for risk of a CV event. Thus, these five proteins provide for a simple urine-based test to be used in the subject methods of screening individuals for risk of a CV event.

For purposes of this specification, the phrase "data attributed to a biological sample from an individual" is intended to mean that the data in some form derived from, or were generated using, the biological sample of the individual. The data may have been reformatted, revised, or mathematically altered to some degree after having been generated, such as by conversion from units in one measurement system to units in another measurement system; but, the data are understood to have been derived from, or were generated using, the biological sample.

"Target", "target molecule", and "analyte" are used interchangeably herein to refer to any molecule of interest that may be present in a biological sample. A "molecule of interest" includes any minor variation of a particular molecule, such as, in the case of a protein, for example, minor variations in amino acid sequence, disulfide bond formation, glycosylation, lipidation, acetylation, phosphorylation, or any other manipulation or modification, such as conjugation with a labeling component, which does not substantially alter the identity of the molecule. A "target molecule", "target", or "analyte" is a set of copies of one type or species of molecule or multi-molecular structure. "Target molecules", "targets", and "analytes" refer to more than one such set of molecules. Exemplary target molecules include proteins, polypeptides, nucleic acids, carbohydrates, lipids, polysaccharides, glycoproteins, hormones, receptors, antigens, antibodies, affybodies, antibody mimics, viruses, pathogens, toxic substances, substrates, metabolites, transition state analogs, cofactors, inhibitors, drugs, dyes, nutrients, growth factors, cells, tissues, and any fragment or portion of any of the foregoing.

As used herein, "polypeptide," "peptide," and "protein" are used interchangeably herein to refer to polymers of amino acids of any length. The polymer may be linear or branched, it may comprise modified amino acids, and it may be interrupted by non-amino acids. The terms also encompass an amino acid polymer that has been modified naturally or by intervention; for example, disulfide bond formation, glycosylation, lipidation, acetylation, phosphorylation, or any other manipulation or modification, such as conjugation with a labeling component. Also included within the definition are, for example, polypeptides containing one or more analogs of an amino acid (including, for example, unnatural amino acids, etc.), as well as other modifications known in the art. Polypeptides can be single chains or associated chains. Also included within the definition are preproteins and intact mature proteins; peptides or polypeptides derived from a mature protein; fragments of a protein; splice variants; recombinant forms of a protein; protein variants with amino acid modifications, deletions, or substitutions; digests; and post-translational modifications, such as glycosylation, acetylation, phosphorylation, and the like.

As used herein, "marker" and "biomarker" are used interchangeably to refer to a target molecule that indicates or is a sign of a normal or abnormal process in an individual or of a disease or other condition in an individual. More specifically, a "marker" or "biomarker" is an anatomic, physiologic, biochemical, or molecular parameter associated with the presence of a specific physiological state or process, whether normal or abnormal, and, if abnormal, whether chronic or acute. Biomarkers are detectable and measurable by a variety of methods including laboratory assays and medical imaging. When a biomarker is a protein, it is also possible to use the expression of the corresponding gene as a surrogate measure of the amount or presence or absence of the corresponding protein biomarker in a biological sample or methylation state of the gene encoding the biomarker or proteins that control expression of the biomarker.

As used herein, "biomarker value", "value", "biomarker level", and "level" are used interchangeably to refer to a measurement that is made using any analytical method for detecting the biomarker in a biological sample and that indicates the presence, absence, absolute amount or concentration, relative amount or concentration, titer, a level, an expression level, a ratio of measured levels, or the like, of, for, or corresponding to the biomarker in the biological sample. The exact nature of the "value" or "level" depends on the specific design and components of the particular analytical method employed to detect the biomarker.

When a biomarker indicates or is a sign of an abnormal process or a disease or other condition in an individual, that biomarker is generally described as being either over-expressed or under-expressed as compared to an expression level or value of the biomarker that indicates or is a sign of a normal process or an absence of a disease or other condition in an individual. "Up-regulation", "up-regulated", "over-expression", "over-expressed", and any variations thereof are used interchangeably to refer to a value or level of a biomarker in a biological sample that is greater than a value or level (or range of values or levels) of the biomarker that is typically detected in similar biological samples from healthy or normal individuals. The terms may also refer to a value or level of a biomarker in a biological sample that is greater than a value or level (or range of values or levels) of the biomarker that may be detected at a different stage of a particular disease.

"Down-regulation", "down-regulated", "under-expression", "under-expressed", and any variations thereof are used interchangeably to refer to a value or level of a biomarker in a biological sample that is less than a value or level (or range of values or levels) of the biomarker that is typically detected in similar biological samples from healthy or normal individuals. The terms may also refer to a value or level of a biomarker in a biological sample that is less than a value or level (or range of values or levels) of the biomarker that may be detected at a different stage of a particular disease.

Further, a biomarker that is either over-expressed or under-expressed can also be referred to as being "differentially expressed" or as having a "differential level" or "differential value" as compared to a "normal" expression level or value of the biomarker that indicates or is a sign of a normal process or an absence of a disease or other condition in an individual. Thus, "differential expression" of a biomarker can also be referred to as a variation from a "normal" expression level of the biomarker.

The term "differential gene expression" and "differential expression" are used interchangeably to refer to a gene (or its corresponding protein expression product) whose expression is activated to a higher or lower level in a subject suffering from a specific disease or condition, relative to its expression in a normal or control subject. The terms also include genes (or the corresponding protein expression products) whose expression is activated to a higher or lower level at different stages of the same disease or condition. It is also understood that a differentially expressed gene may be either activated or inhibited at the nucleic acid level or protein level, or may be subject to alternative splicing to result in a different polypeptide product. Such differences may be evidenced by a variety of changes including mRNA levels, surface expression, secretion or other partitioning of a polypeptide. Differential gene expression may include a comparison of expression between two or more genes or their gene products; or a comparison of the ratios of the expression between two or more genes or their gene products; or even a comparison of two differently processed products of the same gene, which differ between normal subjects and subjects suffering from a disease; or between various stages of the same disease. Differential expression includes both quantitative, as well as qualitative, differences in the temporal or cellular expression pattern in a gene or its expression products among, for example, normal and diseased cells, or among cells which have undergone different disease events or disease stages.

As used herein, "individual" refers to a test subject or patient. The individual can be a mammal or a non-mammal. In various embodiments, the individual is a mammal. A mammalian individual can be a human or non-human. In various embodiments, the individual is a human. A healthy or normal individual is an individual in which the disease or condition of interest (including, for example, Cardiovascular Events such as myocardial infarction, stroke and congestive heart failure) is not detectable by conventional diagnostic methods.

"Diagnose", "diagnosing", "diagnosis", and variations thereof refer to the detection, determination, or recognition of a health status or condition of an individual on the basis of one or more signs, symptoms, data, or other information pertaining to that individual. The health status of an individual can be diagnosed as healthy / normal (i.e., a diagnosis of the absence of a disease or condition) or diagnosed as ill / abnormal (i.e., a diagnosis of the presence, or an assessment of the characteristics, of a disease or condition). The terms "diagnose", "diagnosing", "diagnosis", etc., encompass, with respect to a particular disease or condition, the initial detection of the disease; the characterization or classification of the disease; the detection of the progression, remission, or recurrence of the disease; and the detection of disease response after the administration of a treatment or therapy to the individual. The prediction of risk of a CV event includes distinguishing individuals who have an increased risk of a CV event from individuals who do not.

"Prognose", "prognosing", "prognosis", and variations thereof refer to the prediction of a future course of a disease or condition in an individual who has the disease or condition (e.g., predicting patient survival), and such terms encompass the evaluation of disease or condition response after the administration of a treatment or therapy to the individual.

"Evaluate", "evaluating", "evaluation", and variations thereof encompass both "diagnose" and "prognose" and also encompass determinations or predictions about the future course of a disease or condition in an individual who does not have the disease as well as determinations or predictions regarding the risk that a disease or condition will recur in an individual who apparently has been cured of the disease or has had the condition resolved. The term "evaluate" also encompasses assessing an individual's response to a therapy, such as, for example, predicting whether an individual is likely to respond favorably to a therapeutic agent or is unlikely to respond to a therapeutic agent (or will experience toxic or other undesirable side effects, for example), selecting a therapeutic agent for administration to an individual, or monitoring or determining an individual's response to a therapy that has been administered to the individual. Thus, "evaluating" risk of a CV vent can include, for example, any of the following: predicting the future risk of a CV event in an individual; predicting the risk of a CV event in an individual who apparently has no CV issues; or determining or predicting an individual's response to a CV treatment or selecting a CV treatment to administer to an individual based upon a determination of the biomarker values derived from the individual's biological sample. Evaluation of risk of a CV event can include embodiments such as the assessment of risk of a CV event on a continuous scale, or classification of risk of a CV event in escalating classifications. Classification of risk includes, for example, classification into two or more classifications such as "No Elevated Risk of a CV Event" and "Elevated Risk of a CV Event." The evaluation of risk of a CV event is for a defined period; such period can be, for example, 5 years.

As used herein, "additional biomedical information" refers to one or more evaluations of an individual, other than using any of the biomarkers described herein, that are associated with CV risk or, more specifically, CV event risk. "Additional biomedical information" includes any of the following: physical descriptors of an individual, including the height and/or weight of an individual; the age of an individual; the gender of an individual; change in weight; the ethnicity of an individual; occupational history; family history of cardiovascular disease (or other circulatory system disorders); the presence of a genetic marker(s) correlating with a higher risk of cardiovascular disease (or other circulatory system disorders) in the individual or a family member alterations in the carotid intima thickness; clinical symptoms such as chest pain, weight gain or loss gene expression values; physical descriptors of an individual, including physical descriptors observed by radiologic imaging; smoking status; alcohol use history; occupational history; dietary habits - salt, saturated fat and cholesterol intake; caffeine consumption; and imaging information such as electrocardiogram, echocardiography, carotid ultrasound for intima-media thickness, flow mediated dilation, pulse wave velocity, ankle -brachial index, stress echocardiography, myocardial perfusion imaging, coronary calcium by CT, high resolution CT angiography, MRI imaging, and other imaging modalities; and the individual's medications. Testing of biomarker levels in combination with an evaluation of any additional biomedical information, including other laboratory tests (e.g., HDL, LDL testing, CRP levels, Nt-proBNP testing , serum albumin testing, creatine testing), may, for example, improve sensitivity, specificity, and/or AUC for prediction of CV events as compared to biomarker testing alone or evaluating any particular item of additional biomedical information alone (e.g., carotid intima thickness imaging alone). Additional biomedical information can be obtained from an individual using routine techniques known in the art, such as from the individual themselves by use of a routine patient questionnaire or health history questionnaire, etc., or from a medical practitioner, etc. Testing of biomarker levels in combination with an evaluation of any additional biomedical information may, for example, improve sensitivity, specificity, and/or thresholds for prediction of CV events (or other cardiovascular-related uses) as compared to biomarker testing alone or evaluating any particular item of additional biomedical information alone (e.g., CT imaging alone).

As used herein, "detecting" or "determining" with respect to a biomarker value includes the use of both the instrument required to observe and record a signal corresponding to a biomarker value and the material/s required to generate that signal. In various embodiments, the biomarker value is detected using any suitable method, including fluorescence, chemiluminescence, surface plasmon resonance, surface acoustic waves, mass spectrometry, infrared spectroscopy, Raman spectroscopy, atomic force microscopy, scanning tunneling microscopy, electrochemical detection methods, nuclear magnetic resonance, quantum dots, and the like.

"Solid support" refers herein to any substrate having a surface to which molecules may be attached, directly or indirectly, through either covalent or non-covalent bonds. A "solid support" can have a variety of physical formats, which can include, for example, a membrane; a chip (e.g., a protein chip); a slide (e.g., a glass slide or coverslip); a column; a hollow, solid, semi-solid, pore- or cavity- containing particle, such as, for example, a bead; a gel; a fiber, including a fiber optic material; a matrix; and a sample receptacle. Exemplary sample receptacles include sample wells, tubes, capillaries, vials, and any other vessel, groove or indentation capable of holding a sample. A sample receptacle can be contained on a multi-sample platform, such as a microtiter plate, slide, microfluidics device, and the like. A support can be composed of a natural or synthetic material, an organic or inorganic material. The composition of the solid support on which capture reagents are attached generally depends on the method of attachment (e.g., covalent attachment). Other exemplary receptacles include microdroplets and microfluidic controlled or bulk oil/aqueous emulsions within which assays and related manipulations can occur. Suitable solid supports include, for example, plastics, resins, polysaccharides, silica or silica-based materials, functionalized glass, modified silicon, carbon, metals, inorganic glasses, membranes, nylon, natural fibers (such as, for example, silk, wool and cotton), polymers, and the like. The material composing the solid support can include reactive groups such as, for example, carboxy, amino, or hydroxyl groups, which are used for attachment of the capture reagents. Polymeric solid supports can include, e.g., polystyrene, polyethylene glycol tetraphthalate, polyvinyl acetate, polyvinyl chloride, polyvinyl pyrrolidone, polyacrylonitrile, polymethyl methacrylate, polytetrafluoroethylene, butyl rubber, styrenebutadiene rubber, natural rubber, polyethylene, polypropylene, (poly)tetrafluoroethylene, (poly)vinylidenefluoride, polycarbonate, and polymethylpentene. Suitable solid support particles that can be used include, e.g., encoded particles, such as Luminex®-type encoded particles, magnetic particles, and glass particles.

### Exemplary Uses of Biomarkers

In various exemplary embodiments, methods are provided for evaluating risk of a CV event in an individual by detecting one or more biomarker values corresponding to one or more biomarkers that are present in the circulation of an individual, such as in serum or plasma, by any number of analytical methods, including any of the analytical methods described herein. These biomarkers are, for example, differentially expressed in individuals with increased risk of a CV event as compared to individuals without increased risk of a CV event. Detection of the differential expression of a biomarker in an individual can be used, for example, to permit the prediction of risk of a CV event within 5 year time frame.

In addition to testing biomarker levels as a stand-alone diagnostic test, biomarker levels can also be done in conjunction with determination of SNPs or other genetic lesions or variability that are indicative of increased risk of susceptibility of disease or condition. (See, e.g., Amos et al., Nature Genetics 40, 616-622 (2009)).

In addition to testing biomarker levels as a stand-alone diagnostic test, biomarker levels can also be used in conjunction with radiologic screening. Biomarker levels can also be used in conjunction with relevant symptoms or genetic testing. Detection of any of the biomarkers described herein may be useful after the risk of CV event has been evaluated to guide appropriate clinical care of the individual, including increasing to more aggressive levels of care in high risk individuals after the CV event risk has been determined. In addition to testing biomarker levels in conjunction with relevant symptoms or risk factors, information regarding the biomarkers can also be evaluated in conjunction with other types of data, particularly data that indicates an individual's risk for cardiovascular events (e.g., patient clinical history, symptoms, family history of cardiovascular disease, history of smoking or alcohol use, risk factors such as the presence of a genetic marker(s), and/or status of other biomarkers, etc.). These various data can be assessed by automated methods, such as a computer program/software, which can be embodied in a computer or other apparatus/device.

In addition to testing biomarker levels in conjunction with radiologic screening in high risk individuals (e.g., assessing biomarker levels in conjunction with blockage detected in a coronary angiogram), information regarding the biomarkers can also be evaluated in conjunction with other types of data, particularly data that indicates an individual's risk for having a CV event (e.g., patient clinical history, symptoms, family history of cardiovascular disease, risk factors such as whether or not the individual is a smoker, heavy alcohol user and/or status of other biomarkers, etc.). These various data can be assessed by automated methods, such as a computer program/software, which can be embodied in a computer or other apparatus/device.

Testing of biomarkers can also be associated with guidelines and cardiovascular risk algorithms currently in use in clincal practice. For example, the Framingham Risk score uses the following risk factors to result in a risk score: vascular tone, LDL-cholesterol and HDL-cholesterol levels, impaired glucose levels, smoking, systolic blood pressure, and diabetes. The frequency of high-risk patients increases with age, and men comprise a greater proportion of high-risk patients than women.

Any of the described biomarkers may also be used in imaging tests. For example, an imaging agent can be coupled to any of the described biomarkers, which can be used to aid in prediction of risk of a Cardiovascular Event, to monitor reponse to therapeutic interventions, to select for target populations in a clinical trial among other uses.

### Detection and Determination of Biomarkers and Biomarker Values

A biomarker value for the biomarkers described herein can be detected using any of a variety of known analytical methods. In one embodiment, a biomarker value is detected using a capture reagent. As used herein, a "capture agent" or "capture reagent" refers to a molecule that is capable of binding specifically to a biomarker. In various embodiments, the capture reagent can be exposed to the biomarker in solution or can be exposed to the biomarker while the capture reagent is immobilized on a solid support. In other embodiments, the capture reagent contains a feature that is reactive with a secondary feature on a solid support. In these embodiments, the capture reagent can be exposed to the biomarker in solution, and then the feature on the capture reagent can be used in conjunction with the secondary feature on the solid support to immobilize the biomarker on the solid support. The capture reagent is selected based on the type of analysis to be conducted. Capture reagents include but are not limited to SOMAmers, antibodies, adnectins, ankyrins, other antibody mimetics and other protein scaffolds, autoantibodies, chimeras, small molecules, an F(ab')2 fragment, a single chain antibody fragment, an Fv fragment, a single chain Fv fragment, a nucleic acid, a lectin, a ligand-binding receptor, affybodies, nanobodies, imprinted polymers, avimers, peptidomimetics, a hormone receptor, a cytokine receptor, and synthetic receptors, and modifications and fragments of these.

In some embodiments, a biomarker value is detected using a biomarker/capture reagent complex.

In other embodiments, the biomarker value is derived from the biomarker/capture reagent complex and is detected indirectly, such as, for example, as a result of a reaction that is subsequent to the biomarker/capture reagent interaction, but is dependent on the formation of the biomarker/capture reagent complex.

In some embodiments, the biomarker value is detected directly from the biomarker in a biological sample.

In one embodiment, the biomarkers are detected using a multiplexed format that allows for the simultaneous detection of two or more biomarkers in a biological sample. In one embodiment of the multiplexed format, capture reagents are immobilized, directly or indirectly, covalently or non-covalently, in discrete locations on a solid support. In another embodiment, a multiplexed format uses discrete solid supports where each solid support has a unique capture reagent associated with that solid support, such as, for example quantum dots. In another embodiment, an individual device is used for the detection of each one of multiple biomarkers to be detected in a biological sample. Individual devices can be configured to permit each biomarker in the biological sample to be processed simultaneously. For example, a microtiter plate can be used such that each well in the plate is used to uniquely analyze one of multiple biomarkers to be detected in a biological sample.

In one or more of the foregoing embodiments, a fluorescent tag can be used to label a component of the biomarker/capture complex to enable the detection of the biomarker value. In various embodiments, the fluorescent label can be conjugated to a capture reagent specific to any of the biomarkers described herein using known techniques, and the fluorescent label can then be used to detect the corresponding biomarker value. Suitable fluorescent labels include rare earth chelates, fluorescein and its derivatives, rhodamine and its derivatives, dansyl, allophycocyanin, PBXL-3, Qdot 605, Lissamine, phycoerythrin, Texas Red, and other such compounds.

In one embodiment, the fluorescent label is a fluorescent dye molecule. In some embodiments, the fluorescent dye molecule includes at least one substituted indolium ring system in which the substituent on the 3-carbon of the indolium ring contains a chemically reactive group or a conjugated substance. In some embodiments, the dye molecule includes an AlexFluor molecule, such as, for example, AlexaFluor 488, AlexaFluor 532, AlexaFluor 647, AlexaFluor 680, or AlexaFluor 700. In other embodiments, the dye molecule includes a first type and a second type of dye molecule, such as, e.g., two different AlexaFluor molecules. In other embodiments, the dye molecule includes a first type and a second type of dye molecule, and the two dye molecules have different emission spectra.

Fluorescence can be measured with a variety of instrumentation compatible with a wide range of assay formats. For example, spectrofluorimeters have been designed to analyze microtiter plates, microscope slides, printed arrays, cuvettes, etc. See Principles of Fluorescence Spectroscopy, by J.R. Lakowicz, Springer Science + Business Media, Inc., 2004. See Bioluminescence & Chemiluminescence: Progress & Current Applications; Philip E. Stanley and Larry J. Kricka editors, World Scientific Publishing Company, January 2002.

In one or more of the foregoing embodiments, a chemiluminescence tag can optionally be used to label a component of the biomarker/capture complex to enable the detection of a biomarker value. Suitable chemiluminescent materials include any of oxalyl chloride, Rodamin 6G, Ru(bipy)32+, TMAE (tetrakis(dimethylamino)ethylene), Pyrogallol (1,2,3-trihydroxibenzene), Lucigenin, peroxyoxalates, Aryl oxalates, Acridinium esters, dioxetanes, and others.

In yet other embodiments, the detection method includes an enzyme/substrate combination that generates a detectable signal that corresponds to the biomarker value. Generally, the enzyme catalyzes a chemical alteration of the chromogenic substrate which can be measured using various techniques, including spectrophotometry, fluorescence, and chemiluminescence. Suitable enzymes include, for example, luciferases, luciferin, malate dehydrogenase, urease, horseradish peroxidase (HRPO), alkaline phosphatase, beta-galactosidase, glucoamylase, lysozyme, glucose oxidase, galactose oxidase, and glucose-6-phosphate dehydrogenase, uricase, xanthine oxidase, lactoperoxidase, microperoxidase, and the like.

In yet other embodiments, the detection method can be a combination of fluorescence, chemiluminescence, radionuclide or enzyme/substrate combinations that generate a measurable signal. Multimodal signaling could have unique and advantageous characteristics in biomarker assay formats.

More specifically, the biomarker values for the biomarkers described herein can be detected using known analytical methods including, singleplex SOMAmer assays, multiplexed SOMAmer assays, singleplex or multiplexed immunoassays, mRNA expression profiling, miRNA expression profiling, mass spectrometric analysis, histological/cytological methods, etc. as detailed below.

### Determination of Biomarker Values using SOMAmer-Based Assays

Assays directed to the detection and quantification of physiologically significant molecules in biological samples and other samples are important tools in scientific research and in the health care field. One class of such assays involves the use of a microarray that includes one or more aptamers immobilized on a solid support. The aptamers are each capable of binding to a target molecule in a highly specific manner and with very high affinity. See, e.g., U.S. Patent No. 5,475,096 entitled "Nucleic Acid Ligands"; see also, e.g., U.S. Patent No. 6,242,246, U.S. Patent No. 6,458,543, and U.S. Patent No. 6,503,715, each of which is entitled "Nucleic Acid Ligand Diagnostic Biochip". Once the microarray is contacted with a sample, the aptamers bind to their respective target molecules present in the sample and thereby enable a determination of a biomarker value corresponding to a biomarker.

As used herein, an "aptamer" refers to a nucleic acid that has a specific binding affinity for a target molecule. It is recognized that affinity interactions are a matter of degree; however, in this context, the "specific binding affinity" of an aptamer for its target means that the aptamer binds to its target generally with a much higher degree of affinity than it binds to other components in a test sample. An "aptamer" is a set of copies of one type or species of nucleic acid molecule that has a particular nucleotide sequence. An aptamer can include any suitable number of nucleotides, including any number of chemically modified nucleotides. "Aptamers" refers to more than one such set of molecules. Different aptamers can have either the same or different numbers of nucleotides. Aptamers can be DNA or RNA or chemically modified nucleic acids and can be single stranded, double stranded, or contain double stranded regions, and can include higher ordered structures. An aptamer can also be a photoaptamer, where a photoreactive or chemically reactive functional group is included in the aptamer to allow it to be covalently linked to its corresponding target. Any of the aptamer methods disclosed herein can include the use of two or more aptamers that specifically bind the same target molecule. As further described below, an aptamer may include a tag. If an aptamer includes a tag, all copies of the aptamer need not have the same tag. Moreover, if different aptamers each include a tag, these different aptamers can have either the same tag or a different tag.

An aptamer can be identified using any known method, including the SELEX process. Once identified, an aptamer can be prepared or synthesized in accordance with any known method, including chemical synthetic methods and enzymatic synthetic methods.

As used herein, a "SOMAmer" or Slow Off-Rate Modified Aptamer refers to an aptamer having improved off-rate characteristics. SOMAmers can be generated using the improved SELEX methods described in U.S. Publication No. 2009/0004667, entitled "Method for Generating Aptamers with Improved Off-Rates."

The terms "SELEX" and "SELEX process" are used interchangeably herein to refer generally to a combination of (1) the selection of aptamers that interact with a target molecule in a desirable manner, for example binding with high affinity to a protein, with (2) the amplification of those selected nucleic acids. The SELEX process can be used to identify aptamers with high affinity to a specific target or biomarker.

SELEX generally includes preparing a candidate mixture of nucleic acids, binding of the candidate mixture to the desired target molecule to form an affinity complex, separating the affinity complexes from the unbound candidate nucleic acids, separating and isolating the nucleic acid from the affinity complex, purifying the nucleic acid, and identifying a specific aptamer sequence. The process may include multiple rounds to further refine the affinity of the selected aptamer. The process can include amplification steps at one or more points in the process. See, e.g., U.S. Patent No. 5,475,096, entitled "Nucleic Acid Ligands". The SELEX process can be used to generate an aptamer that covalently binds its target as well as an aptamer that non-covalently binds its target. See, e.g., U.S. Patent No. 5,705,337 entitled "Systematic Evolution of Nucleic Acid Ligands by Exponential Enrichment: Chemi-SELEX."

The SELEX process can be used to identify high-affinity aptamers containing modified nucleotides that confer improved characteristics on the aptamer, such as, for example, improved *in vivo* stability or improved delivery characteristics. Examples of such modifications include chemical substitutions at the ribose and/or phosphate and/or base positions. SELEX process-identified aptamers containing modified nucleotides are described in U.S. Patent No. 5,660,985, entitled "High Affinity Nucleic Acid Ligands Containing Modified Nucleotides", which describes oligonucleotides containing nucleotide derivatives chemically modified at the 5'- and 2'-positions of pyrimidines. U.S. Patent No. 5,580,737, see supra, describes highly specific aptamers containing one or more nucleotides modified with 2'-amino (2'-NH2), 2'-fluoro (2'-F), and/or 2'-O-methyl (2'-OMe). See also, U.S. Patent Application Publication 20090098549, entitled "SELEX and PHOTOSELEX", which describes nucleic acid libraries having expanded physical and chemical properties and their use in SELEX and photoSELEX.

SELEX can also be used to identify aptamers that have desirable off-rate characteristics. See U.S. Patent Application Publication 20090004667, entitled "Method for Generating Aptamers with Improved Off-Rates", which describes improved SELEX methods for generating aptamers that can bind to target molecules. As mentioned above, these slow off-rate aptamers are known as "SOMAmers." Methods for producing aptamers or SOMAmers and photoaptamers or SOMAmers having slower rates of dissociation from their respective target molecules are described. The methods involve contacting the candidate mixture with the target molecule, allowing the formation of nucleic acid-target complexes to occur, and performing a slow off-rate enrichment process wherein nucleic acid-target complexes with fast dissociation rates will dissociate and not reform, while complexes with slow dissociation rates will remain intact. Additionally, the methods include the use of modified nucleotides in the production of candidate nucleic acid mixtures to generate aptamers or SOMAmers with improved off-rate performance.

A variation of this assay employs aptamers that include photoreactive functional groups that enable the aptamers to covalently bind or "photocrosslink" their target molecules. See, e.g., U.S. Patent No. 6,544,776 entitled "Nucleic Acid Ligand Diagnostic Biochip". These photoreactive aptamers are also referred to as photoaptamers. See, e.g., U.S. Patent No. 5,763,177, U.S. Patent No. 6,001,577, and U.S. Patent No. 6,291,184, each of which is entitled "Systematic Evolution of Nucleic Acid Ligands by Exponential Enrichment: Photoselection of Nucleic Acid Ligands and Solution SELEX"; see also, e.g., U.S. Patent No. 6,458,539, entitled "Photoselection of Nucleic Acid Ligands". After the microarray is contacted with the sample and the photoaptamers have had an opportunity to bind to their target molecules, the photoaptamers are photoactivated, and the solid support is washed to remove any non-specifically bound molecules. Harsh wash conditions may be used, since target molecules that are bound to the photoaptamers are generally not removed, due to the covalent bonds created by the photoactivated functional group(s) on the photoaptamers. In this manner, the assay enables the detection of a biomarker value corresponding to a biomarker in the test sample.

In both of these assay formats, the aptamers or SOMAmers are immobilized on the solid support prior to being contacted with the sample. Under certain circumstances, however, immobilization of the aptamers or SOMAmers prior to contact with the sample may not provide an optimal assay. For example, pre-immobilization of the aptamers or SOMAmers may result in inefficient mixing of the aptamers or SOMAmers with the target molecules on the surface of the solid support, perhaps leading to lengthy reaction times and, therefore, extended incubation periods to permit efficient binding of the aptamers or SOMAmers to their target molecules. Further, when photoaptamers or photoSOMAmers are employed in the assay and depending upon the material utilized as a solid support, the solid support may tend to scatter or absorb the light used to effect the formation of covalent bonds between the photoaptamers or photoSOMAmers and their target molecules. Moreover, depending upon the method employed, detection of target molecules bound to their aptamers or photoSOMAmers can be subject to imprecision, since the surface of the solid support may also be exposed to and affected by any labeling agents that are used. Finally, immobilization of the aptamers or SOMAmers on the solid support generally involves an aptamer or SOMAmer-preparation step (i.e., the immobilization) prior to exposure of the aptamers or SOMAmers to the sample, and this preparation step may affect the activity or functionality of the aptamers or SOMAmers.

SOMAmer assays that permit a SOMAmer to capture its target in solution and then employ separation steps that are designed to remove specific components of the SOMAmer-target mixture prior to detection have also been described (see U.S. Patent Application Publication 20090042206, entitled "Multiplexed Analyses of Test Samples"). The described SOMAmer assay methods enable the detection and quantification of a non-nucleic acid target (e.g., a protein target) in a test sample by detecting and quantifying a nucleic acid (i.e., a SOMAmer). The described methods create a nucleic acid surrogate (i.e, the SOMAmer) for detecting and quantifying a non-nucleic acid target, thus allowing the wide variety of nucleic acid technologies, including amplification, to be applied to a broader range of desired targets, including protein targets.

SOMAmers can be constructed to facilitate the separation of the assay components from a SOMAmer biomarker complex (or photoSOMAmer biomarker covalent complex) and permit isolation of the SOMAmer for detection and/or quantification. In one embodiment, these constructs can include a cleavable or releasable element within the SOMAmer sequence. In other embodiments, additional functionality can be introduced into the SOMAmer, for example, a labeled or detectable component, a spacer component, or a specific binding tag or immobilization element. For example, the SOMAmer can include a tag connected to the SOMAmer via a cleavable moiety, a label, a spacer component separating the label, and the cleavable moiety. In one embodiment, a cleavable element is a photocleavable linker. The photocleavable linker can be attached to a biotin moiety and a spacer section, can include an NHS group for derivatization of amines, and can be used to introduce a biotin group to a SOMAmer, thereby allowing for the release of the SOMAmer later in an assay method.

Homogenous assays, done with all assay components in solution, do not require separation of sample and reagents prior to the detection of signal. These methods are rapid and easy to use. These methods generate signal based on a molecular capture or binding reagent that reacts with its specific target. For prediction of CV events, the molecular capture reagents would be a SOMAmer or an antibody or the like and the specific target would be a CV event biomarker of Table 1, Col. 7.

In one embodiment, a method for signal generation takes advantage of anisotropy signal change due to the interaction of a fluorophore-labeled capture reagent with its specific biomarker target. When the labeled capture reagent reacts with its target, the increased molecular weight causes the rotational motion of the fluorophore attached to the complex to become much slower changing the anisotropy value. By monitoring the anisotropy change, binding events may be used to quantitatively measure the biomarkers in solutions. Other methods include fluorescence polarization assays, molecular beacon methods, time resolved fluorescence quenching, chemiluminescence, fluorescence resonance energy transfer, and the like.

An exemplary solution-based SOMAmer assay that can be used to detect a biomarker value corresponding to a biomarker in a biological sample includes the following: (a) preparing a mixture by contacting the biological sample with a SOMAmer that includes a first tag and has a specific affinity for the biomarker, wherein a SOMAmer affinity complex is formed when the biomarker is present in the sample; (b) exposing the mixture to a first solid support including a first capture element, and allowing the first tag to associate with the first capture element; (c) removing any components of the mixture not associated with the first solid support; (d) attaching a second tag to the biomarker component of the SOMAmer affinity complex; (e) releasing the SOMAmer affinity complex from the first solid support; (f) exposing the released SOMAmer affinity complex to a second solid support that includes a second capture element and allowing the second tag to associate with the second capture element; (g) removing any non-complexed SOMAmer from the mixture by partitioning the non-complexed SOMAmer from the SOMAmer affinity complex; (h) eluting the SOMAmer from the solid support; and (i) detecting the biomarker by detecting the SOMAmer component of the SOMAmer affinity complex.

Any means known in the art can be used to detect a biomarker value by detecting the SOMAmer component of a SOMAmer affinity complex. A number of different detection methods can be used to detect the SOMAmer component of an affinity complex, such as, for example, hybridization assays, mass spectroscopy, or QPCR. In some embodiments, nucleic acid sequencing methods can be used to detect the SOMAmer component of a SOMAmer affinity complex and thereby detect a biomarker value. Briefly, a test sample can be subjected to any kind of nucleic acid sequencing method to identify and quantify the sequence or sequences of one or more SOMAmers present in the test sample. In some embodiments, the sequence includes the entire SOMAmer molecule or any portion of the molecule that may be used to uniquely identify the molecule. In other embodiments, the identifying sequencing is a specific sequence added to the SOMAmer; such sequences are often referred to as "tags," "barcodes," or "zipcodes." In some embodiments, the sequencing method includes enzymatic steps to amplify the SOMAmer sequence or to convert any kind of nucleic acid, including RNA and DNA that contain chemical modifications to any position, to any other kind of nucleic acid appropriate for sequencing.

In some embodiments, the sequencing method includes one or more cloning steps. In other embodiments the sequencing method includes a direct sequencing method without cloning.

In some embodiments, the sequencing method includes a directed approach with specific primers that target one or more SOMAmers in the test sample. In other embodiments, the sequencing method includes a shotgun approach that targets all SOMAmers in the test sample.

In some embodiments, the sequencing method includes enzymatic steps to amplify the molecule targeted for sequencing. In other embodiments, the sequencing method directly sequences single molecules. An exemplary nucleic acid sequencing-based method that can be used to detect a biomarker value corresponding to a biomarker in a biological sample includes the following: (a) converting a mixture of SOMAmers that contain chemically modified nucleotides to unmodified nucleic acids with an enzymatic step; (b) shotgun sequencing the resulting unmodified nucleic acids with a massively parallel sequencing platform such as, for example, the 454 Sequencing System (454 Life Sciences/Roche), the Illumina Sequencing System (Illumina), the ABI SOLiD Sequencing System (Applied Biosystems), the HeliScope Single Molecule Sequencer (Helicos Biosciences), or the Pacific Biosciences Real Time Single-Molecule Sequencing System (Pacific BioSciences) or the Polonator G Sequencing System (Dover Systems); and (c) identifying and quantifying the SOMAmers present in the mixture by specific sequence and sequence count.

### Determination of Biomarker Values using Immunoassays

Immunoassay methods are based on the reaction of an antibody to its corresponding target or analyte and can detect the analyte in a sample depending on the specific assay format. To improve specificity and sensitivity of an assay method based on immuno-reactivity, monoclonal antibodies are often used because of their specific epitope recognition. Polyclonal antibodies have also been successfully used in various immunoassays because of their increased affinity for the target as compared to monoclonal antibodies. Immunoassays have been designed for use with a wide range of biological sample matrices. Immunoassay formats have been designed to provide qualitative, semi-quantitative, and quantitative results.

Quantitative results are generated through the use of a standard curve created with known concentrations of the specific analyte to be detected. The response or signal from an unknown sample is plotted onto the standard curve, and a quantity or value corresponding to the target in the unknown sample is established.

Numerous immunoassay formats have been designed. ELISA or EIA can be quantitative for the detection of an analyte. This method relies on attachment of a label to either the analyte or the antibody and the label component includes, either directly or indirectly, an enzyme. ELISA tests may be formatted for direct, indirect, competitive, or sandwich detection of the analyte. Other methods rely on labels such as, for example, radioisotopes (1125) or fluorescence. Additional techniques include, for example, agglutination, nephelometry, turbidimetry, Western blot, immunoprecipitation, immunocytochemistry, immunohistochemistry, flow cytometry, Luminex assay, and others (see ImmunoAssay: A Practical Guide, edited by Brian Law, published by Taylor & Francis, Ltd., 2005 edition).

Exemplary assay formats include enzyme-linked immunosorbent assay (ELISA), radioimmunoassay, fluorescent, chemiluminescence, and fluorescence resonance energy transfer (FRET) or time resolved-FRET (TR-FRET) immunoassays. Examples of procedures for detecting biomarkers include biomarker immunoprecipitation followed by quantitative methods that allow size and peptide level discrimination, such as gel electrophoresis, capillary electrophoresis, planar electrochromatography, and the like.

Methods of detecting and/or quantifying a detectable label or signal generating material depend on the nature of the label. The products of reactions catalyzed by appropriate enzymes (where the detectable label is an enzyme; see above) can be, without limitation, fluorescent, luminescent, or radioactive or they may absorb visible or ultraviolet light. Examples of detectors suitable for detecting such detectable labels include, without limitation, x-ray film, radioactivity counters, scintillation counters, spectrophotometers, colorimeters, fluorometers, luminometers, and densitometers.

Any of the methods for detection can be performed in any format that allows for any suitable preparation, processing, and analysis of the reactions. This can be, for example, in multi-well assay plates (e.g., 96 wells or 384 wells) or using any suitable array or microarray. Stock solutions for various agents can be made manually or robotically, and all subsequent pipetting, diluting, mixing, distribution, washing, incubating, sample readout, data collection and analysis can be done robotically using commercially available analysis software, robotics, and detection instrumentation capable of detecting a detectable label.

### Determination of Biomarker Values using Gene Expression Profiling

Measuring mRNA in a biological sample may be used as a surrogate for detection of the level of the corresponding protein in the biological sample. Thus, any of the biomarkers or biomarker panels described herein can also be detected by detecting the appropriate RNA.

mRNA expression levels are measured by reverse transcription quantitative polymerase chain reaction (RT-PCR followed with qPCR). RT-PCR is used to create a cDNA from the mRNA. The cDNA may be used in a qPCR assay to produce fluorescence as the DNA amplification process progresses. By comparison to a standard curve, qPCR can produce an absolute measurement such as number of copies of mRNA per cell. Northern blots, microarrays, Invader assays, and RT-PCR combined with capillary electrophoresis have all been used to measure expression levels of mRNA in a sample. See Gene Expression Profiling: Methods and Protocols, Richard A. Shimkets, editor, Humana Press, 2004.

miRNA molecules are small RNAs that are non-coding but may regulate gene expression. Any of the methods suited to the measurement of mRNA expression levels can also be used for the corresponding miRNA. Recently many laboratories have investigated the use of miRNAs as biomarkers for disease. Many diseases involve wide-spread transcriptional regulation, and it is not surprising that miRNAs might find a role as biomarkers. The connection between miRNA concentrations and disease is often even less clear than the connections between protein levels and disease, yet the value of miRNA biomarkers might be substantial. Of course, as with any RNA expressed differentially during disease, the problems facing the development of an *in vitro* diagnostic product will include the requirement that the miRNAs survive in the diseased cell and are easily extracted for analysis, or that the miRNAs are released into blood or other matrices where they must survive long enough to be measured. Protein biomarkers have similar requirements, although many potential protein biomarkers are secreted intentionally at the site of pathology and function, during disease, in a paracrine fashion. Many potential protein biomarkers are designed to function outside the cells within which those proteins are synthesized.

### Detection of Biomarkers Using In Vivo Molecular Imaging Technologies

Any of the described biomarkers (see Table 1, Col. 7) may also be used in molecular imaging tests. For example, an imaging agent can be coupled to any of the described biomarkers, which can be used to aid in prediction of risk of Cardiovascular Events within 5 years, to monitor response to therapeutic interventions, to select a population for clinical trials among other uses.

*In vivo* imaging technologies provide non-invasive methods for determining the state of a particular disease or condition in the body of an individual. For example, entire portions of the body, or even the entire body, may be viewed as a three dimensional image, thereby providing valuable information concerning morphology and structures in the body. Such technologies may be combined with the detection of the biomarkers described herein to provide information concerning the cardiovascular status of an individual.

The use of *in vivo* molecular imaging technologies is expanding due to various advances in technology. These advances include the development of new contrast agents or labels, such as radiolabels and/or fluorescent labels, which can provide strong signals within the body; and the development of powerful new imaging technology, which can detect and analyze these signals from outside the body, with sufficient sensitivity and accuracy to provide useful information. The contrast agent can be visualized in an appropriate imaging system, thereby providing an image of the portion or portions of the body in which the contrast agent is located. The contrast agent may be bound to or associated with a capture reagent, such as a SOMAmer or an antibody, for example, and/or with a peptide or protein, or an oligonucleotide (for example, for the detection of gene expression), or a complex containing any of these with one or more macromolecules and/or other particulate forms.

The contrast agent may also feature a radioactive atom that is useful in imaging. Suitable radioactive atoms include technetium-99m or iodine-123 for scintigraphic studies. Other readily detectable moieties include, for example, spin labels for magnetic resonance imaging (MRI) such as, for example, iodine-123 again, iodine-131, indium-Ill, fluorine-19, carbon-13, nitrogen-15, oxygen-17, gadolinium, manganese or iron. Such labels are well known in the art and could easily be selected by one of ordinary skill in the art.

Standard imaging techniques include but are not limited to magnetic resonance imaging, computed tomography scanning (coronary calcium score), positron emission tomography (PET), single photon emission computed tomography (SPECT), computed tomography angiography, and the like. For diagnostic *in vivo* imaging, the type of detection instrument available is a major factor in selecting a given contrast agent, such as a given radionuclide and the particular biomarker that it is used to target (protein, mRNA, and the like). The radionuclide chosen typically has a type of decay that is detectable by a given type of instrument. Also, when selecting a radionuclide for *in vivo* diagnosis, its half-life should be long enough to enable detection at the time of maximum uptake by the target tissue but short enough that deleterious radiation of the host is minimized.

Exemplary imaging techniques include but are not limited to PET and SPECT, which are imaging techniques in which a radionuclide is synthetically or locally administered to an individual. The subsequent uptake of the radiotracer is measured over time and used to obtain information about the targeted tissue and the biomarker. Because of the high-energy (gamma-ray) emissions of the specific isotopes employed and the sensitivity and sophistication of the instruments used to detect them, the two-dimensional distribution of radioactivity may be inferred from outside of the body.

Commonly used positron-emitting nuclides in PET include, for example, carbon-11, nitrogen-13, oxygen-15, and fluorine-18. Isotopes that decay by electron capture and/or gamma-emission are used in SPECT and include, for example iodine-123 and technetium-99m. An exemplary method for labeling amino acids with technetium-99m is the reduction of pertechnetate ion in the presence of a chelating precursor to form the labile technetium-99m-precursor complex, which, in turn, reacts with the metal binding group of a bifunctionally modified chemotactic peptide to form a technetium-99m-chemotactic peptide conjugate.

Antibodies are frequently used for such *in vivo* imaging diagnostic methods. The preparation and use of antibodies for *in vivo* diagnosis is well known in the art. Labeled antibodies which specifically bind any of the biomarkers in Table 1, Col. 7 can be injected into an individual suspected of having an increased risk of a CV event, detectable according to the particular biomarker used, for the purpose of diagnosing or evaluating the disease status or condition of the individual. The label used will be selected in accordance with the imaging modality to be used, as previously described. Localization of the label permits determination of the tissue damage or other indications related to the risk of a CV event. The amount of label within an organ or tissue also allows determination of the involvement of the CV event biomarkers due to the risk of a CV event in that organ or tissue.

Similarly, SOMAmers may be used for such *in vivo* imaging diagnostic methods. For example, a SOMAmer that was used to identify a particular biomarker described in Table 1, Col. 7 (and therefore binds specifically to that particular biomarker) may be appropriately labeled and injected into an individual suspected of having had a CV event, detectable according to the particular biomarker, for the purpose of diagnosing or evaluating the levels of tissue damage, atherosclerotic plaques, components of inflammatory response and other factors associated with the risk of a CV event in the individual. The label used will be selected in accordance with the imaging modality to be used, as previously described. Localization of the label permits determination of the site of the processes leading to increased risk. The amount of label within an organ or tissue also allows determination of the infiltration of the pathological process in that organ or tissue. SOMAmer-directed imaging agents could have unique and advantageous characteristics relating to tissue penetration, tissue distribution, kinetics, elimination, potency, and selectivity as compared to other imaging agents.

Such techniques may also optionally be performed with labeled oligonucleotides, for example, for detection of gene expression through imaging with antisense oligonucleotides. These methods are used for in situ hybridization, for example, with fluorescent molecules or radionuclides as the label. Other methods for detection of gene expression include, for example, detection of the activity of a reporter gene.

Another general type of imaging technology is optical imaging, in which fluorescent signals within the subject are detected by an optical device that is external to the subject. These signals may be due to actual fluorescence and/or to bioluminescence. Improvements in the sensitivity of optical detection devices have increased the usefulness of optical imaging for *in vivo* diagnostic assays.

The use of *in vivo* molecular biomarker imaging is increasing, including for clinical trials, for example, to more rapidly measure clinical efficacy in trials for new disease or condition therapies and/or to avoid prolonged treatment with a placebo for those diseases, such as multiple sclerosis, in which such prolonged treatment may be considered to be ethically questionable.

For a review of other techniques, see N. Blow, Nature Methods, 6, 465-469, 2009.

### Determination of Biomarker Values using Mass Spectrometry Methods

A variety of configurations of mass spectrometers can be used to detect biomarker values. Several types of mass spectrometers are available or can be produced with various configurations. In general, a mass spectrometer has the following major components: a sample inlet, an ion source, a mass analyzer, a detector, a vacuum system, and instrument-control system, and a data system. Difference in the sample inlet, ion source, and mass analyzer generally define the type of instrument and its capabilities. For example, an inlet can be a capillary-column liquid chromatography source or can be a direct probe or stage such as used in matrix-assisted laser desorption. Common ion sources are, for example, electrospray, including nanospray and microspray or matrix-assisted laser desorption. Common mass analyzers include a quadrupole mass filter, ion trap mass analyzer and time-of-flight mass analyzer. Additional mass spectrometry methods are well known in the art (see Burlingame et al. Anal. Chem. 70:647 R-716R (1998); Kinter and Sherman, New York (2000)).

Protein biomarkers and biomarker values can be detected and measured by any of the following: electrospray ionization mass spectrometry (ESI-MS), ESI-MS/MS, ESI-MS/(MS)n, matrix-assisted laser desorption ionization time-of-flight mass spectrometry (MALDI-TOF-MS), surface-enhanced laser desorption/ionization time-of-flight mass spectrometry (SELDI-TOF-MS), desorption/ionization on silicon (DIOS), secondary ion mass spectrometry (SIMS), quadrupole time-of-flight (Q-TOF), tandem time-of-flight (TOF/TOF) technology, called ultraflex III TOF/TOF, atmospheric pressure chemical ionization mass spectrometry (APCI-MS), APCI-MS/MS, APCI-(MS)N, atmospheric pressure photoionization mass spectrometry (APPI-MS), APPI-MS/MS, and APPI-(MS)N, quadrupole mass spectrometry, Fourier transform mass spectrometry (FTMS), quantitative mass spectrometry, and ion trap mass spectrometry.

Sample preparation strategies are used to label and enrich samples before mass spectroscopic characterization of protein biomarkers and determination biomarker values. Labeling methods include but are not limited to isobaric tag for relative and absolute quantitation (iTRAQ) and stable isotope labeling with amino acids in cell culture (SILAC). Capture reagents used to selectively enrich samples for candidate biomarker proteins prior to mass spectroscopic analysis include but are not limited to SOMAmers, antibodies, nucleic acid probes, chimeras, small molecules, an F(ab')2 fragment, a single chain antibody fragment, an Fv fragment, a single chain Fv fragment, a nucleic acid, a lectin, a ligand-binding receptor, affybodies, nanobodies, ankyrins, domain antibodies, alternative antibody scaffolds (e.g. diabodies etc) imprinted polymers, avimers, peptidomimetics, peptoids, peptide nucleic acids, threose nucleic acid, a hormone receptor, a cytokine receptor, and synthetic receptors, and modifications and fragments of these.

### Determination of Biomarker Values using a Proximity Ligation Assay

A proximity ligation assay can be used to determine biomarker values. Briefly, a test sample is contacted with a pair of affinity probes that may be a pair of antibodies or a pair of SOMAmers, with each member of the pair extended with an oligonucleotide. The targets for the pair of affinity probes may be two distinct determinates on one protein or one determinate on each of two different proteins, which may exist as homo- or hetero-multimeric complexes. When probes bind to the target determinates, the free ends of the oligonucleotide extensions are brought into sufficiently close proximity to hybridize together. The hybridization of the oligonucleotide extensions is facilitated by a common connector oligonucleotide which serves to bridge together the oligonucleotide extensions when they are positioned in sufficient proximity. Once the oligonucleotide extensions of the probes are hybridized, the ends of the extensions are joined together by enzymatic DNA ligation.

Each oligonucleotide extension comprises a primer site for PCR amplification. Once the oligonucleotide extensions are ligated together, the oligonucleotides form a continuous DNA sequence which, through PCR amplification, reveals information regarding the identity and amount of the target protein, as well as, information regarding protein-protein interactions where the target determinates are on two different proteins. Proximity ligation can provide a highly sensitive and specific assay for real-time protein concentration and interaction information through use of real-time PCR. Probes that do not bind the determinates of interest do not have the corresponding oligonucleotide extensions brought into proximity and no ligation or PCR amplification can proceed, resulting in no signal being produced.

The foregoing assays enable the detection of biomarker values that are useful in methods for prediction of risk of CV events, where the methods comprise detecting, in a biological sample from an individual, at least N biomarker values that each correspond to a biomarker selected from the group consisting of the biomarkers provided in Table 1, Col. 7, wherein a classification, as described in detail below, using the biomarker values indicates whether the individual has elevated risk of a CV event occuring within a 5 year time period. While certain of the described CV event biomarkers are useful alone for predicting risk of a CV event, methods are also described herein for the grouping of multiple subsets of the CV event biomarkers that are each useful as a panel of three or more biomarkers. Thus, various embodiments of the instant application provide combinations comprising N biomarkers, wherein N is at least three biomarkers. In other embodiments, N is selected to be any number from 2-155 biomarkers. It will be appreciated that N can be selected to be any number from any of the above described ranges, as well as similar, but higher order, ranges. In accordance with any of the methods described herein, biomarker values can be detected and classified individually or they can be detected and classified collectively, as for example in a multiplex assay format.

A biomarker "signature" for a given diagnostic or predictive test contains a set of markers, each marker having different levels in the populations of interest. Different levels, in this context, may refer to different means of the marker levels for the individuals in two or more groups, or different variances in the two or more groups, or a combination of both. For the simplest form of a diagnostic test, these markers can be used to assign an unknown sample from an individual into one of two groups, either at increased risk of a CV event or not. The assignment of a sample into one of two or more groups is known as classification, and the procedure used to accomplish this assignment is known as a classifier or a classification method. Classification methods may also be referred to as scoring methods. There are many classification methods that can be used to construct a diagnostic classifier from a set of biomarker values. In general, classification methods are most easily performed using supervised learning techniques where a data set is collected using samples obtained from individuals within two (or more, for multiple classification states) distinct groups one wishes to distinguish. Since the class (group or population) to which each sample belongs is known in advance for each sample, the classification method can be trained to give the desired classification response. It is also possible to use unsupervised learning techniques to produce a diagnostic classifier.

Common approaches for developing diagnostic classifiers include decision trees; bagging, boosting, forests and random forests; rule inference based learning; Parzen Windows; linear models; logistic; neural network methods; unsupervised clustering; K-means; hierarchical ascending/ descending; semi-supervised learning; prototype methods; nearest neighbor; kernel density estimation; support vector machines; hidden Markov models; Boltzmann Learning; and classifiers may be combined either simply or in ways which minimize particular objective functions. For a review, see, e.g., Pattern Classification, R.O. Duda, et al., editors, John Wiley & Sons, 2nd edition, 2001; see also, The Elements of Statistical Learning - Data Mining, Inference, and Prediction, T. Hastie, et al., editors, Springer Science+Business Media, LLC, 2nd edition, 2009.

To produce a classifier using supervised learning techniques, a set of samples called training data are obtained. In the context of diagnostic tests, training data includes samples from the distinct groups (classes) to which unknown samples will later be assigned. For example, samples collected from individuals in a control population and individuals in a particular disease, condition or event population can constitute training data to develop a classifier that can classify unknown samples (or, more particularly, the individuals from whom the samples were obtained) as either having the disease, condition or elevated risk of an event or being free from the disease, condition or elevated risk of an event. The development of the classifier from the training data is known as training the classifier. Specific details on classifier training depend on the nature of the supervised learning technique (see, e.g., Pattern Classification, R.O. Duda, et al., editors, John Wiley & Sons, 2nd edition, 2001; see also, The Elements of Statistical Learning - Data Mining, Inference, and Prediction, T. Hastie, et al., editors, Springer Science+Business Media, LLC, 2nd edition, 2009).

Since typically there are many more potential biomarker values than samples in a training set, care must be used to avoid over-fitting. Over-fitting occurs when a statistical model describes random error or noise instead of the underlying relationship. Over-fitting can be avoided in a variety of ways, including, for example, by limiting the number of markers used in developing the classifier, by assuming that the marker responses are independent of one another, by limiting the complexity of the underlying statistical model employed, and by ensuring that the underlying statistical model conforms to the data.

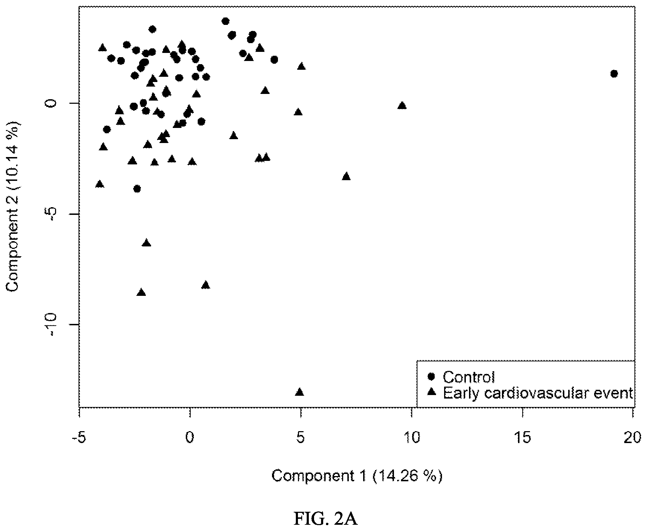

In order to identify a set of biomarkers associated with occurence of events, the combined set of control and early event samples were analyzed using Principal Component Analysis (PCA). PCA displays the samples with respect to the axes defined by the strongest variations between all the samples, without regard to the case or control outcome, thus mitigating the risk of overfitting the distinction between case and control. Since the occurrence of serious thrombotic events has a strong component of chance involved, requiring unstable plaque to rupture in vital vessels to be reported, one would not expect to see a clear separation between the control and event sample sets. While the observed separation between case and control is not large, it occurs on the second principal component, corresponding to around 10% of the total variation in this set of samples, which indicates that the underlying biological variation is relatively simple to quantify (Figure 2A).

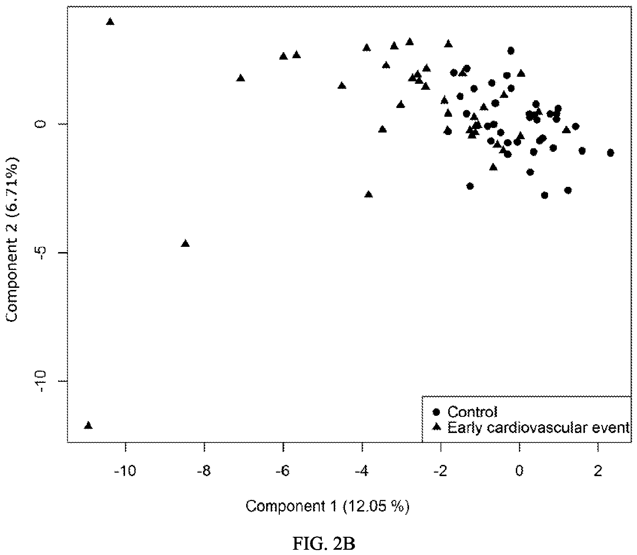

In the next set of analyses, biomarkers can be analyzed for those components of difference between samples which were specific to the separation between the control samples and early event samples. One method that may be employed is the use of DSGA (Bair,E. and Tibshirani,R. (2004) Semi-supervised methods to predict patient survival from gene expression data. PLOS Biol., 2, 511-522) to remove (deflate) the first three principal component directions of variation between the samples in the control set. Although the dimensionality reduction is performed on the control set to discover, both the samples in the control and the samples from the early event samples are run through the PCA. Separation of cases from early events can be observed along the horizontal axis (Figure 2B).

### Cross validated selection of proteins relevant to cardiovascular risk

In order to avoid over-fitting of protein predictive power to idiosyncratic features of a particular selection of samples, a cross-validation and dimensional reduction approach was taken. Cross-validation involves the multiple selection of sets of samples to determine the association of risk by protein combined with the use of the unselected samples to monitor the ability of the method to apply to samples which were not used in producing the model of risk (The Elements of Statistical Learning - Data Mining, Inference, and Prediction, T. Hastie, et al., editors, Springer Science+Business Media, LLC, 2nd edition, 2009). We applied the supervised PCA method of Tibshirani et al (Bair,E. and Tibshirani,R. (2004) Semi-supervised methods to predict patient survival from gene expression data. PLOS Biol., 2, 511-522.) which is applicable to high dimensional datasets in the modeling of event risk. The supervised PCA (SPCA) method inolves the univariate selection of a set of proteins statistically associated with the observed event hazard in the data and the determination of the correlated component which combines information from all of these proteins. This determination of the correlated component is a dimensionality reduction step which not only combines information across proteins, but also mitigates the likelihood of overfitting by reducing the number of independent variables from the full protein menu of over 1000 proteins down to a few principal components (in this work, we only examined the first principal component). The SPCA method of Tibshirani et al. applies cross validation to the selection of proteins in order to determine the number of proteins which are truly predictive in the withheld cross-validation test sets. These proteins are used to create the correlated component of protein variation associated with event risk. Using SPCA we found a list of 155 proteins which were statistically associated with event risk using this cross-validated dimensional reduction technique. In this application of SPCA, we also allowed test protein signals corresponding to random SOMAmer sequences as well as signals corresponding to nonhuman proteins, which were not present in the samples. None of these 10-20 known nonbiological signals were selected in the 155 proteins by SPCA (Table 1). This step using the cross-validated SPCA approach is important to screen against false positive protein marker associations. The approach in Tibshirani et al is especially protected against false discovery by the use of prevalidation method of cross-validation and the dimensional reduction inherent in PCA. The list of 155 proteins from SPCA was used to check subsequent analyses using different techniques to detect the false discovery of protein markers, not contained in the list of 155 proteins from SPCA.

### Univariate analysis and multivariate analysis of the relationship of individual proteins to time to event

The Cox proportional hazard model (Cox, David R (1972). "Regression Models and Life-Tables". Journal of the Royal Statistical Society. Series B (Methodological) 34 (2): 187-220.)) is widely used in medical statistics. Cox regression avoids fitting a specific function of time to the cumulative survival, and instead employs a model of relative risk referred to a baseline hazard function (which may vary with time). The baseline hazard function describes the common shape of the survival time distribution for all individuals, while the relative risk gives the level of the hazard for a set of covariate values (such as a single individual or group), as a multiple of the baseline hazard. The relative risk is constant with time in the Cox model.

We fitted 1092 simple univariate Cox models to all signals. 46 proteins (Table 2) have P-values (Wald test, (Wald, Abraham. (1943). A Method of Estimating Plane Vulnerability Based on Damage of Survivors. Statistical Research Group, Columbia University) better than 10⁻¹⁴. All of these 46 proteins were included in the list of 155 proteins selected using SPCA (above, Table 1). The large number of highly significant proteins is at first surprising, however the involvement of the kidney in the cardiovascular disease implies changes in GFR (Glomerular filtration rate). Decreases in GFR will increase all proteins with non-zero renal clearance, the concentration of a protein in the blood is reduced through loss of the protein into the urine via the kidney (clearance), reduced renal filtration as measured by GFR is thus associated with increased concentration of those proteins in the blood which are partially filtered by the kidney.

A useful model would be more parsimonious than the full list of 46 proteins. Also as seen in the PCA many proteins are likely to be highly correlated, an effective model will take this into account. We filtered the list of 46 highly significant proteins down to 10 proteins in two steps. First, we restricted the list to the 20 proteins which gave a coefficient with a magnitude greater than 0.37 (equivalent to a 30% hazard change for a doubling in protein signal), this step was taken on a single multivariate Cox model using all 46 proteins. (The natural log of the protein measurements were taken before fitting the Cox models, therefore exponential of the Cox coefficient corresponds to the hazard ratio of an e-fold (2.71) change in the protein measurement.)

The next step filtered the 20 proteins down to ten by requiring that the p-value should be more significant than 0.01. This step suppresses covariant proteins and allows independent proteins to contribute. A final adjustment was made to the biomarker selection in that C9, a member of the membrane attack complex in the final common pathway of the complement system, was judged to be too unspecific in its signaling, a matter which cannot be decided from this study alone, since the study is created to cleanly demonstrate Cardio vascular risk. C9 was removed and all the remaining proteins were evaluated in its place. The substitute proteins were ranked on the improvement in the Wald test score, and KLK3.SerpinA3 was close to as effective as C9.

The Kaplan Meier survival curves are shown in Figures 3A-3E for this ten marker model of cardiovascular risk.

Table 1 identifies 155 biomarkers that are useful for evaluation the risk of a future CV event in an individual. This is a surprisingly larger number than expected when compared to what is typically found during biomarker discovery efforts and may be attributable to the scale of the described study, which encompassed over 1000 proteins measured in hundreds of individual samples, in some cases at concentrations in the low femtomolar range. Presumably, the large number of discovered biomarkers reflects the diverse biochemical pathways implicated in the biology leading up to a cardiovascular event and the body's response to the CV event; each pathway and process involves many proteins. The results show that no single protein of a small group of proteins is uniquely informative about such complex processes; rather, that multiple proteins are involved in relevant processes, such as GFR, atherosclerosis, inflammation and hormonal CV regulation, for example.

The results from Example 2 suggest certain possible conclusions: First, the identification of a large number of biomarkers enables their aggregation into a vast number of classifiers that offer similarly high performance. Second, classifiers can be constructed such that particular biomarkers may be substituted for other biomarkers in a manner that reflects the redundancies that undoubtedly pervade the complexities of the underlying disease, condition or event processes. That is to say, the information about the disease, condition or event contributed by any individual biomarker identified in Table 1 overlaps with the information contributed by other biomarkers, such that it may be that no particular biomarker or small group of biomarkers in Table 1 must be included in any classifier.

### Kits

Any combination of the biomarkers of Table 1, Col. 7 can be detected using a suitable kit, such as for use in performing the methods disclosed herein. Furthermore, any kit can contain one or more detectable labels as described herein, such as a fluorescent moiety, etc.

In one embodiment, a kit includes (a) one or more capture reagents (such as, for example, at least one SOMAmer or antibody) for detecting one or more biomarkers in a biological sample, wherein the biomarkers include any of the biomarkers set forth in Table 1, Col. 7, and optionally (b) one or more software or computer program products for classifying the individual from whom the biological sample was obtained as either having or not having increased risk of a CV event or for determining the likelihood that the individual has increased risk of a CV event, as further described herein. Alternatively, rather than one or more computer program products, one or more instructions for manually performing the above steps by a human can be provided.

The combination of a solid support with a corresponding capture reagent having a signal generating material is referred to herein as a "detection device" or "kit". The kit can also include instructions for using the devices and reagents, handling the sample, and analyzing the data. Further the kit may be used with a computer system or software to analyze and report the result of the analysis of the biological sample.

The kits can also contain one or more reagents (e.g., solubilization buffers, detergents, washes, or buffers) for processing a biological sample. Any of the kits described herein can also include, e.g., buffers, blocking agents, mass spectrometry matrix materials, antibody capture agents, positive control samples, negative control samples, software and information such as protocols, guidance and reference data.

In one aspect, the disclosure provides kits for the analysis of CV event risk status. The kits include PCR primers for one or more SOMAmers specific to biomarkers selected from Table 1, Col. 7. The kit may further include instructions for use and correlation of the biomarkers with prediction of risk of a CV event. The kit may also include a DNA array containing the complement of one or more of the Somamers specific for the biomarkers selected from Table 1, Col. 7, reagents, and/or enzymes for amplifying or isolating sample DNA. The kits may include reagents for real-time PCR, for example, TaqMan probes and/or primers, and enzymes.

For example, a kit can comprise (a) reagents comprising at least capture reagent for quantifying one or more biomarkers in a test sample, wherein said biomarkers comprise the biomarkers set forth in Table 1, Col. 7, or any other biomarkers or biomarkers panels described herein, and optionally (b) one or more algorithms or computer programs for performing the steps of comparing the amount of each biomarker quantified in the test sample to one or more predetermined cutoffs and assigning a score for each biomarker quantified based on said comparison, combining the assigned scores for each biomarker quantified to obtain a total score, comparing the total score with a predetermined score, and using said comparison to determine whether an individual has an increased risk of a CV event. Alternatively, rather than one or more algorithms or computer programs, one or more instructions for manually performing the above steps by a human can be provided.

### Computer Methods and Software

Once a biomarker or biomarker panel is selected, a method for diagnosing an individual can comprise the following: 1) collect or otherwise obtain a biological sample; 2) perform an analytical method to detect and measure the biomarker or biomarkers in the panel in the biological sample; 3) perform any data normalization or standardization required for the method used to collect biomarker values; 4) calculate the marker score; 5) combine the marker scores to obtain a total diagnostic or predictive score; and 6) report the individual's diagnostic or predictive score. In this approach, the diagnostic or predictive score may be a single number determined from the sum of all the marker calculations that is compared to a preset threshold value that is an indication of the presence or absence of disease. Or the diagnostic or predictive score may be a series of bars that each represent a biomarker value and the pattern of the responses may be compared to a pre-set pattern for determination of the presence or absence of disease, condition or the increased risk (or not) of an event.

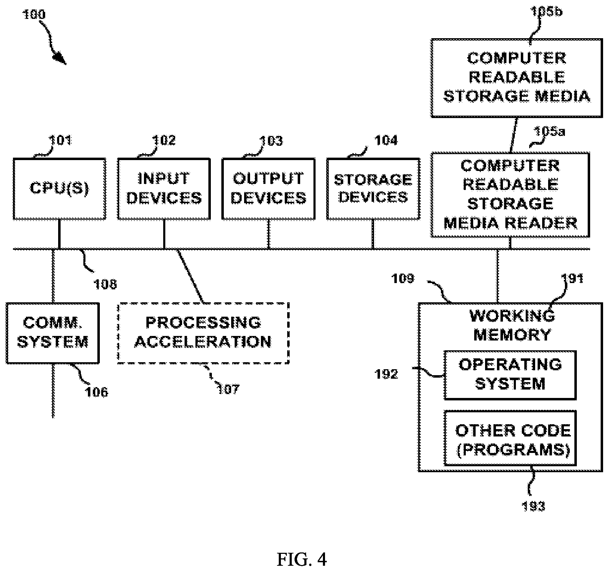

At least some embodiments of the methods described herein can be implemented with the use of a computer. An example of a computer system 100 is shown in Figure 4. With reference to Figure 4, system 100 is shown comprised of hardware elements that are electrically coupled via bus 108, including a processor 101, input device 102, output device 103, storage device 104, computer-readable storage media reader 105a, communications system 106, processing acceleration (e.g., DSP or special-purpose processors) 107 and memory 109. Computer-readable storage media reader 105a is further coupled to computer-readable storage media 105b, the combination comprehensively representing remote, local, fixed and/or removable storage devices plus storage media, memory, etc. for temporarily and/or more permanently containing computer-readable information, which can include storage device 104, memory 109 and/or any other such accessible system 100 resource. System 100 also comprises software elements (shown as being currently located within working memory 191) including an operating system 192 and other code 193, such as programs, data and the like.

With respect to Figure 4, system 100 has extensive flexibility and configurability. Thus, for example, a single architecture might be utilized to implement one or more servers that can be further configured in accordance with currently desirable protocols, protocol variations, extensions, etc. However, it will be apparent to those skilled in the art that embodiments may well be utilized in accordance with more specific application requirements. For example, one or more system elements might be implemented as sub-elements within a system 100 component (e.g., within communications system 106). Customized hardware might also be utilized and/or particular elements might be implemented in hardware, software or both. Further, while connection to other computing devices such as network input/output devices (not shown) may be employed, it is to be understood that wired, wireless, modem, and/or other connection or connections to other computing devices might also be utilized.

In one aspect, the system can comprise a database containing features of biomarkers characteristic of prediction of risk of a CV event. The biomarker data (or biomarker information) can be utilized as an input to the computer for use as part of a computer implemented method. The biomarker data can include the data as described herein.

In one aspect, the system further comprises one or more devices for providing input data to the one or more processors.

The system further comprises a memory for storing a data set of ranked data elements.

In another aspect, the device for providing input data comprises a detector for detecting the characteristic of the data element, e.g., such as a mass spectrometer or gene chip reader.

The system additionally may comprise a database management system. User requests or queries can be formatted in an appropriate language understood by the database management system that processes the query to extract the relevant information from the database of training sets.

The system may be connectable to a network to which a network server and one or more clients are connected. The network may be a local area network (LAN) or a wide area network (WAN), as is known in the art. Preferably, the server includes the hardware necessary for running computer program products (e.g., software) to access database data for processing user requests.

The system may include an operating system (e.g., UNIX or Linux) for executing instructions from a database management system. In one aspect, the operating system can operate on a global communications network, such as the internet, and utilize a global communications network server to connect to such a network.

The system may include one or more devices that comprise a graphical display interface comprising interface elements such as buttons, pull down menus, scroll bars, fields for entering text, and the like as are routinely found in graphical user interfaces known in the art. Requests entered on a user interface can be transmitted to an application program in the system for formatting to search for relevant information in one or more of the system databases. Requests or queries entered by a user may be constructed in any suitable database language.

The graphical user interface may be generated by a graphical user interface code as part of the operating system and can be used to input data and/or to display inputted data. The result of processed data can be displayed in the interface, printed on a printer in communication with the system, saved in a memory device, and/or transmitted over the network or can be provided in the form of the computer readable medium.

The system can be in communication with an input device for providing data regarding data elements to the system (e.g., expression values). In one aspect, the input device can include a gene expression profiling system including, e.g., a mass spectrometer, gene chip or array reader, and the like.

The methods and apparatus for analyzing CV event risk prediction biomarker information according to various embodiments may be implemented in any suitable manner, for example, using a computer program operating on a computer system. A conventional computer system comprising a processor and a random access memory, such as a remotely-accessible application server, network server, personal computer or workstation may be used. Additional computer system components may include memory devices or information storage systems, such as a mass storage system and a user interface, for example a conventional monitor, keyboard and tracking device. The computer system may be a stand-alone system or part of a network of computers including a server and one or more databases.

The CV event risk prediction biomarker analysis system can provide functions and operations to complete data analysis, such as data gathering, processing, analysis, reporting and/or diagnosis. For example, in one embodiment, the computer system can execute the computer program that may receive, store, search, analyze, and report information relating to the CV event risk prediction biomarkers. The computer program may comprise multiple modules performing various functions or operations, such as a processing module for processing raw data and generating supplemental data and an analysis module for analyzing raw data and supplemental data to generate a CV event risk prediction status and/or diagnosis or risk calculation. Calculation of risk status for a CV event may optionally comprise generating or collecting any other information, including additional biomedical information, regarding the condition of the individual relative to the disease, condition or event, identifying whether further tests may be desirable, or otherwise evaluating the health status of the individual.

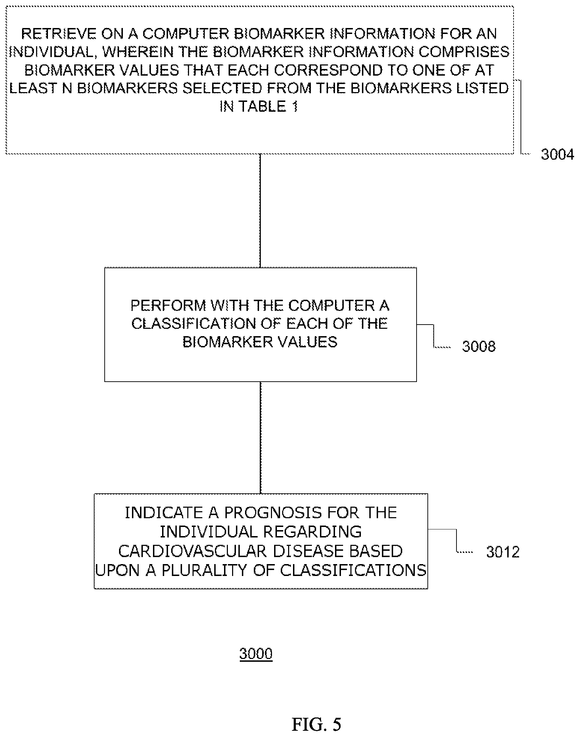

Referring now to Figure 5, an example of a method of utilizing a computer in accordance with principles of a disclosed embodiment can be seen. In Figure 5, a flowchart 3000 is shown. In block 3004, biomarker information can be retrieved for an individual. The biomarker information can be retrieved from a computer database, for example, after testing of the individual's biological sample is performed. The biomarker information can comprise biomarker values that each correspond to one of at least N biomarkers selected from a group consisting of the biomarkers provided in Table 1 , Col. 7, wherein N = 2-155. In block 3008, a computer can be utilized to classify each of the biomarker values. And, in block 3012, a determination can be made as to the likelihood that an individual has increased risk of a CV event based upon a plurality of classifications. The indication can be output to a display or other indicating device so that it is viewable by a person. Thus, for example, it can be displayed on a display screen of a computer or other output device.

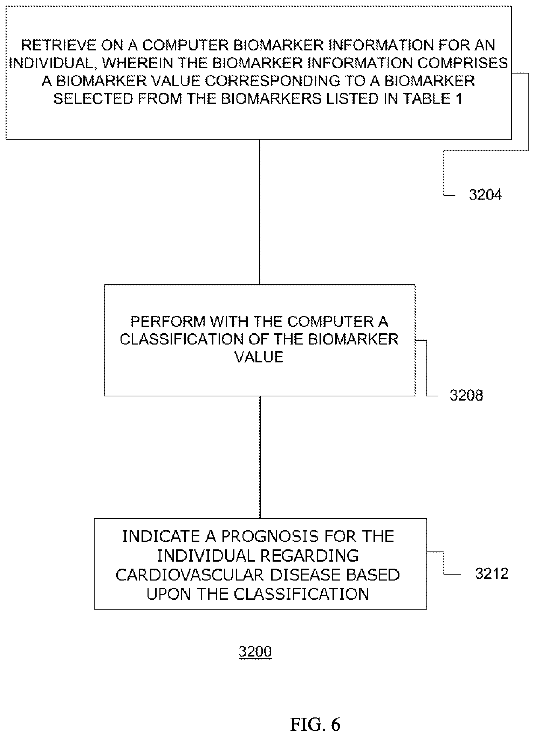

Referring now to Figure 6, an alternative method of utilizing a computer in accordance with another embodiment can be illustrated via flowchart 3200. In block 3204, a computer can be utilized to retrieve biomarker information for an individual. The biomarker information comprises a biomarker value corresponding to a biomarker selected from the group of biomarkers provided in Table 1 , Col.7. In block 3208, a classification of the biomarker value can be performed with the computer. And, in block 3212, an indication can be made as to the likelihood that the individual has increaseed risk of a CV event based upon the classification. The indication can be output to a display or other indicating device so that it is viewable by a person. Thus, for example, it can be displayed on a display screen of a computer or other output device.

Some embodiments described herein can be implemented so as to include a computer program product. A computer program product may include a computer readable medium having computer readable program code embodied in the medium for causing an application program to execute on a computer with a database.

As used herein, a "computer program product" refers to an organized set of instructions in the form of natural or programming language statements that are contained on a physical media of any nature (e.g., written, electronic, magnetic, optical or otherwise) and that may be used with a computer or other automated data processing system. Such programming language statements, when executed by a computer or data processing system, cause the computer or data processing system to act in accordance with the particular content of the statements. Computer program products include without limitation: programs in source and object code and/or test or data libraries embedded in a computer readable medium. Furthermore, the computer program product that enables a computer system or data processing equipment device to act in pre-selected ways may be provided in a number of forms, including, but not limited to, original source code, assembly code, object code, machine language, encrypted or compressed versions of the foregoing and any and all equivalents.

In one aspect, a computer program product is provided for evaluation of the risk of a CV event. The computer program product includes a computer readable medium embodying program code executable by a processor of a computing device or system, the program code comprising: code that retrieves data attributed to a biological sample from an individual, wherein the data comprises biomarker values that each correspond to one of at least N biomarkers in the biological sample selected from the group of biomarkers provided in Table 1, Col. 7, wherein N = 2-155; and code that executes a classification method that indicates a CV event risk status of the individual as a function of the biomarker values.

In still another aspect, a computer program product is provided for indicating a likelihood of risk of a CV event. The computer program product includes a computer readable medium embodying program code executable by a processor of a computing device or system, the program code comprising: code that retrieves data attributed to a biological sample from an individual, wherein the data comprises a biomarker value corresponding to a biomarker in the biological sample selected from the group of biomarkers provided in Table 1 , Col. 7; and code that executes a classification method that indicates a CV event risk status of the individual as a function of the biomarker value.

While various embodiments have been described as methods or apparatuses, it should be understood that embodiments can be implemented through code coupled with a computer, e.g., code resident on a computer or accessible by the computer. For example, software and databases could be utilized to implement many of the methods discussed above. Thus, in addition to embodiments accomplished by hardware, it is also noted that these embodiments can be accomplished through the use of an article of manufacture comprised of a computer usable medium having a computer readable program code embodied therein, which causes the enablement of the functions disclosed in this description. Therefore, it is desired that embodiments also be considered protected by this patent in their program code means as well. Furthermore, the embodiments may be embodied as code stored in a computer-readable memory of virtually any kind including, without limitation, RAM, ROM, magnetic media, optical media, or magneto-optical media. Even more generally, the embodiments could be implemented in software, or in hardware, or any combination thereof including, but not limited to, software running on a general purpose processor, microcode, PLAs, or ASICs.

It is also envisioned that embodiments could be accomplished as computer signals embodied in a carrier wave, as well as signals (e.g., electrical and optical) propagated through a transmission medium. Thus, the various types of information discussed above could be formatted in a structure, such as a data structure, and transmitted as an electrical signal through a transmission medium or stored on a computer readable medium.

It is also noted that many of the structures, materials, and acts recited herein can be recited as means for performing a function or step for performing a function. Therefore, it should be understood that such language is entitled to cover all such structures, materials, or acts disclosed within this specification and their equivalents.

The biomarker identification process, the utilization of the biomarkers disclosed herein, and the various methods for determining biomarker values are described in detail above with respect to evaluation of risk of a CV event. However, the application of the process, the use of identified biomarkers, and the methods for determining biomarker values are fully applicable to other specific types of cardiovascular conditions, to any other disease or medical condition, or to the identification of individuals who may or may not be benefited by an ancillary medical treatment.

### EXAMPLES

The following examples are provided for illustrative purposes only and are not intended to limit the scope of the application as defined by the appended claims. All examples described herein were carried out using standard techniques, which are well known and routine to those of skill in the art. Routine molecular biology techniques described in the following examples can be carried out as described in standard laboratory manuals, such as Sambrook et al., Molecular Cloning: A Laboratory Manual, 3rd. ed., Cold Spring Harbor Laboratory Press, Cold Spring Harbor, N.Y., (2001).

### Example 1. Multiplexed Aptamer Analysis of Samples

This example describes the multiplex aptamer assay used to analyze the samples and controls for the identification of the biomarkers set forth in Table 1. The general protocol for analysis of a sample is illustrated in Figures 1A and 1B. Commonly in medical studies of survival data, the Cox proportional hazard model is employed to produce a risk score from multiple covariates of pathological state. In this work, we have employed this simple and well known approach to devise a model from the population data in the Heart and Soul study, for example, suitable for application to individual samples according to this flexible and widely used Cox Proportional Hazard Formalism. The biomarker values are combined as shown in Figure 1B by taking the log ratio of the biomarker measurements relative to the normal levels. The Cox model uses the exponential of the weighted sum of these log ratios to produce an estimate of the hazard ratio to the normal population.

In this method, pipette tips were changed for each solution addition.

Also, unless otherwise indicated, most solution transfers and wash additions used the 96-well head of a Beckman Biomek FxP. Method steps manually pipetted used a twelve channel P200 Pipetteman (Rainin Instruments, LLC, Oakland, CA), unless otherwise indicated. A custom buffer referred to as SB 17 was prepared in-house, comprising 40mM HEPES, 100mM NaCl, 5mM KCl, 5mM MgCl2, 1mM EDTA at pH7.5. All steps were performed at room temperature unless otherwise indicated.

### 1. Preparation of Aptamer Stock Solution

Custom stock aptamer solutions for 5%, 0.316% and 0.01% serum were prepared at 2x concentration in 1x SB17, 0.05% Tween-20.
These solutions are stored at -20°C until use. The day of the assay, each aptamer mix was thawed at 37°C for 10 minutes, placed in a boiling water bath for 10 minutes and allowed to cool to 25°C for 20 minutes with vigorous mixing in between each heating step. After heat-cool, 55µl of each 2x aptamer mix (was manually pipetted into a 96-well Hybaid plate and the plate foil sealed. The final result was three, 96-well, foil-sealed Hybaid plates with 5%, 0.316% or 0.01% aptamer mixes. The individual aptamer concentration was 2x final or 1 nM.

### 2. Assay Sample Preparation

Frozen aliquots of 100% serum, stored at -80°C, were placed in 25°C water bath for 10 minutes. Thawed samples were placed on ice, gently vortexed (set on 4) for 8 seconds and then replaced on ice.

A 10% sample solution (2x final) was prepared by transferring 8 µL of sample using a 50 µL 8-channel spanning pipettor into 96-well Hybaid plates, each well containing 72 µL of the appropriate sample diluent at 4°C (1x SB17, 0.06% Tween-20, 11.1µM Z-block_2, 0.44 mM MgCl₂, 2.2mM AEBSF, 1.1mM EGTA, 55.6uM EDTA for serum). This plate was stored on ice until the next sample dilution steps were initiated on the Biomek FxP robot.

To commence sample and aptamer equilibration, the 10% sample plate was briefly centrifuged and placed on the Biomek FxP where it was mixed by pipetting up and down with the 96-well pipettor. A -0.632% sample plate (2x final) was then prepared by transferring 6µL of the 10% sample plate into 89 µL of 1xSB17, 0.05% Tween-20 with 2mM AEBSF. Next, dilution of 6 µL of the resultant 0.632% sample into 184 µL of 1xSB17, 0.05% Tween-20 made a 0.02% sample plate (2x final). Dilutions were done on the Beckman Biomek FxP. After each transfer, the solutions were mixed by pipetting up and down. The 3 sample dilution plates were then transferred to their respective aptamer solutions by adding 55 µL of the sample to 55 µL of the appropriate 2x aptamer mix. The sample and aptamer solutions were mixed on the robot by pipetting up and down.

### 3. Sample Equilibration binding

The sample/aptamer plates were sealed with silicon cap mats and placed into a 37°C incubator for 3.5 hours before proceeding to the Catch 1 step.

### 4. Preparation of Catch 2 bead plate

An 11 mL aliquot of MyOne (Invitrogen Corp., Carlsbad, CA) Streptavidin C1 beads was washed 2 times with equal volumes of 20 mM NaOH (5 minute incubation for each wash), 3 times with equal volumes of 1x SB17, 0.05% Tween-20 and resuspended in 11 mL 1x SB17, 0.05% Tween-20. Using a 12-channel pipettor, 50 µL of this solution was manually pipetted into each well of a 96-well Hybaid plate. The plate was then covered with foil and stored at 4°C for use in the assay.

### 5. Preparation of Catch 1 bead plates

Three 0.45 µm Millipore HV plates (Durapore membrane, Cat# MAHVN4550) were equilibrated with 100 µL of 1x SB17, 0.05% Tween-20 for at least 10 minutes. The equilibration buffer was then filtered through the plate and 133.3 µL of a 7.5% Streptavidin-agarose bead slurry (in 1x SB17, 0.05% Tween-20) was added into each well. To keep the streptavidin-agarose beads suspended while transferring them into the filter plate, the bead solution was manually mixed with a 200 µL, 12-channel pipettor, at least 6 times between pipetting events. After the beads were distributed across the 3 filter plates, a vacuum was applied to remove the bead supernatant. Finally, the beads were washed in the filter plates with 200 µL 1x SB17, 0.05% Tween-20 and then resuspended in 200 µL 1x SB17, 0.05% Tween-20. The bottoms of the filter plates were blotted and the plates stored for use in the assay.

### 6. Loading the Cytomat

The cytomat was loaded with all tips, plates, all reagents in troughs (except NHS-biotin reagent which was prepared fresh right before addition to the plates), 3 prepared catch 1 filter plates and 1 prepared MyOne plate.

### 7. Catch 1

After a 3.5 hour equilibration time, the sample/aptamer plates were removed from the incubator, centrifuged for about 1 minute, cap mat covers removed, and placed on the deck of the Beckman Biomek FxP. The Beckman Biomek FxP program was initiated. All subsequent steps in Catch 1 were performed by the Beckman Biomek FxP robot unless otherwise noted. Within the program, the vacuum was applied to the Catch 1 filter plates to remove the bead supernatant. One hundred microlitres of each of the 5%, 0.316% and 0.01% equilibration binding reactions were added to their respective Catch 1 filtration plates, and each plate was mixed using an on-deck orbital shaker at 800 rpm for 10 minutes.

Unbound solution was removed via vacuum filtration. The Catch 1 beads were washed with 190 µL of 100 µM biotin in 1x SB17, 0.05% Tween-20 followed by 5x 190 µL of 1x SB17, 0.05% Tween-20 by dispensing the solution and immediately drawing a vacuum to filter the solution through the plate.

### 8. Tagging

A 100mM NHS-PEO4-biotin aliquot in anhydrous DMSO (stored at -20°C) was thawed at 37°C for 6 minutes and then was diluted 1:100 with tagging buffer (SB17 at pH=7.25, 0.05% Tween-20), immediately before manual addition to an on-deck trough whereby the robot dispensed 100 µL of the NHS-PEO4-biotin into each well of each Catch 1 filter plate. This solution was allowed to incubate with Catch 1 beads shaking at 800 rpm for 5 minutes on the orbital shakers.

### 9. Kinetic Challenge and Photo-cleavage

The tagging reaction was removed by vacuum filtration and the reaction quenched by the addition of 150 µL of 20 mM glycine in 1x SB17, 0.05% Tween-20 to the Catch 1 plates The glycine solution was removed via vacuum filtration and another 1500µL of 20 mM glycine (in 1x SB17, 0.05% Tween-20) was added to each plate and incubated for 1 minute on orbital shakers at 800 rpm before removal by vacuum filtration.

The wells of the Catch 1 plates were subsequently washed by adding 190 µL 1x SB17, 0.05% Tween-20, followed immediately by vacuum filtration and then by adding 190 µL 1x SB17, 0.05% Tween-20 with shaking for 1 minute at 800 rpm before vacuum filtration. These two wash steps were repeated two more times with the exception that the last wash was not removed by vacuum filtration. After the last wash the plates were placed on top of a 1 mL deep-well plate and removed from the deck for centrifugation at 1000 rpm for 1 minute to remove as much extraneous volume from the agarose beads before elution as possible.

The plates were placed back onto the Beckman Biomek FxP and 85 µL of 10 mM DxSO4 in 1x SB 17, 0.05% Tween-20 was added to each well of the filter plates.

The filter plates were removed from the deck, placed onto a Variomag Thermoshaker (Thermo Fisher Scientific, Inc., Waltham, MA) under the BlackRay (Ted Pella, Inc., Redding, CA) light sources, and irradiated for 5 minutes while shaking at 800 rpmAfter the 5-minute incubation the plates were rotated 180 degrees and irradiated with shaking for 5 minutes more.

The photocleaved solutions were sequentially eluted from each Catch 1 plate into a common deep well plate by first placing the 5% Catch 1 filter plate on top of a 1 mL deep-well plate and centrifuging at 1000 rpm for 1 minute. The 0.316% and 0.01% Catch 1 plates were then sequentially centrifuged into the same deep well plate.

### 10. Catch 2 bead capture

The 1 mL deep well block containing the combined eluates of Catch 1 was placed on the deck of the Beckman Biomek FxP for Catch 2.

The robot transferred all of the photo-cleaved eluate from the 1 mL deep-well plate onto the Hybaid plate containing the previously prepared Catch 2 MyOne magnetic beads (after removal of the MyOne buffer via magnetic separation).

The solution was incubated while shaking at 1350 rpm for 5 minutes at 25°C on a Variomag Thermoshaker (Thermo Fisher Scientific, Inc., Waltham, MA).

The robot transferred the plate to the on deck magnetic separator station. The plate was incubated on the magnet for 90 seconds before removal and discarding of the supernatant.

### 11. 37°C 30% glycerol washes

The Catch 2 plate was moved to the on-deck thermal shaker and 75 µL of 1x SB17, 0.05% Tween-20 was transferred to each well. The plate was mixed for 1 minute at 1350 rpm and 37°C to resuspend and warm the beads. To each well of the catch 2 plate, 75 µL of 60% glycerol at 37°C was transferred and the plate continued to mix for another minute at 1350 rpm and 3°C. The robot transferred the plate to the 37°C magnetic separator where it was incubated on the magnet for 2 minutes and then the robot removed and discarded the supernatant. These washes were repeated two more times.

After removal of the third 30% glycerol wash from the Catch 2 beads, 150 µL of 1x SB17, 0.05% Tween-20 was added to each well and incubated at 37°C, shaking at 1350 rpm for 1 minute, before removal by magnetic separation on the 37°C magnet.

The Catch 2 beads were washed a final time using 150 µL 1x SB19, 0.05% Tween-20 with incubation for 1 minute while shaking at 1350 rpm, prior to magnetic separation.

### 12. Catch 2 Bead Elution and Neutralization

The aptamers were eluted from Catch 2 beads by adding 105 µL of 100 mM CAPSO with 1 M NaCl, 0.05% Tween-20 to each well. The beads were incubated with this solution with shaking at 1300 rpm for 5 minutes.

The Catch 2 plate was then placed onto the magnetic separator for 90 seconds prior to transferring 63 µL of the eluate to a new 96-well plate containing 7µL of 500 mM HCl, 500 mM HEPES, 0.05% Tween-20 in each well. After transfer, the solution was mixed robotically by pipetting 60 µL up and down five times.

### 13. Hybridization

The Beckman Biomek FxP transferred 20 µL of the neutralized Catch 2 eluate to a fresh Hybaid plate, and 6 µL of 10x Agilent Block, containing a 10x spike of hybridization controls, was added to each well. Next, 30 µL of 2x Agilent Hybridization buffer was manually pipetted to the each well of the plate containing the neutralized samples and blocking buffer and the solution was mixed by manually pipetting 25 µL up and down 15 times slowly to avoid extensive bubble formation. The plate was spun at 1000 rpm for 1 minute.

Custom Agilent microarray slides (Agilent Technologies, Inc., Santa Clara, CA) were designed to contain probes complementary to the aptamer random region plus some primer region. For the majority of the aptamers, the optimal length of the complementary sequence was empirically determined and ranged between 40-50 nucleotides. For later aptamers a 46-mer complementary region was chosen by default. The probes were linked to the slide surface with a poly-T linker for a total probe length of 60 nucleotides.

A gasket slide was placed into an Agilent hybridization chamber and 40 µL of each of the samples containing hybridization and blocking solution was manually pipetted into each gasket. An 8-channel variable spanning pipettor was used in a manner intended to minimize bubble formation. The custom Agilent slides, with the barcode facing up, were then slowly lowered onto the gasket slides (see Agilent manual for detailed description).

The top of the hybridization chambers were placed onto the slide/backing sandwich and clamping brackets slid over the whole assembly. These assemblies were tightly clamped by turning the screws securely.

Each slide/backing slide sandwich was visually inspected to assure the solution bubble could move freely within the sample. If the bubble did not move freely the hybridization chamber assembly was gently tapped to disengage bubbles lodged near the gasket.

The assembled hybridization chambers were incubated in an Agilent hybridization oven for 19 hours at 60°C rotating at 20 rpm.

### 14. Post Hybridization Washing

Approximately 400 mL Agilent Wash Buffer 1 was placed into each of two separate glass staining dishes. One of the staining dishes was placed on a magnetic stir plate and a slide rack and stir bar were placed into the buffer.

A staining dish for Agilent Wash 2 was prepared by placing a stir bar into an empty glass staining dish.

A fourth glass staining dish was set aside for the final acetonitrile wash.

Each of six hybridization chambers was disassembled. One-by-one, the slide/backing sandwich was removed from its hybridization chamber and submerged into the staining dish containing Wash 1. The slide/backing sandwich was pried apart using a pair of tweezers, while still submerging the microarray slide. The slide was quickly transferred into the slide rack in the Wash 1 staining dish on the magnetic stir plate.

The slide rack was gently raised and lowered 5 times. The magnetic stirrer was turned on at a low setting and the slides incubated for 5 minutes.

When one minute was remaining for Wash 1, Wash Buffer 2 pre-warmed to 37°C in an incubator was added to the second prepared staining dish. The slide rack was quickly transferred to Wash Buffer 2 and any excess buffer on the bottom of the rack was removed by scraping it on the top of the stain dish. The slide rack was gently raised and lowered 5 times. The magnetic stirrer was turned on at a low setting and the slides incubated for 5 minutes. The slide rack was slowly pulled out of Wash 2, taking approximately 15 seconds to remove the slides from the solution.

With one minute remaining in Wash 2 acetonitrile (ACN) was added to the fourth staining dish. The slide rack was transferred to the acetonitrile stain dish. The slide rack was gently raised and lowered 5 times. The magnetic stirrer was turned on at a low setting and the slides incubated for 5 minutes.

The slide rack was slowly pulled out of the ACN stain dish and placed on an absorbent towel. The bottom edges of the slides were quickly dried and the slide was placed into a clean slide box.

### 15. Microarray Imaging

The microarray slides were placed into Agilent scanner slide holders and loaded into the Agilent Microarray scanner according to the manufacturer's instructions.

The slides were imaged in the Cy3-channel at 5 µm resolution at the 100% PMT setting and the XRD option enabled at 0.05. The resulting tiff images were processed using Agilent feature extraction software version 10.5.

### Example 2. Biomarker Identification

The identification of potential CV event biomarkers was performed for prediction of risk of a CV event in a population of individuals in the San Francisco Bay Area. Participants had to meet one of the following enrollment criteria for this study: prior myocardial infarction, angiographic evidence of greater than 50% stenosis in 1 or more coronary vessels, exercise-induced ischemia by treadmill or nuclear testing, or prior coronary revascularization. Exclusion criteria included recent myocardial infarction, inability to walk around 1 block, and plans to relocate. Fasting blood samples were collected, and serum and plasma aliquots were stored at -70°C. The multiplexed SOMAmer affinity assay as described in Example 1 was used to measure and report the RFU value for 1034 analytes in each of these 987 samples.

In order to identify a set of biomarkers associated with occurence of events, the combined set of control and early event samples were analyzed using PCA. PCA displays the samples with respect to the axes defined by the strongest variations between all the samples, without regard to the case or control outcome, thus mitigating the risk of overfitting the distinction between case and control. ("Control" refers to individuals who met at least one of the entry criteria, but who did not have a CV event during the course of the study; "case" refers to individuals who met at least one of the entry criteria, but who did have a CV event during the course of the study.) While the observed separation between case and control is not large, it occurs on the second principal component, corresponding to around 10% of the total variation in this set of samples, which indicates that the underlying biological variation is relatively simple to quantify (Figure 2A).

In the next set of analyses, biomarkers can be analyzed for those components of difference between samples which were specific to the separation between the control samples and early event samples. Although the dimensionality reduction is performed on the control set alone, to determine the multivariate multidimensional space of variation spanned by the differences between control samples, both the samples in the control set and the set of early event samples are deflated for space of variation determined between control samples, the residual variation is enriched in those components separating case from control. This is known as the DSGA method. Separation of cases from early events can be observed along the horizontal axis (Figure 2B) (Nicolau M, Tibshirani R, Børresen-Dale AL, Jeffrey SS. Disease-specific genomic analysis: Identifying the signature of pathologic biology. Bioinformatics. 2007;23:957-965.)

In order to avoid over-fitting of protein predictive power to idiosyncratic features of a particular selection of samples, a cross-validation and dimensional reduction approach was taken. This determination of the correlated component is a dimensionality reduction step which not only combines information across proteins, but also mitigates the likelihood of overfitting by reducing the number of independent variables from the full protein menu of over 1000 proteins down to a few principal components (in this work, we only examined the first principal component). These proteins are used to create the correlated component of protein variation associated with event risk. Using SPCA (Supervised Principal Component Analysis) we found a list of 155 proteins which were statistically associated with event risk using this cross-validated dimensional reduction technique. In this application of SPCA, we also allowed test protein signals corresponding to random SOMAmer sequences as well as signals corresponding to non-human proteins, which were not present in the samples. None of these 10-20 known non-biological signals were selected in the 155 proteins by SPCA (Table 1). This step using the cross-validated SPCA approach is important to screen against false positive protein marker associations. The approach in Tibshirani et al. (Bair,E. and Tibshirani,R. (2004) Semi-supervised methods to predict patient survival from gene expression data. PLOS Biol., 2, 511-522) is especially protected against false discovery by the use of prevalidation method of cross-validation and the dimensional reduction inherent in PCA. The list of 155 proteins from SPCA was used to check subsequent analyses using different techniques to detect the false discovery of protein markers, not contained in the list of 155 proteins from SPCA.

### Example 3. Univariate Analysis of the Relationship of Individual Proteins to Time to CV Event

The Cox proportional hazard model (Cox, David R (1972). "Regression Models and Life-Tables". Journal of the Royal Statistical Society. Series B (Methodological) 34 (2): 187-220)) is widely used in medical statistics. Cox regression avoids fitting a specific function of time to the cumulative survival, and instead employs a model of relative risk referred to a baseline hazard function (which may vary with time). The baseline hazard function describes the common shape of the survival time distribution for all individuals, while the relative risk gives the level of the hazard for a set of covariate values (such as a single individual or group), as a multiple of the baseline hazard. The relative risk is constant with time in the Cox model.

The method involved fitting 1092 simple univariate Cox models to all signals. Forty-six proteins have P-values (Wald, Abraham. (1943). A Method of Estimating Plane Vulnerability Based on Damage of Survivors. Statistical Research Group, Columbia University)) better than 10⁻¹⁴. The large number of highly significant proteins is at first surprising, however the involvement of the kidney in the cardiovascular disease implies changes in the glomerular filtration rate (GFR). Decreases in GFR will increase all proteins with non-zero renal clearance.

A useful model (in terms of technical complexity and cost in the laboratory) would be more parsimonious than the full list of 46 proteins shown in Table 2. Also as seen in the PCA many proteins are likely to be highly correlated; an effective model will take this into account. The list of 46 highly significant proteins was filtered down to 10 proteins as shown in Table 3 in two steps. Firstly, the list was restricted to the 20 proteins which gave a coefficient with a magnitude greater than 0.37 (equivalent to a 30% hazard change for a doubling in protein signal). This step was taken on a single multivariate Cox model using all 46 proteins. (The natural log of the protein measurements were taken before fitting the Cox models. Therefore the exponential of the Cox coefficient corresponds to the hazard ratio of an e-fold (2.71) change in the protein measurement.)

The next step filtered the 20 proteins down to nine by requiring that the P-value should be more significant than 0.01. This step suppresses covariant proteins and allows independent proteins to contribute. A final adjustment was made to the biomarker selection in that C9, a member of the membrane attack complex in the final common pathway of the complement system, was judged to be too unspecific in its signaling, a matter which cannot be decided from this study alone, since the study is created to cleanly demonstrate CV event risk. C9 was removed and all the remaining proteins were evaluated in its place. The substitute proteins were ranked on the improvement in the Wald test score, and KLK3.SerpinA3 was close to as effective as C9.

The Kaplan Meier survival curves are shown in Figures 3A-4F for this ten marker model of cardiovascular risk (Table 3).

### Example 4. Univariate Analysis of the Relationship of Individual Proteins to the specific type of CV Event

Cardiovascular events largely fall into two classes: thrombotic and CHF. Distinguishing between thrombotic and CHF risk has medical utility in guiding therapy, choosing between anti-thrombotic and diuretic medications, for example. Although much of the biology is shared between the thrombotic and CHF classes of events, thrombotic events specifically involve the biology of blood coagulation (as implied by the name thrombotic). Using the ten proteins of Table 3 identified in the Cox proportional hazard model (Example 3), it was possible to look for the signals linked to coagulation and to signals linked to tissue remodeling. To determine any differential signal between the CHF and thrombotic events the relevant Kaplan Meier curves were plotted separately for CHF and thrombotic events.

Platelets, or thrombocytes are a key player in the biology of coagulation. GPVI is a platelet membrane glycoprotein, and for this protein, analysis was conducted of the association with event free survival for both CHF and thrombotic events in Figure 8. Figure 8A shows a strong association of thrombotic event free survival with the level of GPVI, plotted as quartiles of the population distribution. Figure 8B shows that the quartiles of GPVI are not associated with event free survival for CHF events.

In contrast to GPVI, MATN2 (matrilin 2) is an extracellular matrix associated protein. Figure 9A shows that the quartiles of MATN2 are not associated with risk for thrombotic events, while Figure 9B shows a strong association between MATN2 and CHF events. The event free survival for those individuals with a MATN2 in the 4th quartile of the population is markedly worse than the first three quartiles.

Taken together, the results of this example demonstrate that our ten protein markers can discriminate between thrombotic events and CHF events in terms of the risk over a few years after blood sampling.

### Example 5. Usefulness of Angiopoietin 2 and CHRDL1 in Individuals Medicated with Statins.

Many individuals are medicated with statins, both with known cardiovascular disease, and many without specific cardiovascular conditions, such as those with high LDL cholesterol. These powerful drugs alter the ability of many biomarkers to discriminate those at risk from those not at risk. It is valuable for biomarkers to function in this population.

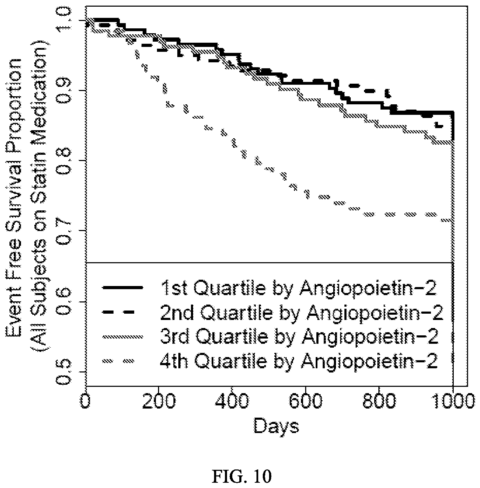

Figure 10 shows that in those subjects on statins, angiopoietin 2 is still strongly useful for prediction of a CV event in high risk individuals. Figure10 shows Kaplan Meier plots of all 538 subjects taking statin medication and illustrates that those individuals in the 4th quartile of the population distribution for angiopoietin-2, suffer cardiovascular events at an increased rate compared to those not in the 4th Quartile for angiopoietin-2. Thus despite the effects of treatment with statins, angiopoietin-2 is a useful biomarker of the risk of cardiovascular events.

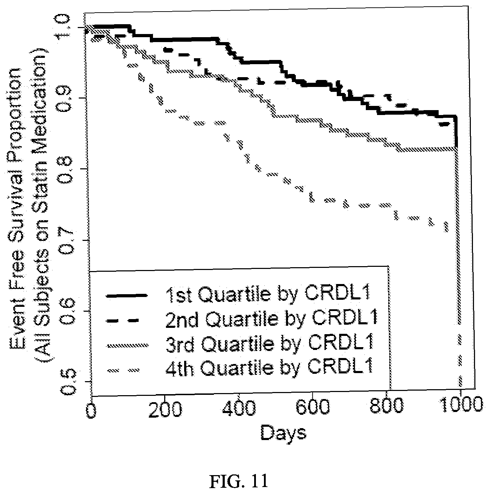

Figure 11 shows that in those subjects on statins, CHRDL1 is also associated with the risk of cardiovascular events in this high risk population. Figure 11 shows Kaplan Meier plots of all 538 subjects taking statin medication. It illustrates that CHRDL1 is associated with the event free survival of cardiovascular events in individuals treated with statin medications. Thus, despite the effects of treatment with statins, CHRDL1 is a useful biomarker of the risk of cardiovascular events.

**Table 1: CV Event Biomarkers**

| | SOMAmer Designation | Gene Name | Target | Swiss Prot Id | Entrez Gene Id | PUBLIC_NAME | Up or down |
|---|---|---|---|---|---|---|---|
| 1 | CRP.4337.49.2 | CRP | CRP | P02741 | 1401 | C-reactive protein | up |
| 2 | IGFBP1.2771.35.2 | IGFBP1 | IGFBP-1 | P08833 | 3484 | Insulin-like growth factor-binding protein 1 | up |
| 3 | IGFBP2.2570.72.5 | IGFBP2 | IGFBP-2 | P18065 | 3485 | Insulin-like growth factor-binding protein 2 | up |
| 4 | B2M.3485.28.2 | B2M | b2-Microglobulin | P61769 | 567 | β2-Microglobulin | up |
| 5 | TNFRSF1B.3152.57.1 | TNFRSF 1B | TNF sR-II | P20333 | 7133 | Tumor necrosis factor receptor superfamily member 1B | up |
| 6 | CFD.2946.52.2 | CFD | Factor D | P00746 | 1675 | Complement factor D | up |
| 7 | FCER2.3291.30.2 | FCER2 | CD23 | P06734 | 2208 | CD23 | up |
| 8 | LYZ.4920.10.1 | LYZ | Lysozyme | P61626 | 4069 | Lysozyme | up |
| 9 | FSTL3.3438.10.2 | FSTL3 | FSTL3 | 095633 | 10272 | Follistatin-like 3 | up |
| 10 | ANGPT2.2602.2.2 | ANGPT2 | Angiopoietin-2 | 015123 | 285 | Angiopoietin-2 | up |
| 11 | PI3.4982.54.1 | PI3 | Elafin | P19957 | 5266 | Elafin | up |
| 12 | MMP7.2789.26.2 | MMP7 | MMP-7 | P09237 | 4316 | Matrix metalloproteinase 7/Matrilysin | up |
| 13 | PLA2G2A.2692.74.2 | PLA2G2 A | NPS-PLA2 | P14555 | 5320 | Phospholipase A2, Group IIA | up |
| 14 | LCN2.2836.68.2 | LCN2 | Lipocalin 2 | P80188 | 3934 | Lipocalin 2 | up |
| 15 | PAPPA.4148.49.2 | PAPPA | PAPP-A | Q13219 | 5069 | Pregnancy-associated plasma protein-A | up |
| 16 | RETN.3046.31.1 | RETN | resistin | Q9HD8 9 | 56729 | Resistin | up |
| 17 | CCL14.2900.53.3 | CCL14 | HCC-1 | Q16627 | 6358 | Hemofiltrate CC Chemokine 1/CCL14 | up |
| 18 | PRSS2.5034.79.1 | PRSS2 | Trypsin 2 | P07478 | 5645 | Trypsin-2 | up |
| 19 | CAPG.4968.50.1 | CAPG | CAPG | P40121 | 822 | Macrophage-capping protein | up |
| 20 | TFF3.4721.54.2 | TFF3 | TFF3 | Q07654 | 7033 | Trefoil factor 3 | up |
| 21 | CHRDL1.3362.61.2 | CHRDL1 | CRDL1 | Q9BU4 0 | 91851 | Chordin-Like 1 | up |
| 22 | PPY.4588.1.2 | PPY | PH | P01298 | 5539 | Pancreatic hormone | up |
| 23 | GP6.3194.36.2 | GP6 | GPVI | Q9HCN 6 | 51206 | GPVI/Platelet Glycoprotein VI | up |
| 24 | CNDP1.3604.6.4 | CNDP1 | CNDP1 | Q96KN 2 | 84735 | Carnosine dipeptidase 1 | down |
| 25 | CA3.3799.11.2 | CA3 | Carbonic anhydrase III | P07451 | 761 | Carbonic anhydrase III | up |
| 26 | CCL15.3509.1.1 | CCL15 | MIP-5 | Q16663 | 6359 | Macrophage inflammatory protein 5/CCL15 | up |
| 27 | TIMP1.2211.9.6 | TIMP1 | TIMP-1 | P01033 | 7076 | Tissue inhibitor of metalloproteinases 1 | up |
| 28 | CTSH.3737.6.3 | CTSH | Cathepsin H | P09668 | 1512 | Cathepsin H | up |
| 29 | FCGR2B.3310.62.1 | FCGR2B | FCG2B | P31994 | 2213 | Immunoglobulin G Fc region receptor II-b, low affinity | up |
| 30 | AKR1A1.4192.10.2 | AKR1A1 | AK1A1 | P14550 | 10327 | Alcohol dehydrogenase (NADP+)/Ado-keto reductase family 1 member A1 | up |
| 31 | PGLYRP1.3329.14.2 | PGLYRP1 | PGRP-S | O75594 | 8993 | Peptidoglycan recognition protein, short | up |
| 32 | ROR1.2590.69.4 | ROR1 | ROR1 | Q01973 | 4919 | Tyrosine-protein kinase transmembrane receptor ROR1 | up |
| 33 | ESAM.2981.9.3 | ESAM | ESAM | Q96AP 7 | 90952 | Endothelial cell-selective adhesion molecule | up |
| 34 | VCAM1.2967.8.1 | VCAM1 | VCAM-1 | P19320 | 7412 | Vascular cell adhesion protein 1/VCAM 1 | up |
| 35 | CD5L.3293.2.3 | CD5L | CD5L | 043866 | 922 | CD5 antigen-like | up |
| 36 | COL18A1.2201.17.6 | COL18A1 | Endostatin | P39060 | 80781 | Endostatin | up |
| 37 | CTSZ.4971.1.1 | CTSZ | CATZ | Q9UBR 2 | 1522 | Cathepsin Z | up |
| 38 | CCL18.3044.3.2 | CCL18 | PARC | P55774 | 6362 | Macrophage inflammatory protein 4/Pulmonary and activation-regulated chemokine/CCL18 | up |
| 39 | IGFBP4.2950.57.2 | IGFBP4 | IGFBP-4 | P22692 | 3487 | Insulin-like growth factor-binding protein 4 | up |
| 40 | PLAUR.2652.15.1 | PLAUR | suPAR | Q03405 | 5329 | Urokinase plasminogen activator surface receptor | up |
| 41 | IL16.2774.10.3 | IL16 | IL-16 | Q14005 | 3603 | Interleukin-16 | up |
| 42 | THBS2.3339.33.1 | THBS2 | TSP2 | P35442 | 7058 | Thrombospondin-2 | up |
| 43 | IGFBP6.2686.67 .2 | IGFBP6 | IGFBP-6 | P24592 | 3489 | Insulin-like growth factor-binding protein 6 | up |
| 44 | TNFRSF1A.2654.19.1 | TNFRSF1A | TNF sR-I | P19438 | 7132 | Tumor necrosis factor receptor superfamily member 1A | up |
| 45 | MATN2.3325.2.2 | MATN2 | MATN2 | O00339 | 4147 | Matrilin-2 | up |
| 46 | MMP1.4924.32.1 | MMP1 | MMP-1 | P03956 | 4312 | Matrix metalloproteinase 1/collagenase 1 | up |
| 47 | IGF1.2952.75.2 | IGF1 | IGF-I | P05019 | 3479 | Insulin-like growth factor I | down |
| 48 | CRK.4976.57.1 | CRK | CRK | P46108 | 1398 | Adaptor protein Crk-I | up |
| 49 | MB.3042.7.2 | MB | Myoglobin | P02144 | 4151 | Myoglobin | up |
| 50 | SLPI.4413.3.2 | SLPI | SLPI | P03973 | 6590 | Secretory leukocyte protease inhibitor | up |
| 51 | IL18BP.3073.51.2 | IL18BP | IL-18 BPa | O95998 | 10068 | Interleukin-18 binding protein | up |
| 52 | IL1RL1.4234.8.2 | IL1RL1 | IL-1 R4 | Q01638 | 9173 | Interleukin-1 receptor 4 | up |
| 53 | F3.4931.59.1 | F3 | TF | P13726 | 2152 | Tissue Factor | up |
| 54 | STC1.4930.21.1 | STC1 | Stanniocalcin-1 | P52823 | 6781 | Stanniocalcin-1 | up |
| 55 | ADIPOQ.3554.24.1 | ADIPOQ | Adiponectin | Q15848 | 9370 | Adiponectin | up |
| 56 | PROC.2961.1.2 | PROC | Protein C | P04070 | 5624 | Protein C | down |
| 57 | REN.3396.54.2 | REN | Renin | P00797 | 5972 | Renin | up |
| 58 | CCL23.2913.1.2 | CCL23 | MPIF-1 | P55773 | 6368 | Myeloid progenitor inhibitory factor 1/CCL23 | up |
| 59 | LBP.3074.6.2 | LBP | LBP | P18428 | 3929 | Lipopolysaccharide-binding protein | up |
| 60 | GCG.4891.50.1 | GCG | Glucagon | P01275 | 2641 | Glucagon | up |
| 61 | YWHAG.4179.57.3 | YWHAG | 14-3-3 protein gamma | P61981 | 7532 | 14-3-3 protein γ | up |
| 62 | CCDC80.3234.23.2 | CCDC80 | URB | Q76M9 6 | 15188 7 | Coiled-coil domain-containing protein 80 | up |
| 63 | CNTFR.2711.6.2 | CNTFR | CNTFR alpha | P26992 | 1271 | Ciliary neurotrophic factor receptor α | up |
| 64 | EFNA5.2615.60.2 | EFNA5 | Ephrin-A5 | P52803 | 1946 | Ephrin-A5 | up |
| 65 | CST3.2609.59.2 | CST3 | Cystatin C | P01034 | 1471 | Cystatin C | up |
| 66 | FUT5.4549.78.2 | FUT5 | FUT5 | Q11128 | 2527 | Fucosyltransferase 5 | up |
| 67 | TNFRSF17.2665.26.2 | TNFRSF 17 | BCMA | Q02223 | 608 | B-cell maturation protein | up |
| 68 | ERP29.4983.6.1 | ERP29 | ERP29 | P30040 | 10961 | Endoplasmic reticulum resident protein 29 | up |
| 69 | RARRES2.3079.62.2 | RARRES 2 | TIG2 | Q99969 | 5919 | Chemerin | up |
| 70 | MAP2K2.3628.3.4 | MAP2K2 | MP2K2 | P36507 | 5605 | MAPK kinase 2 | up |
| 71 | EPHA1.3431.54.2 | EPHA1 | EphA1 | P21709 | 2041 | Ephrin type-A receptor 1 | up |
| 72 | CLEC11A.4500.50.2 | CLEC11 A | SCGF-alpha | Q9Y240 | 6320 | Stem Cell Growth Factor-α | up |
| 73 | F10.4878.34.1 | F10 | Coagulation Factor X | P00742 | 2159 | Coagulation Factor X | down |
| 74 | CHIT1.3600.2.3 | CHIT1 | Chitotriosidase -1 | Q13231 | 1118 | Chitotriosidase-1 | up |
| 75 | ITGA1.ITGB1..3503.4.2 | ITGA1 ITGB 1 | Integrin a1b1 | P56199, P05556 | 3672 3688 | Integrin α-I: β-1 complex | up |
| 76 | CKB.CKM..3714.49.2 | CKB CKM | CK-MB | P12277 P06732 | 1152 1158 | Creatine kinase-MB | down |
| 77 | CTSB.3061.61.2 | CTSB | Cathepsin B | P07858 | 1508 | Cathepsin B | up |
| 78 | CD163.5028.59.1 | CD163 | sCD163 | Q86VB 7 | 9332 | Scavenger receptor cysteine-rich type 1 protein M130 chain/Soluble CD163 | up |
| 79 | PTN.3045.72.2 | PTN | PTN | P21246 | 5764 | Pleiotrophin | up |
| 80 | CSF1R.2638.12.2 | CSF1R | M-CSF R | P07333 | 1436 | Macrophage colony-stimulating factor 1 receptor | up |
| 81 | IGFBP3.2571.12.3 | IGFBP3 | IGFBP-3 | P17936 | 3486 | Insulin-like growth factor-binding protein 3 | down |
| 82 | CXCL12.3516.60.2 | CXCL12 | SDF-1b | P48061 | 6387 | Stromal cell-derived factor 1β | up |
| 83 | PEBP1.4276.10.2 | PEBP1 | prostatic binding protein | P30086 | 5037 | Phosphatidylethanolamine-bi nding protein 1 | up |
| 84 | CCL22.3508.78.3 | CCL22 | MDC | O00626 | 6367 | Macrophage-derived chemokine | up |
| 85 | CST2.4324.33.2 | CST2 | CYTT | P09228 | 1470 | Cystatin SA | up |
| 86 | CCL23.3028.36.2 | CCL23 | Ck-b-8-1 | P55773 | 6368 | Ck-β-8-1/Macrophage inflammatory protein 3 splice | up |
| | | | | | | variant (aa 46-137) | |
| 87 | CADM1..3326.58.2 | CADM1 | Nectin-like protein 2 | Q9BY6 7 | 23705 | Nectin-like protein 2 | up |
| 88 | VWF.3050.7.2 | VWF | vWF | P04275 | 7450 | von Willebrand factor | up |
| 89 | CST5.3803.10.2 | CST5 | CYTD | P28325 | 1473 | Cystatin D | up |
| 90 | CHST15.4469.78.2 | CHST15 | ST4S6 | Q7LFX 5 | 51363 | Carbohydrate sulfotransferase 15 | up |
| 91 | IL1R1.2991.9.2 | IL1R1 | IL-1 sRI | P14778 | 3554 | Interleukin-1 receptor 1 | up |
| 92 | C9.3060.43.2 | C9 | C9 | P02748 | 735 | Complement C9 | up |
| 93 | AFM.4763.31.3 | AFM | Afamin | P43652 | 173 | Afamin | down |
| 94 | TGFBR3.3009.3.2 | TGFBR3 | TGF-b R III | Q03167 | 7049 | Transforming growth factor β receptor type III | up |
| 95 | FRZB.2841.13.2 | FRZB | sFRP-3 | Q92765 | 2487 | Frizzled-related protein 3, secreted | up |
| 96 | MMP12.4496.60.2 | MMP12 | MMP-12 | P39900 | 4321 | Matrix metalloproteinase 12/Macrophage metalloelastase | up |
| 97 | CD33.3166.92.1 | CD33 | Siglec- 3 | P20138 | 945 | Siglec-3 | up |
| 98 | EPHA2.4834.61.2 | EPHA2 | Epithelial cell kinase | P29317 | 1969 | Ephrin type-A receptor 2 | up |
| 99 | CXCL16.2436.49.4 | CXCL16 | CXCL16, soluble | Q9H2A 7 | 58191 | Scavenger receptor for phosphatidylserine and oxidized low density lipoprotein/CXCL16 | up |
| 100 | IGFBP7.3320.49.2 | IGFBP7 | IGFBP-7 | Q16270 | 3490 | Insulin-like growth factor-binding protein 7 | up |
| 101 | KLK3.SERPINA 3.4153.11.2 | KLK3 SERPIN A3 | PSA-ACT | P07288, P01011 | 354 12 | PSA: α-1-antichymotrypsin complex | up |
| 102 | PRTN3.3514.49.2 | PRTN3 | Proteinase-3 | P24158 | 5657 | Proteinase-3 | up |
| 103 | GNLY.3195.50.2 | GNLY | Granulysin | P22749 | 10578 | Granulysin | up |
| 104 | LY9.3324.51.1 | LY9 | LY9 | Q9HBG 7 | 4063 | T-lymphocyte surface antigen Ly-9/CD229 | up |
| 105 | NRP1.3214.3.2 | NRP1 | NRP1 | O14786 | 8829 | Neuropilin-1 | up |
| 106 | C7.2888.49.2 | C7 | C7 | P10643 | 730 | Complement C7 | up |
| 107 | KLKB1.4152.58.2 | KLKB1 | Prekallikrein | P03952 | 3818 | Prekallikrein | down |
| 108 | CCL21.2516.57.3 | CCL21 | 6Ckine | O00585 | 6366 | 6Ckine/CCL21 | up |
| 109 | RGMB.3331.8.1 | RGMB | RGMB | Q6NW4 0 | 28570 4 | RGM domain family member B | up |
| 110 | ESM1.3805.16.2 | ESM1 | Endocan | Q9NQ3 0 | 11082 | Endocan | up |
| 111 | DKK3.3607.71.1 | DKK3 | DKK3 | Q9UBP 4 | 27122 | Dickkopf-related protein 3 | up |
| 112 | CDNF.4962.52.1 | CDNF | ARMEL | Q49AH 0 | 44154 9 | Conserved dopamine neurotrophic factor | up |
| 113 | SPON1.4297.62.3 | SPON1 | Spondin-1 | Q9HCB 6 | 10418 | Spondin-1 | up |
| 114 | LSAMP.2999.6.2 | LSAMP | LSAMP | Q13449 | 4045 | Limbic system-associated membrane protein | up |
| 115 | DCTPP1.4314.12.2 | DCTPP1 | XTP3A | Q9H773 | 79077 | dCTP pyrophosphatase 1 | up |
| 116 | NID1.3213.65.2 | NIDI | Nidogen | P14543 | 4811 | Nidogen | up |
| 117 | A2M.3708.62.1 | A2M | a2-Macroglobu lin | P01023 | 2 | a2-Macroglobulin | up |
| 118 | FCGR3B.3311.27.1 | FCGR3B | FCG3B | O75015 | 2215 | Immunoglobulin G Fc region receptor III-B, low affinity | up |
| 119 | PROC.3758.63.3 | PROC | Activated Protein C | P04070 | 5624 | Activated Protein C | down |
| 120 | ADAMTS13.3175.51.5 | ADAMT S13 | ATS13 | Q76LX 8 | 11093 | ADAM metallopeptidase with thrombospondin motifs 13 | down |
| 121 | ANG.4874.3.1 | ANG | Angiogenin | P03950 | 283 | Angiogenin | up |
| 122 | GRN.4992.49.1 | GRN | GRN | P28799 | 2896 | Progranulin | up |
| 123 | CD48.3292.75.1 | CD48 | CD48 | P09326 | 962 | CD48 | up |
| 124 | FGA.FGB.FGG. 4907.56.1 | FGA FGB FGG | D-dimer | P02671 P02675 P02679 | 2243 2244 2266 | D-dimer | up |
| 125 | IGHG1.IGHG2.I GHG3.IGHG4.I GK..IGL..3700.1 5.4 | IGHG1 IGHG2 IGHG3 IGHG4 IGK@ IGL@ | IgG | NA | NA | IgG | |
| 126 | NAGK.3894.15.2 | NAGK | NAGK | Q9UJ70 | 55577 | N-acetyl-D-glucosamine kinase | up |
| 127 | TNC.4155.3.2 | TNC | Tenascin | P24821 | 3371 | Tenascin | up |
| 128 | RET.3220.40.2 | RET | RET | P07949 | 5979 | Proto-oncogene tyrosine-protein kinase receptor Ret | down |
| 129 | MDK.2911.27.2 | MDK | Midkine | P21741 | 4192 | Midkine | up |
| 130 | TNFRSF10D.3129.73.2 | TNFRSF 10D | TRAIL R4 | Q9UBN 6 | 8793 | Tumor necrosis factor receptor superfamily member 10D | up |
| 131 | CD84.3642.4.1 | CD84 | SLAF5 | Q9UIB8 | 8832 | Signaling lymphocytic activation molecule 5 | up |
| 132 | EGFR.2677.1.1 | EGFR | ERBB1 | P00533 | 1956 | erbB1/HER1 | down |
| 133 | SERPINA4.3449.58.2 | SERPIN A4 | Kallistatin | P29622 | 5267 | Kallistatin | down |
| 134 | MRC2.3041.55.2 | MRC2 | MRC2 | Q9UBG 0 | 9902 | Macrophage mannose receptor 2 | up |
| 135 | GHR.2948.58.2 | GHR | Growth hormone receptor | P10912 | 2690 | Growth hormone receptor | down |
| 136 | CXCL12.2330.2.1 | CXCL12 | SDF-1a | P48061 | 6387 | Stromal cell-derived factor 1α | up |
| 137 | SERPINF2.3024.18.2 | SERPINF 2 | a2- Antiplasmin | P08697 | 5345 | α2-Antiplasmin | down |
| 138 | RUNX2.3457.57.1 | RUNX2 | Osteoblast-spec if transcr fact 2 | Q13950 | 860 | Osteoblast-specific transcription factor 2 | up |
| 139 | CLEC11A.2966.65.2 | CLEC11 A | SCGF-beta | Q9Y240 | 6320 | Stem Cell Growth Factor-β | up |
| 140 | SIGLEC7.2742.68.2 | SIGLEC7 | Siglec-7 | Q9Y286 | 27036 | Siglec-7 | up |
| 141 | VEGFA.2597.8.3 | VEGFA | VEGF | P15692 | 7422 | Vascular endothelial growth factor A | up |
| 142 | BMPER.3654.27.1 | BMPER | BMPER | Q8N8U 9 | 16866 7 | Bone morphogenetic protein-binding endothelial regulator protein | down |
| 143 | SOD2.5008.51.1 | SOD2 | Mn SOD | P04179 | 6648 | Superoxide dismutase [Mn] | down |
| 144 | OPCML.3634.5.4 | OPCML | OBCAM | Q14982 | 4978 | Opioid-binding cell adhesion molecule | down |
| 145 | CTSS.3181.50.2 | CTSS | Cathepsin S | P25774 | 1520 | Cathepsin S | up |
| 146 | PLG.4151.6.2 | PLG | Plasminogen | P00747 | 5340 | Plasminogen | down |
| 147 | PAFAH1B2.2642.4.1 | PAFAH1 B2 | PAFAH beta subunit | P68402 | 5049 | Platelet-activating factor acetylhydrolase IB subunit β/PAFAH β subunit | up |
| 148 | IGF1R.4232.19.2 | IGF1R | IGF-I sR | P08069 | 3480 | Insulin-like growth factor I receptor | up |
| 149 | AGT.3484.60.2 | AGT | Angiotensinoge n | P01019 | 183 | Angiotensinogen | down |
| 150 | GDF11.2765.4.3 | GDF11 | GDF-11 | O95390 | 10220 | Growth-differentiation factor 11 | down |
| 151 | MMP14.5002.76.1 | MMP14 | MMP-14 | P50281 | 4323 | Matrix metalloproteinase 14/Membrane type matrix metalloproteinase 1 | up |
| 152 | SELP.4154.57.2 | SELP | P-Selectin | P16109 | 6403 | P-Selectin | up |
| 153 | BGN.3284.75.1 | BGN | BGN | P21810 | 633 | Biglycan | up |
| 154 | IL6ST.2620.4.2 | IL6ST | gp130, soluble | P40189 | 3572 | Interleukin-6 receptor subunit β/gp130 | up |
| 155 | PSMA2.4280.47.2 | PSMA2 | PSA2 | P25787 | 5683 | Proteasome subunit α2 | up |

**Table 2: List of 46 Biomarkers**

| | SOMAmer Designation | Gene Name | Target | Swiss Prot ID | Entrez Gene Id | PUBLIC_NAME | Up or down |
|---|---|---|---|---|---|---|---|
| 1 | ANGPT2.2602.2.2 | ANGPT2 | Angiopoietin-2 | O15123 | 285 | Angiopoietin-2 | up |
| 2 | CTSH.3737.6.3 | CTSH | Cathepsin H | P09668 | 1512 | Cathepsin H | up |
| 3 | TIMP1.2211.9.6 | TIMP1 | TIMP-1 | P01033 | 7076 | Tissue inhibitor of metalloproteinases 1 | up |
| 4 | B2M.3485.28.2 | B2M | b2-Microglob ulin | P61769 | 567 | β2-Microglobulin | up |
| 5 | TNFRSF1B.3152.57.1 | TNFRSF1 B | TNF sR-II | P20333 | 7133 | Tumor necrosis factor receptor superfamily member 1B | up |
| 6 | FSTL3.3438.10.2 | FSTL3 | FSTL3 | O95633 | 10272 | Follistatin-like 3 | up |
| 7 | MMP7.2789.26.2 | MMP7 | MMP-7 | P09237 | 4316 | Matrix metalloproteinase 7/Matrilysin | up |
| 8 | IGFBP4.2950.57.2 | IGFBP4 | IGFBP-4 | P22692 | 3487 | Insulin-like growth factor-binding protein 4 | up |
| 9 | SLPI.4413.3.2 | SLPI | SLPI | P03973 | 6590 | Secretory leukocyte protease inhibitor | up |
| 10 | THBS2.3339.33.1 | THBS2 | TSP2 | P35442 | 7058 | Thrombospondin-2 | up |
| 11 | CHRDL1.3362.61.2 | CHRDL1 | CRDL1 | Q9BU40 | 91851 | Chordin-Like 1 | up |
| 12 | CFD.2946.52.2 | CFD | Factor D | P00746 | 1675 | Complement factor D | up |
| 13 | SPON1.4297.62.3 | SPON1 | Spondin-1 | Q9HCB6 | 10418 | Spondin-1 | up |
| 14 | COL18A1.2201.17.6 | COL18A1 | Endostatin | P39060 | 80781 | Endostatin | up |
| 15 | IGFBP2.2570.72.5 | IGFBP2 | IGFBP-2 | P18065 | 3485 | Insulin-like growth factor-binding protein 2 | up |
| 16 | MMP12.4496.60.2 | MMP12 | MMP-12 | P39900 | 4321 | Matrix metalloproteinase 12/Macrophage metalloelastase | up |
| 17 | CCL14.2900.53.3 | CCL14 | HCC-1 | Q16627 | 6358 | Hemofiltrate CC Chemokine 1/CCL14 | up |
| 18 | EFNA5.2615.60.2 | EFNA5 | Ephrin-A5 | P52803 | 1946 | Ephrin-A5 | up |
| 19 | PLAUR.2652.15.1 | PLAUR | suPAR | Q03405 | 5329 | Urokinase plasminogen activator surface receptor | up |
| 20 | C7.2888.49.2 | C7 | C7 | P10643 | 730 | Complement C7 | up |
| 21 | STC1.4930.21.1 | STC1 | Stanniocalcin -1 | P52823 | 6781 | Stanniocalcin-1 | up |
| 22 | KLK3.SERPINA 3.4153.11.2 | KLK3 SERPINA 3 | PSA-ACT | P07288, P01011 | 354 12 | PSA:α-1-antichymotrypsin complex | up |
| 23 | CST3.2609.59.2 | CST3 | Cystatin C | P01034 | 1471 | Cystatin C | up |
| 24 | CTSZ.4971.1.1 | CTSZ | CATZ | Q9UBR2 | 1522 | Cathepsin Z | up |
| 25 | SERPINF2.3024. 18.2 | SERPINF 2 | a2-Antiplasm in | P08697 | 5345 | α2-Antiplasmin | down |
| 26 | PROC.2961.1.2 | PROC | Protein C | P04070 | 5624 | Protein C | down |
| 27 | CCL15.3509.1.1 | CCL15 | MIP-5 | Q16663 | 6359 | Macrophage inflammatory protein 5/CCL15 | up |
| 28 | CD84.3642.4.1 | CD84 | SLAF5 | Q9UIB8 | 8832 | Signaling lymphocytic activation molecule 5 | up |
| 29 | VCAM1.2967.8.1 | VCAM1 | VCAM-1 | P19320 | 7412 | Vascular cell adhesion protein 1/VCAM 1 | up |
| 30 | TNFRSF1A.2654.19.1 | TNFRSF1 A | TNF sR-I | P19438 | 7132 | Tumor necrosis factor receptor superfamily member 1A | up |
| 31 | CCDC80.3234.23.2 | CCDC80 | URB | Q76M96 | 151887 | Coiled-coil domain-containing protein 80 | up |
| 32 | IL18BP.3073.51.2 | IL18BP | IL-18 BPa | O95998 | 10068 | Interleukin-18 binding protein | up |
| 33 | IL1R1.2991.9.2 | IL1R1 | IL-1 sRI | P14778 | 3554 | Interleukin-1 receptor 1 | up |
| 34 | CXCL12.2330.2.1 | CXCL12 | SDF-1a | P48061 | 6387 | Stromal cell-derived factor 1α | up |
| 35 | PLA2G2A.2692.74.2 | PLA2G2 A | NPS-PLA2 | P14555 | 5320 | Phospholipase A2, Group IIA | up |
| 36 | CAPG.4968.50.1 | CAPG | CAPG | P40121 | 822 | Macrophage-capping protein | up |
| 37 | CNDP1.3604.6.4 | CNDP1 | CNDP1 | Q96KN2 | 84735 | Carnosine dipeptidase 1 | down |
| 38 | ESAM.2981.9.3 | ESAM | ESAM | Q96AP7 | 90952 | Endothelial cell-selective adhesion molecule | up |
| 39 | MATN2.3325.2.2 | MATN2 | MATN2 | O00339 | 4147 | Matrilin-2 | up |
| 40 | LYZ.4920.10.1 | LYZ | Lysozyme | P61626 | 4069 | Lysozyme | up |
| 41 | PLG.4151.6.2 | PLG | Plasminogen | P00747 | 5340 | Plasminogen | down |
| 42 | C9.3060.43.2 | C9 | C9 | P02748 | 735 | Complement C9 | up |
| 43 | LCN2.2836.68.2 | LCN2 | Lipocalin 2 | P80188 | 3934 | Lipocalin 2 | up |
| 44 | NID1.3213.65.2 | NID1 | Nidogen | P14543 | 4811 | Nidogen | up |
| 45 | CHST15.4469.78.2 | CHST15 | ST4S6 | Q7LFX5 | 51363 | Carbohydrate sulfotransferase 15 | up |
| 46 | ROR1.2590.69.4 | ROR1 | ROR1 | Q01973 | 4919 | Tyrosine-protein kinase transmembrane receptor ROR1 | up |

**Table 3: List of 10 Biomarkers**

| | SOMAmer Designation | GeneName | Target | SwissProt ID | Entrez Gene Id | PUBLIC_NAME | up or down in high risk |
|---|---|---|---|---|---|---|---|
| 1 | ANGPT2.2602.2.2 | ANGPT2 | Angiopoietin-2 | O15123 | 285 | Angiopoietin-2 | up |
| 2 | MMP7.2789.26.2 | MMP7 | MMP-7 | P09237 | 4316 | Matrix metalloproteinase 7/Matrilysin | up |
| 3 | THBS2.3339.33.1 | THBS2 | TSP2 | P35442 | 7058 | Thrombospondin-2 | up |
| 4 | CHRDL1.3362.61.2 | CHRDL1 | CRDL1 | Q9BU40 | 91851 | Chordin-Like 1 | up |
| 5 | C7.2888.49.2 | C7 | C7 | P10643 | 730 | Complement C7 | up |
| 6 | KLK3.SERPINA3.4153.11.2 | KLK3 SERPINA3 | PSA-ACT | P07288, P01011 | 354 12 | PSA:α-1-antichymotrypsin complex | up |
| 7 | ESAM.2981.9.3 | ESAM | ESAM | Q96AP7 | 90952 | Endothelial cell-selective adhesion molecule | up |
| 8 | MATN2.3325.2.2 | MATN2 | MATN2 | O00339 | 4147 | Matrilin-2 | up |
| 9 | PLG.4151.6.2 | PLG | Plasminogen | P00747 | 5340 | Plasminogen | down |
| 10 | GP6.3194.36.2 | GP6 | GPVI | Q9HCN6 | 51206 | GPVI/Platelet Glycoprotein VI | up |

**Table 4: Heart & Soul Study Population Event Time and Type**

| TIME | CHF | Chronic CHF CHF | DEATH | MI | No.Event | Chronic. CHF No Event | Stroke |
|---|---|---|---|---|---|---|---|
| 0-6months | 8 | 5 | 8 | 13 | 0 | 0 | 5 |
| 6-12months | 3 | 6 | 8 | 11 | 0 | 0 | 2 |
| 12-24months | 16 | 8 | 15 | 32 | 0 | 0 | 8 |
| 2-3years | 8 | 6 | 22 | 16 | 0 | 0 | 9 |
| 3-4years | 12 | 6 | 17 | 9 | 0 | 0 | 1 |
| 4-5years | 13 | 5 | 39 | 14 | 0 | 0 | 5 |
| 5 years plus | 4 | 3 | 48 | 5 | 0 | 0 | 4 |
| No Event during study | 0 | 0 | 0 | 0 | 515 | 77 | 0 |
| | | | | | | | |
| Total | 64 | 39 | 157 | 100 | 515 | 77 | 34 |
| 986 | | | | | | | |

## Claims

1. A method for screening an individual for evaluation of risk of a CV event, the method comprising:
providing a biomarker panel comprising N of the biomarker proteins listed in Table 1; detecting biomarker proteins, in a biological sample from an individual, to give biomarker values that correspond to the biomarkers in the biomarker panel in order to predict risk for a CV event, based on said biomarker values, and wherein N = 3 - 155, and wherein the biomarker panel includes PSA-ACT.

2. The method of claim 1, wherein the biomarkers are selected from Table 2 and N=3-46 or the biomarkets are selected from Table 3 and N=3-10.

3. The method of claim 1 or claim 2, wherein the biomarker panel includes angiopoietin 2 MMP7, CHRDL1 or MATN2.

4. The method of claim 1 wherein the biomarker panel comprises the ten biomarkers of Table 3.

5. The method of any one of the preceding claims, wherein detecting the biomarker values comprises performing an in vitro assay comprising at least one capture reagent corresponding to each of said biomarkers, and further comprising selecting said at least one capture reagent from the group consisting of SOMAmers and antibodies.

6. The method of any one of the preceding claims, wherein the biological sample is selected from the group consisting of whole blood, dried blood spots, plasma, serum and urine.

7. The method according to any one of the preceding claims, wherein said individual is evaluated for risk of a CV event, based on said biomarker values and at least one item of additional biomedical information corresponding to said individual, wherein said at least one item of additional biomedical information is independently selected from the group consisting of:
(a) information corresponding to the presence of cardiovascular risk factors selected from the group consisting of prior myocardial infarction, angiographic evidence of greater than 50% stenosis in one or more coronary vessels, exercise-induced ischemia by treadmill or nuclear testing or prior coronary revascularization,
(b) information corresponding to physical descriptors of said individual,
(c) information corresponding to a change in weight of said individual,
(d) information corresponding to the ethnicity of said individual,
(e) information corresponding to the gender of said individual,
(f) information corresponding to said individual's smoking history,
(g) information corresponding to said individual's alcohol use history,
(h) information corresponding to said individual's occupational history,
(i) information corresponding to said individual's family history of cardiovascular disease or other circulatory system conditions,
(j) information corresponding to the presence or absence in said individual of at least one genetic marker correlating with a higher risk of cardiovascular disease in said individual or a family member of said individual,
(k) information corresponding to clinical symptoms of said individual,
(l) information corresponding to other laboratory tests,
(m) information corresponding to gene expression values of said individual, and
(n) information corresponding to said individual's consumption of known cardiovascular risk factors such as diet high in saturated fats, high salt, high cholesterol,
(o) information corresponding to the individual's imaging results obtained by techniques selected from the group consisting of electrocardiogram, echocardiography, carotid ultrasound for intima-media thickness, flow mediated dilation, pulse wave velocity, ankle-brachial index, stress echocardiography, myocardial perfusion imaging, coronary calcium by CT, high resolution CT angiography, MRI imaging, and other imaging modalities, and
(p) information regarding the individual's medications.

8. The method according to any one of the preceding claims wherein the risk of a future cardiovascular (CV) Event within a 5 year time period is evaluated.

9. The method of claim 8, further comprising:
selecting the population on the basis of a characteristic selected from the group consisting of:
a) a population having or not having a prior history of cardiovascular disease; and
b) a population having or not having genetic risk factors comprising mutations, single nucleotide polymorphisms and/or insertion/deletions.

10. The method of claim 8, wherein the evaluated risk for CV event for individuals permits their stratification into categories selected from the group consisting of:
a) different categories for increased or decreased disease manage programs;
b) different risk categories relating to life insurance coverage;
c) different risk categories relating to health insurance coverage;
d) different categories of candidacy for partnership;
e) different categories of eligibility for entry into clinical trials of CV therapeutics;
f) different risk categories relating to cardiovascular safety for a CV therapeutic or any therapeutic.

11. A computer-implemented method for evaluating the risk of a cardiovascular (CV) Event, the method comprising:
providing a biomarker panel comprising N of the protein biomarkers from Table 1;
retrieving on a computer biomarker information for an individual, wherein the biomarker information comprises biomarker values that correspond to one of at least N biomarkers selected from Table 1;
performing with the computer a classification of each of said biomarker values;
indicating a result of the evaluation of risk for a CV event for said individual based upon a plurality of classifications, wherein indicating the result of the evaluation of risk of a CV event for the individual optionally comprises displaying the result on a computer display; and
wherein N = 3 - 155, and wherein the biomarker panel includes PSA-ACT.

## Patentansprüche

1. Verfahren zum Screenen eines Individuums zur Bewertung eines Risikos für ein kardiovaskuläres Ereignis, wobei das Verfahren Folgendes umfasst:
Bereitstellen einer Biomarker-Tafel, umfassend N der in Tabelle 1 aufgeführten Biomarkerproteine;
Detektieren von Biomarkerproteinen in einer biologischen Probe eines Individuums, um Biomarkerwerte zu ergeben, die den Biomarkern in der Biomarker-Tafel entsprechen, um ein Risiko für ein kardiovaskuläres Ereignis basierend auf den Biomarkerwerten vorherzusagen, und wobei N = 3-155 ist und wobei die Biomarker-Tafel PSA-ACT umfasst.

2. Verfahren nach Anspruch 1, wobei die Biomarker aus Tabelle 2 ausgewählt sind und N = 3-46 ist oder die Biomarker aus Tabelle 3 ausgewählt sind und N = 3-10 ist.

3. Verfahren nach Anspruch 1 oder Anspruch 2, wobei die Biomarker-Tafel Angiopoietin 2 MMP7, CHRDL1 oder MATN2 umfasst.

4. Verfahren nach Anspruch 1, wobei die Biomarker-Tafel die 10 Biomarker aus Tabelle 3 umfasst.

5. Verfahren nach einem der vorangegangenen Ansprüche, wobei das Detektieren der Biomarkerwerte das Durchführen eines In-vitro-Assays, umfassend zumindest ein Einfangreagens, das jedem der Biomarker entspricht, und weiters das Auswählen des zumindest einen Einfangreagens aus der aus SOMAmeren und Antikörpern bestehenden Gruppe umfasst.

6. Verfahren nach einem der vorangegangenen Ansprüche, wobei die biologische Probe aus der aus Vollblut, getrockneten Blutflecken, Plasma, Serum und Harn bestehenden Gruppe ausgewählt ist.

7. Verfahren nach einem der vorangegangenen Ansprüche, wobei das Individuum auf ein Risiko für ein kardiovaskuläres Ereignis basierend auf den Biomarkerwerten und zumindest einem Punkt von zusätzlichen biomedizinischen Informationen, die dem Individuum entsprechen, bewertet wird, wobei der zumindest eine Punkt von zusätzlichen biomedizinischen Informationen unabhängig aus der aus Folgendem bestehenden Gruppe ausgewählt ist:
(a) Informationen, die dem Vorliegen von kardiovaskulären Risikofaktoren entsprechen, die aus der aus vorangegangenem Myokardinfarkt, angiographischem Nachweis von einer mehr als 50%igen Stenose in einem oder mehreren Koronargefäßen, Belastungsischämie durch Ergometer oder Nukleartests oder vorangegangener Koronarrevaskularisierung bestehenden Gruppe ausgewählt sind,
(b) Informationen entsprechend physischen Beschreibungsfaktoren des Individuums,
(c) Informationen entsprechend einer Gewichtsveränderung des Individuums,
(d) Informationen entsprechend der Ethnie des Individuums,
(e) Informationen entsprechend dem Geschlecht des Individuums,
(f) Informationen entsprechend dem Raucherstatus des Individuums,
(g) Informationen entsprechend dem Alkoholkonsum des Individuums,
(h) Informationen entsprechend dem Berufsfeld des Individuums,
(i) Informationen entsprechend der Familienanamnese des Individuums in Hinblick auf kardiovaskuläre Erkrankungen oder andere Leiden des Kreislaufsystems,
(j) Informationen entsprechend der Gegenwart oder Abwesenheit von zumindest einem genetischen Marker in dem Individuum, der mit einem höheren Risiko für eine kardiovaskuläre Erkrankung in dem Individuum oder einem Familienmitglied des Individuums in Zusammenhang steht,
(k) Informationen entsprechend klinischen Symptomen des Individuums,
(l) Informationen entsprechend anderen Labortests,
(m) Informationen entsprechend Genexpressionswerten des Individuums und
(n) Informationen entsprechend der Ernährung des Individuums in Hinblick auf kardiovaskuläre Risikofaktoren wie große Mengen an gesättigten Fetten, große Mengen Salz, hohes Cholesterin,
(o) Informationen entsprechend den Ergebnissen einer Bildgebungsdiagnostik des Individuums, die über Verfahren erhalten wurden, die aus der aus Elektrokardiogramm, Echokardiographie, Karotis-Ultraschall auf Intima-media-Dicke, strömungsvermittelter Erweiterung, Impulswellengeschwindigkeit, Knöchel-Arm-Index, Stress-Echokardiographie, Myokardperfusionsbildgebung, Koronarcalcium mittels CT, hochauflösender CT-Angiographie, MRI-Bildgebung und anderen Bildgebungsmodalitäten bestehenden Gruppe ausgewählt sind, und
(p) Informationen in Hinblick auf die Medikamenteneinnahme des Individuums.

8. Verfahren nach einem der vorangegangenen Ansprüche, wobei das Risiko für ein zukünftiges kardiovaskuläres (CV-) Ereignis innerhalb eines Zeitraums von 5 Jahren bewertet wird.

9. Verfahren nach Anspruch 8, das weiters Folgendes umfasst:
Auswählen der Population auf der Basis eines Merkmals, das aus der aus Folgendem bestehenden Gruppe ausgewählt ist:
a) einer Population, bei der eine Vorgeschichte für kardiovaskuläre Erkrankungen vorliegt oder nicht; und
b) einer Population, bei der genetische Risikofaktoren, umfassend Mutationen, Einzelnucleotidpolymorphismen und/oder Insertionen/Deletionen, vorliegen oder nicht.

10. Verfahren nach Anspruch 8, wobei das bewertete Risiko für ein CV-Ereignis für Individuen ihre Gliederung in Kategorien ermöglicht, die aus der aus Folgendem bestehenden Gruppen ausgewählt sind:
a) verschiedenen Kategorien für erweiterte oder eingeschränkte Krankheitsbekämpfungsprogramme;
b) verschiedenen Risikokategorien in Bezug auf die Deckung durch eine Lebensversicherung;
c) verschiedenen Risikokategorien in Bezug auf die Deckung durch eine Gesundheitsversicherung;
d) verschiedenen Kategorien einer Eignung für eine Partnerschaft;
e) verschiedenen Kategorien einer Eignung für Eintritt in klinische Studien für CV-Therapeutika;
f) verschiedenen Risikokategorien in Bezug auf die kardiovaskuläre Sicherheit für ein CV-Therapeutikum oder ein beliebiges Therapeutikum.

11. Computerimplementiertes Verfahren zum Bewerten des Risikos für ein kardiovaskuläres (CV-) Ereignis, wobei das Verfahren Folgendes umfasst:
Bereitstellen einer Biomarker-Tafel, umfassend N der Proteinbiomarker aus Tabelle 1;
Abrufen von Biomarker-Informationen für ein Individuum auf einem Computer, wobei die Biomarkerinformationen Biomarkerwerte umfassen, die einem aus zumindest N Biomarkern entsprechen, die aus Tabelle 1 ausgewählt sind;
Durchführen einer Klassifizierung jedes der Biomarkerwerte mit dem Computer;
Anzeigen eines Ergebnisses der Bewertung eines Risikos für ein CV-Ereignis für das Individuum basierend auf einer Vielzahl von Klassifikationen, wobei das Anzeigen des Ergebnisses der Bewertung des Risikos für ein CV-Ereignis für das Individuum gegebenenfalls das Anzeigen des Ergebnisses auf einer Computeranzeige umfasst; und
wobei N = 3-155 und wobei die Biomarker-Tafel PSA-ACT umfasst.

## Revendications

1. Procédé de criblage d'un individu pour l'évaluation du risque d'un événement CV, le procédé comprenant :
la fourniture d'un panel de biomarqueurs comprenant N des protéines de biomarqueur énumérées dans le tableau 1 ; la détection des protéines de biomarqueur, dans un échantillon biologique provenant d'un individu, pour obtenir des valeurs de biomarqueur qui correspondent aux biomarqueurs dans le panel de biomarqueurs afin de prédire le risque d'un événement CV, sur la base desdites valeurs de biomarqueur, et dans lequel N = 3 à 155, et dans lequel le panel de biomarqueurs comprend PSA-ACT.

2. Procédé selon la revendication 1, dans lequel les biomarqueurs sont choisis parmi le tableau 2 et N = 3 à 46 ou les biomarqueurs sont choisis parmi le tableau 3 et N = 3 à 10.

3. Procédé selon la revendication 1 ou la revendication 2, dans lequel le panel de biomarqueurs comprend l'angiopoïétine 2, MMP7, CHRDL1 ou MATN2.

4. Procédé selon la revendication 1, dans lequel le panel de biomarqueurs comprend les dix biomarqueurs du tableau 3.

5. Procédé selon l'une quelconque des revendications précédentes, dans lequel la détection des valeurs de biomarqueur comprend la conduite d'un essai *in vitro* comprenant au moins un réactif de capture correspondant à chacun desdits biomarqueurs, et comprenant en outre la sélection dudit au moins un réactif de capture dans le groupe constitué de SOMAmères et d'anticorps.

6. Procédé selon l'une quelconque des revendications précédentes, dans lequel l'échantillon biologique est choisi dans le groupe constitué de sang total, de taches de sang séché, de plasma, de sérum et d'urine.

7. Procédé selon l'une quelconque des revendications précédentes, dans lequel ledit individu est évalué pour le risque d'un événement CV, sur la base desdites valeurs de biomarqueur et au moins une information biomédicale supplémentaire correspondant audit individu, dans lequel ladite au moins une information biomédicale supplémentaire est indépendamment choisie dans le groupe constitué de :
(a) informations correspondant à la présence de facteurs de risque cardiovasculaire choisis dans le groupe constitué d'antécédent d'infarctus du myocarde, preuves angiographiques de plus de 50 % de sténose dans un ou plusieurs vaisseaux coronaires, ischémie induite par l'effort par essai sur tapis roulant ou nucléaire ou antécédent de revascularisation coronaire,
(b) informations correspondant à des descripteurs physiques dudit individu,
(c) informations correspondant à un changement de poids dudit individu,
(d) informations correspondant à l'ethnicité dudit individu,
(e) informations correspondant au sexe dudit individu,
(f) informations correspondant à l'historique de tabagisme dudit individu,
(g) informations correspondant à l'historique de consommation d'alcool dudit individu,
(h) informations correspondant à l'historique professionnel dudit individu,
(i) informations correspondant aux antécédents familiaux de cardiovasculaire maladie ou d'autres affections du système circulatoire dudit individu,
(j) informations correspondant à la présence ou l'absence chez ledit individu d'au moins un marqueur génétique corrélé à un risque plus élevé de maladie cardiovasculaire chez ledit individu ou un membre de la famille dudit individu,
(k) informations correspondant aux symptômes cliniques dudit individu,
(l) informations correspondant à d'autres essais de laboratoire,
(m) informations correspondant à des valeurs d'expression génique dudit individu, et
(n) informations correspondant à la consommation par ledit individu de facteurs de risque cardiovasculaire connus tels qu'une alimentation riche en graisses saturées, riche en sel, riche en cholestérol,
(o) informations correspondant aux résultats d'imagerie de l'individu obtenus par des techniques choisies dans le groupe constitué d'un électrocardiogramme, une échocardiographie, une échographie carotidienne pour l'épaisseur intima-média, la dilatation médiée par le flux, la vitesse d'onde de pouls, l'indice tibio-brachial, l'échocardiographie de stress, l'imagerie de perfusion myocardique, le calcium des coronaires par TDM, l'angiographie par TDM haute résolution, l'imagerie IRM, et d'autres modalités d'imagerie, et
(p) informations concernant les médications de l'individu.

8. Procédé selon l'une quelconque des revendications précédentes, dans lequel le risque d'un événement cardiovasculaire (CV) futur dans un délai de 5 ans est évalué.

9. Procédé selon la revendication 8, comprenant en outre :
la sélection de la population sur la base d'une caractéristique choisie dans le groupe constitué de :
a) une population ayant ou n'ayant pas un antécédent de maladie cardiovasculaire ; et
b) une population ayant ou n'ayant pas des facteurs de risque génétiques comprenant des mutations, des polymorphismes d'un seul nucléotide et/ou des insertion/délétions.

10. Procédé selon la revendication 8, dans lequel le risque d'événement CV évalué pour des individus permet leur stratification en catégories choisies dans le groupe constitué de :
a) différentes catégories pour des programmes de prise en charge augmentés ou diminués ;
b) différentes catégories de risque liées à une couverture d'assurance sur la vie ;
c) différentes catégories de risque liées à une couverture d'assurance de santé ;
d) différentes catégories de candidature pour un partenariat ;
e) différentes catégories d'éligibilité pour entrée dans des essais cliniques d'agents thérapeutiques CV ;
f) différentes catégories de risque liées à la sécurité cardiovasculaire pour un agent thérapeutique CV ou un agent thérapeutique quelconque.

11. Procédé mis en œuvre par ordinateur pour évaluer le risque d'un événement cardiovasculaire (CV), le procédé comprenant :
la fourniture d'un panel de biomarqueurs comprenant N des biomarqueurs protéiques du tableau 1 ;
l'extraction sur un ordinateur d'informations de biomarqueur pour un individu, les informations de biomarqueur comprenant des valeurs de biomarqueur qui correspondent à l'un d'au moins N biomarqueurs choisis dans le tableau 1 ;
la conduite avec l'ordinateur d'une classification de chacune desdites valeurs de biomarqueur ;
l'indication d'un résultat de l'évaluation du risque d'un événement CV pour ledit individu sur la base d'une pluralité de classifications, l'indication du résultat de l'évaluation du risque d'un événement CV pour l'individu comprenant facultativement l'affichage du résultat sur un écran d'ordinateur ; et
dans lequel N = 3 à 155, et dans lequel le panel de biomarqueurs comprend PSA-ACT.
